# EUROPÄISCHE PATENTSCHRIFT

(11) **EP 3 573 680 B1**
(45) Veröffentlichungstag und Bekanntmachung des Hinweises auf die Patenterteilung: **02.03.2022**
(21) Anmeldenummer: 18702269.4
(22) Anmeldetag: 29.01.2018
(51) Int. Cl.: A61M 1/16, A61B 50/18, A61B 50/13, A61G 12/00, G16H 20/40

(54) **MOBILES AUSWAHLSYSTEM UND BEHANDLUNGSWAGEN**
MOBILE SELECTION SYSTEM AND TREATMENT CARRIAGE
SYSTÈME MOBILE DE SÉLECTION ET CHARIOT DE TRAITEMENT

(30) Priorität: 30.01.2017 DE 102017201443
(43) Veröffentlichungstag der Anmeldung: 04.12.2019
(73) Patentinhaber: Fresenius Medical Care Deutschland GmbH, 61352 Bad Homburg (DE)
(72) Erfinder: SCHERMEIER, Olaf, 60320 Frankfurt (DE); KOULECHOV, Kirill, 61118 Bad Vilbel (DE)
(74) Vertreter: Ricker, Mathias
(86) Internationale Anmeldenummer: PCT/EP2018/052150
(87) Internationale Veröffentlichungsnummer: WO 2018/138345

(56) Entgegenhaltungen:
- US-A- 5 292 029
- US-A1- 2008 319 575
- US-A1- 2011 189 048
- US-B1- 6 655 545

## Beschreibung

Die vorliegende Erfindung liegt auf dem Gebiet der Medizintechnik und/oder Krankenhauslogistik und betrifft insbesondere ein mobiles Auswahlsystem zur Auswahl von medizinischen Zubehören sowie einen Behandlungswagen für ein solches Auswahlsystem.

Üblicherweise ist es für eine, insbesondere medizinische, Behandlung eines Patienten neben der eigentlichen Behandlung auch erforderlich, medizinische Zubehöre bereitzustellen. Eine solche Behandlung ist insbesondere eine Dialysebehandlung eines Patienten, welche üblicherweise regelmäßig wiederholt werden muss. Einerseits sind mit dem Fortschritt der Medizin die Behandlungen vielfältiger und das Zubehör zahlreicher, diversifizierter und spezialisierter geworden, woraus ein steigender Aufwand bei der Bereitstellung von medizinischem Zubehör für die Behandlung eines Patienten resultieren kann. Andererseits besteht auch im Medizinsektor, insbesondere bei der Medizintechnik und/oder Krankenhauslogistik, welche zu den eigentlichen medizinischen Behandlungen hinzutreten, der zunehmende Wunsch zur Optimierung und effizienten Ressourcennutzung bei gleich bleibend hoher oder noch weiter gesteigerter Qualität der Behandlung. Um medizinische Zubehöre bereitzustellen, werden diese üblicherweise in einem Behandlungswagen gelagert. Solche Behandlungswagen sind etwa aus dem Stand der Technik bekannt und weisen üblicherweise eine Arbeitsplatte, Schubladen, Schrankfächer und/oder ein Fahrwerk mit Rädern oder Rollen auf.

Aus der US 5,292,029 A ist ein mobiles Ausgabesystem für Medikamente und für die bei einer Behandlung zu verwendenden Instrumente bzw. Artikel bekannt, welches eine mobile Einheit aufweist, die einen Computer mit einer CPU und einer Eingabeeinrichtung sowie mehrere Schuladen, Fächer und eine Zugangsschublade für eine Tabletten/Arzneimittelkapsel-Ausgabeeinheit umfasst, an denen jeweils eine Lampe vorgesehen ist. Mittels der Eingabeeinrichtung kann beispielsweise von einer Krankenschwester eine jeweilige Patienten-ID eingegeben werden, woraufhin das Ausgabesystem von einem Arzt verschriebene Medikamente durch Entriegeln der Schuladen, Fächer und der Zugangsschublade, und Einschalten der entsprechenden Lampe, oder Behandlungsanweisungen ausgibt. An einem vorderen Ende der mobilen Einheit ist eine im Wesentlichen horizontale Fläche vorgesehen, die eine Arbeitsablage mit einem ausziehbaren Abschnitt bildet.

Aus der US 2008/319575 A1 ist ein System zur Lagerung und Verteilung von Medikamenten und anderen Vorräten in einer Gesundheitseinrichtung bekannt, das mehrere mobile Ausgabegeräte umfasst, welche mehrere Behälter zur Lagerung von Medikamenten und anderer Vorräte zur Behandlung oder Bedienung eines Patienten aufweisen.

Der Erfindung liegt die Aufgabe zu Grunde, die Bereitstellung von medizinischem Zubehör, insbesondere im Hinblick auf die Logistik, die Auswahl von geeignetem medizinischem Zubehör und/oder effiziente Nutzung von medizinischem Zubehör, zu verbessern, die Sicherheit und/oder Qualität bei einer Behandlung zu steigern und/oder die an einer Behandlung eines Patienten beteiligten Personen zu entlasten.

Die Erfindung löst diese Aufgabe jeweils durch ein mobiles Auswahlsystem zur Auswahl von medizinischen Zubehören gemäß der Lehre des unabhängigen Anspruchs 1, einen Behandlungswagen gemäß der Lehre des unabhängigen Anspruchs 13 und ein Verfahren zur Auswahl von medizinischen Zubehören gemäß der Lehre des unabhängigen Anspruchs 14. Bevorzugte Ausführungsformen, Weiterbildungen oder Varianten sind insbesondere Gegenstand der abhängigen Ansprüche. Der Gegenstand der Ansprüche wird ausdrücklich zum Teil der Offenbarung der Beschreibung gemacht. Aspekte der Erfindung werden nachfolgend erläutert:
Ein erster Aspekt der Erfindung betrifft ein mobiles Auswahlsystem zur Auswahl von medizinischen Zubehören, insbesondere für eine Kanülierung von Blutgefäßen von Patienten und/oder für eine Dialysebehandlung. Das mobile Auswahlsystem weist ein Steuerungssystem, eine Benutzerschnittstelle und einen Behandlungswagen auf. Das Steuerungssystem weist eine Zubehördatenbank und eine Datenspeichervorrichtung für die Zubehördatenbank auf, wobei die Zubehördatenbank für Datensätze über eine Mehrzahl an medizinischen Zubehören eingerichtet und auf der wenigstens einen Datenspeichervorrichtung gespeichert ist. Die Benutzerschnittstelle ist für eine oder mehrere Eingaben von einem Benutzer eingerichtet, durch die eine durchzuführende Behandlung festgelegt wird und auf Basis derer das Steuerungssystem infolge dieser Eingaben eine der durchzuführenden Behandlung zugeordnete Behandlungskennung bestimmt. Der Behandlungswagen weist eine oder mehrere Lagereinrichtungen auf, wobei die Lagereinrichtungen wenigstens zwei Lagerbereiche je für eine Zubehörgattung aus der Mehrzahl an medizinischen Zubehören aufweisen. Dabei ist das Steuerungssystem eingerichtet, ein Auswahlverfahren auszuführen, welches die folgenden Verfahrensschritte aufweist. In einem Verfahrensschritt des Auswahlverfahrens wird die Behandlungskennung der durchzuführenden Behandlung bestimmt. In einem weiteren Verfahrensschritt des Auswahlverfahrens wird einer oder werden mehrere Zubehörsetparameter, welche ein medizinisches Zubehörset aus einem oder mehreren für die durchzuführende Behandlung geeigneten medizinischen Zubehören kennzeichnen, anhand der Zubehördatenbank und wenigstens auf Basis der Behandlungskennung der durchzuführenden Behandlung bestimmt. Außerdem weist der Behandlungswagen wenigstens einen Herausgabemechanismus zur Herausgabe von medizinischen Zubehören auf, wobei das Steuerungssystem eingerichtet ist, den wenigstens einen Herausgabemechanismus auf Basis der Zubehörsetparameter so zu steuern, dass der Herausgabemechanismus wenigstens für eine der Lagereinrichtungen jene medizinische Zubehöre herausgibt, für deren Zubehörgattungen diese Lagereinrichtung Lagerbereiche aufweist und welche durch die Zubehörsetparameter, auf denen die Steuerung basiert, gekennzeichnet sind.

In einer breiten Auslegung sind die Mehrzahl an medizinischen Zubehören, die Datensätze über die Mehrzahl an medizinischen Zubehören und/oder das Zubehörset nicht ein Bestandteil des erfindungsgemäßen mobilen Auswahlsystems. Vorzugsweise können aber bei einer engeren Auslegung das Zubehörset, zumindest ein Teil der Mehrzahl an medizinischen Zubehören, insbesondere solche medizinische Zubehöre, welche für eine Kanülierung von Blutgefäßen von Patienten und/oder für eine Dialysebehandlung geeignet sind, und/oder die Datensätze über die Mehrzahl an medizinischen Zubehören ein Bestandteil des erfindungsgemäßen mobilen Auswahlsystems oder zumindest einer bevorzugten Ausführungsform des Auswahlsystems sein.

Unter einem "Behandlungswagen" ist im Sinne der Erfindung zumindest eine Vorrichtung zu verstehen, welche für die Lagerung von medizinischen Zubehören und die Bereitstellung von medizinischen Zubehören für Behandlungen an einem Behandlungsort eingerichtet ist und dafür vorgesehen ist, dass die medizinischen Zubehöre zu dem Behandlungsort mittels dem Behandlungswagen transportiert werden. Solche Behandlungswagen sind etwa aus dem Stand der Technik bekannt und weisen üblicherweise eine Arbeitsplatte, Schubladen, Schrankfächer und/oder ein Fahrwerk mit Rädern oder Rollen auf.

Für die Lagerung und Bereitstellung von medizinischen Zubehören weist ein Behandlungswagen wenigstens zwei Lagerbereiche auf, vorzugsweise etwa eine Schublade, die in wenigstens zwei Lagerbereiche unterteilt ist, oder mehrere Schubladen mit je wenigstens einem Lagerbereich, wobei jeder Lagerbereich für medizinische Zubehöre einer bestimmten Gattung vorgesehen ist. Insbesondere kann ein Behandlungswagen mit medizinischen Zubehören für eine bestimmte Behandlung oder eine bestimmte Gruppe von Behandlungen bestückt sein. Insbesondere kann also ein Behandlungswagen eine Mehrzahl an medizinischen Zubehören lagern, wobei die Mehrzahl an medizinischen Zubehören für eine bestimmte Behandlung oder eine bestimmte Gruppe von Behandlungen vorausgewählt, insbesondere vorsortiert, ist.

Insbesondere für eine Dialysebehandlung können bei einer solchen Vorsortierung die folgenden medizinischen Zubehöre, d.h. insbesondere je eines oder mehrere medizinische Zubehöre einer Zubehörgattung, in Lagerbereichen des Behandlungswagen gelagert und damit für die Dialysebehandlung bereitgestellt werden. Für die Vorbereitung der Behandlung werden in einem Lagerbereich Einmalhandschuhe, insbesondere Einmalhandschuhe aus einem bestimmten Material und mit einer bestimmten Größe - die Zubehörgattung ist also ein Einmalhandschuh, der weiter durch sein bestimmtes Material und seine bestimmte Größe gekennzeichnet ist -, und in weiteren Lagerbereichen Desinfektionsmittel und Tupfer gelagert. Insbesondere können dabei weitere Einmalhandschuhe mit anderen Größen oder aus anderen Materialien in weiteren Lagerbereichen zusätzlich gelagert sein. Für die bei der Dialysebehandlung üblicherweise erforderliche Kanülierung sind in einem Lagerbereich Kanülen, insbesondere Kanülen eines bestimmten Typs, und in einem weiteren Lagerbereich Infusionsschläuche gelagert. Des Weiteren können dazu in weiteren Lagerbereichen Seldinger-Drähte, Katheter und/oder Klebebänder zum Befestigen der Kanüle oder des Katheters gelagert sein. Schließlich können für die Nachbehandlung in weiteren Lagerbereichen Verbandmaterial, weitere Tupfer - sofern für die Nachbehandlung andere Tupfer als für die Vorbereitung verwendet werden sollen -, Pflaster und/oder Kühlkissen gelagert sein. Dabei versteht es sich, dass, sofern bei den Zubehörgattungen insbesondere zwischen verschiedenen Typen, Größen oder Materialien von einer Art von medizinischen Zubehören, etwa Kanülen oder Einmalhandschuhe, differenziert wird, in weiteren Lagerbereichen des Behandlungswagens jeweils medizinische Zubehöre einer solchen weiteren, insbesondere differenzierten, Zubehörgattung gelagert sein können.

Vorzugsweise wird ein Behandlungswagen mit zumindest einem Teil der Mehrzahl an medizinischen Zubehören an einem Ausgangsort, insbesondere von einem Bestückungssystem, bestückt.

Für den Transport der medizinischen Zubehöre weist der Behandlungswagen vorzugsweise eine Fahrvorrichtung, insbesondere Räder oder Rollen, auf oder ist zumindest dafür vorgesehen, mittels einer Fahrvorrichtung für den Transport eingerichtet und dazu insbesondere mit der Fahrvorrichtung verbunden zu werden.

Schließlich weist ein Behandlungswagen räumliche Abmessungen und ein Gewicht auf, welche ein Bewegen des Behandlungswagens in einem Gebäude, insbesondere einem Krankenhaus, einer ambulanten Pflegestation oder einem Wohngebäude eines Patienten - etwa für die Heimdialyse -, ermöglichen. So beträgt insbesondere das Gewicht eines solchen, insbesondere unbestückten, Behandlungswagens höchstens 300kg, vorzugsweise höchstens 100kg, vorzugsweise höchstens 50kg und weiter bevorzugt höchstens 20kg. Dabei kann sich das Gewicht des Behandlungswagens aufgrund seiner Bestückung erhöhen. Die räumlichen Abmessungen eines solchen Behandlungswagens haben insbesondere eine Länge von höchstens 2,5m, vorzugsweise höchstens 1,5m, vorzugsweise höchstens 1m weiter bevorzugt höchstens 0,5 m, wobei entsprechendes für die Breite und Höhe gilt. Dabei versteht es sich insbesondere, dass ein solcher Behandlungswagen eingerichtet sein kann, für den Transport der medizinischen Zubehöre bzw. für das Bewegen des Behandlungswagens in einen Transportzustand bringbar zu sein, welcher geringere räumliche Abmessungen aufweist, als ein Zustand zum Bereitstellen der medizinischen Zubehöre.

Im Sinne der Erfindung ist ein "Herausgabemechanismus" ein Mechanismus, welcher eingerichtet ist, eine oder mehrere Sachen, insbesondere ein medizinisches Zubehör, herauszugeben. Vorzugsweise ist die herauszugebende Sache dabei zunächst bei einer weiteren Vorrichtung angeordnet und lagert insbesondere in einem Bereich der weiteren Vorrichtung. Vorzugsweise ist der Herausgabemechanismus eingerichtet, die Herausgabe der herauszugebenden Sache gemäß einer Steuerung auszuführen.

Zum Herausgeben bewirkt der Herausgabemechanismus dabei insbesondere eine Kraft auf einen Teil des Herausgabemechanismus, auf die herauszugebende Sache und/oder auf einen Teil der weiteren Vorrichtung. Auch kann der Herausgabemechanismus zum Herausgeben vorzugsweise die herauszugebende Sache, einen Teil des Herausgabemechanismus und/oder einen Teil der weiteren Vorrichtung so bewegen, dass die Sache herausgegeben wird, also insbesondere die herauszugebende Sache zu einem Herausgabebereich bewegt wird und/oder ein Bereich, in welchem sich die herauszugebende Sache befindet, zugänglich gemacht wird. Ein solcher Herausgabemechanismus kann insbesondere eingerichtet sein, ein Schubfach, in welchem die herauszugebende Sache lagert, zu öffnen - d.h. insbesondere herauszufahren -, die herauszugebende Sache auszusondern, d.h. insbesondere aus einem Bereich zu entnehmen und in den Herausgabebereich abzugeben - und/oder eine Schließeinrichtung der weiteren Vorrichtung, welche einen Bereich, in dem die herauszugebende Sache angeordnet ist, vor der Umgebung der weiteren Vorrichtung verschließt, zu öffnen - d.h. insbesondere einen Verschluss, eine Klappe oder eine Türe der weiteren Vorrichtung zu öffnen und somit einen Innenbereich der weiteren Vorrichtung, in welchem die herauszugebende Sache angeordnet ist, zugänglich zu machen.

Vorzugsweise und alternativ oder zusätzlich ist der Herausgabemechanismus eingerichtet, gemäß der Steuerung den Bereich, in welchem die auszusondernde Sache lagert, anzuzeigen. Für diese Anzeige weist der Herausgabemechanismus vorzugsweise eine Benutzerschnittstelle, insbesondere zur optischen Ausgabe, auf und/oder ist eingerichtet an eine solche Benutzerschnittstelle Daten zu senden, so dass die Benutzerschnittstelle die weitere Vorrichtung und/oder den Bereich, in welchem die auszusondernde Sache lagert, anzeigt. Zum Herausgeben kann der Herausgabemechanismus lediglich die Anzeige bewirken und eine Bewegung der auszusondernden Sache bzw. einen Zugang zu der auszusondernden Sache erlauben oder zusätzlich zur Anzeige die auszusondernde Sache, wie oben beschrieben, bewegen oder zugänglich machen. So kann etwa der Herausgabemechanismus vorzugsweise einen Teil der weiteren Vorrichtung mit dem Bereich, in welchem die auszusondernde Sache lagert, beweglich lagern sowie mehrere Signalleuchten, etwa LEDs, aufweisen, mittels derer der Herausgabemechanismus die weitere Vorrichtung, in welcher die auszusondernde Sache lagert, sowie den Bereich innerhalb dieser Vorrichtung, in welchem die auszusondernde Sache lagert, anzeigt, wobei die Signalleuchten insbesondere bei der jeweiligen Vorrichtung bzw. dem jeweiligen Bereich angeordnet sein können.

Das mobile Auswahlsystem ermöglicht medizinische Zubehöre auf Basis der durchzuführenden Behandlung auszuwählen und dabei insbesondere automatisiert herauszugeben, womit auf vorteilhafte Weise insbesondere die an der Behandlung beteiligten Personen unterstützt und damit das Personal entlastet und/oder Fehlerquellen, etwa menschliches Versagen, reduziert und so insbesondere die Behandlungssicherheit gesteigert werden kann.

Auch lässt sich der Behandlungswagen mit einer Mehrzahl an medizinischen Zubehören bestücken, welche für eine bestimmte Behandlung oder eine bestimmte Gruppe von Behandlungen, insbesondere Dialysebehandlungen oder eine Kanülierung von Blutgefäßen, geeignet und insbesondere erforderlich sind. Auf diese vorteilhafte Weise kann der Behandlungswagen und damit das mobile Auswahlsystem an einen wechselnden Bedarf an bestimmten Behandlungen angepasst werden. Dabei wird neben dem physischen Bestücken insbesondere auch die Zubehördatenbank so angepasst - also insbesondere Datensätze über medizinische Zubehöre neu angelegt, erweitert, verändert oder gelöscht -, dass die Zubehördatenbank Datensätze zumindest über einen Teil und vorzugsweise über alle medizinischen Zubehöre der Mehrzahl an medizinischen Zubehören, mit welcher der Behandlungswagen bestückt ist, aufweist. Ein solcher Behandlungswagen erlaubt es somit diesen selbst und/oder die medizinischen Zubehöre effizient für verschiedene Behandlungen zu nutzen.

Ein Vorteil der Lagerung, Bereitstellung und/oder Herausgabe der medizinischen Zubehöre mittels des Behandlungswagens kann insbesondere darin liegen, dass die medizinischen Zubehöre an verschiedenen Behandlungsorten transportiert und dort herausgegeben werden können. Insbesondere gegenüber einer festen Zuordnung von Behandlungsorten und dort verfügbaren medizinischen Zubehören erlaubt dies eine Flexibilisierung der Orte, an denen eine Behandlung durchgeführt und/oder ein Patient behandelt werden kann. Auch können die einzelnen medizinischen Zubehöre für eine bestimmte Behandlung oder eine bestimmte Gruppe von Behandlungen durch den mit diesen Zubehören bestückten Behandlungswagen zu einer logistischen Einheit zusammengefasst werden, womit insbesondere der Transport zu einem Behandlungsort, an dem diese bestimmte Behandlung durchgeführt werden soll, vereinfacht werden kann und/oder die Sicherheit darüber erhöht werden kann, dass einzelne für die Behandlung erforderliche medizinische Zubehöre am Behandlungsort bei der Behandlung auch jeweils tatsächlich bereitstehen.

Aus der Auswahl der für die durchzuführende Behandlung geeigneten medizinischen Zubehöre anhand der Zubehördatenbank kann sich, insbesondere bei einer Vielzahl an medizinischen Zubehören, Patienten und/oder Behandlungen, als weiterer Vorteil ergeben, dass ein - insbesondere besonders - geeignetes medizinisches Zubehör ausgewählt und so die Sicherheit und/oder Qualität der Behandlung gesteigert und/oder medizinische Zubehöre behandlungsspezifisch und/oder effizient genutzt werden können.

Auch kann ein Vorteil der automatisierten Auswahl an für die durchzuführende Behandlung geeigneten medizinischen Zubehören anhand der Zubehördatenbank und insbesondere der automatisierten Herausgabe mittels des Herausgabemechanismus insbesondere darin liegen, dass das Ausführen der durchzuführenden Behandlung vereinfacht, die an der Behandlung beteiligten Personen entlastet und/oder Fehlerquellen reduziert werden können. Auf diese vorteilhafte Weise wird es überdies ermöglicht, dass Patienten einfache Behandlungsschritte, für welche ihnen die jeweils benötigten medizinischen Zubehöre - und insbesondere nur diese - ausgewählt und vorzugsweise herausgegeben werden, selbst durchführen können, wodurch das Personal entlastet werden kann oder auch aufgrund der aktiven Teilhabe der Patienten die Patientenzufriedenheit gesteigert werden kann.

Schließlich kann die Handhabung eines solchen Behandlungswagen und der medizinischen Zubehöre gegenüber einem herkömmlichen Behandlungswagen mit medizinischen Zubehören vereinfacht und insbesondere mittels der automatisierten Herausgabe der Kontakt zu Betätigungselementen für die Herausgabe - welche herkömmliche Behandlungswagen üblicherweise aufweisen - reduziert werden, wodurch insbesondere die Hygiene und/oder die Arbeitseffizienz gesteigert werden kann.

Gemäß einer bevorzugten Ausführungsform weist das mobile Auswahlsystem genau den einen Behandlungswagen auf und ein Teil der Bestandteile des mobilen Auswahlsystems - insbesondere alle - sind in einer physischen Geräteeinheit integriert.

Gemäß einer bevorzugten Ausführungsform ist ein Bestandteil des mobilen Auswahlsystems oder sind vorzugsweise mehrere Bestandteile des mobilen Auswahlsystems jeweils eigene physische Geräteeinheiten. Vorzugsweise sind wenigstens zwei der Bestandteile des mobilen Auswahlsystems an geographisch unterschiedlichen Positionen aufgestellt. Dabei ist vorzugsweise die Datenspeichervorrichtung für die Zubehördatenbank an einem geographisch anderen Ort als der Behandlungswagen positioniert.

Gemäß einer bevorzugten Ausführungsform des mobilen Auswahlsystems weist dieses wenigstens einen weiteren Behandlungswagen auf. Dabei gelten die möglichen Vorteile sowie Ausführungsformen, Weiterbildungen oder Varianten des Behandlungswagens des mobilen Auswahlsystems entsprechend für den wenigstens einen weiteren Behandlungswagen. Auf diese vorteilhafte Weise lassen sich mehrere Behandlungsorte gleichzeitig mit dem mobilen Auswahlsystem versorgen. Auch kann einer der Behandlungswagen für eine bestimmte Behandlung oder bestimmte Gruppe von Behandlungen bestückt sein und ein anderer der Behandlungswagen für eine andere bestimmte Behandlung oder eine andere bestimmte Gruppe von Behandlungen bestückt sein. Auf diese vorteilhafte Weise kann, insbesondere auch bei einer begrenzten Lagerkapazität der einzelnen Behandlungswagen, eine Vielzahl an medizinischen Zubehören für wenigstens zwei Behandlungen und vorzugsweise für eine Vielzahl an Behandlungen bereitgestellt werden.

Gemäß einer bevorzugten Ausführungsform des mobilen Auswahlsystems ist das Steuerungssystem eingerichtet, der Benutzerschnittstelle einen der wagenBehandlungswagen des mobilen Auswahlsystems zuzuordnen. Insbesondere können einer oder mehrere der Behandlungswagen je eine Verbindungseinrichtung für Benutzerschnittstellen aufweisen, welche für eine, insbesondere mechanische, Verbindung der Benutzerschnittstelle mit dem jeweiligen Behandlungswagen eingerichtet ist. Vorzugsweise kann dabei die Zuordnung zu einem der Behandlungswagen durch das Verbinden der Benutzerschnittstelle mit dem jeweiligen Behandlungswagen festgelegt werden, insbesondere indem die Benutzerschnittstelle oder der Behandlungswagen eine Sensoreinrichtung zur Erkennung des Behandlungswagens bzw. der Benutzerschnittstelle aufweist. Zusätzlich oder alternativ kann die Zuordnung zu einem der Behandlungswagen vorzugsweise mittels einer Eingabe von dem Benutzer an der Benutzerschnittstelle festgelegt werden. Auf diese vorteilhafte Weise kann die Benutzerschnittstelle einem Behandlungswagen des mobilen Auswahlsystems zugeordnet werden und es lassen sich insbesondere mehrere Behandlungswagen des mobilen Auswahlsystems durch Wechseln der Zuordnung zu dem jeweiligen Behandlungswagen abwechselnd bedienen. So können insbesondere zwei Behandlungswagen, die jeweils für eine Teilbehandlung einer Behandlung bestückt sind, mit der Benutzerschnittstelle gemeinsam bedient werden. Bevorzugt kann das mobile Auswahlsystem auch wenigstens eine weitere Benutzerschnittstelle für Eingaben zur Festlegung der durchzuführenden Behandlung aufweisen. Dabei gelten die möglichen Vorteile sowie Ausführungsformen, Weiterbildungen oder Varianten der Benutzerschnittstelle des mobilen Auswahlsystems entsprechend für die wenigstens eine weitere Benutzerschnittstelle für Eingaben zur Festlegung der durchzuführenden Behandlung. Insbesondere kann das mobile Auswahlsystem wenigstens so viele derartige Benutzerschnittstellen aufweisen, wie es Behandlungswagen aufweist. Ein Vorteil der Mehrzahl an Benutzerschnittstellen für Eingaben zur Festlegung der durchzuführenden Behandlung kann insbesondere darin liegen, dass die Bedienung des Behandlungswagen bzw. der Behandlungswagen an den jeweiligen Bedarf angepasst werden kann.

Gemäß einer bevorzugten Ausführungsform ist der Behandlungswagen modular aufgebaut. Dazu weist der Behandlungswagen eine Verbindungsvorrichtung für Behandlungswagenmodule. Dabei ist ein Behandlungswagenmodul für die Verbindung mit dem Behandlungswagen mittels der Verbindungsvorrichtung eingerichtet, insbesondere geformt, und/oder die Verbindungsvorrichtung ist eingerichtet, eine Verbindung mit einem oder mehreren Behandlungswagenmodulen auszubilden, insbesondere auszuformen. Vorzugsweise weist die Verbindungsvorrichtung eine oder mehrere Verbindungseinrichtungen jeweils zur Verbindung mit einem Behandlungswagenmodul auf. Weiter bevorzugt weist wenigstens eine der Verbindungseinrichtungen eine Aufnahmeeinrichtung auf oder besteht daraus, wobei die Aufnahmeeinrichtung eingerichtet ist, ein Behandlungswagenmodul - insbesondere ein Behandlungswagenmodul, welches mit dieser Aufnahmeeinrichtung korrespondiert - ganz oder zumindest teilweise aufzunehmen und damit die Verbindung zu dem Behandlungswagen auszubilden.

Außerdem weist der Behandlungswagen vorzugsweise eine oder mehrere der folgenden, insbesondere zuvor und/oder nachfolgend weiter beschriebenen, Behandlungswagenmodule auf: eine Lagereinrichtung - insbesondere ein Schubfach, ein Schrankfach, eine Dosiervorrichtung, etwa ein Klebeband-Abroller und/oder eine Desinfektionsmittelabgabeeinrichtung, ein Rollenhalter für Klebebandrollen oder für eine Rolle mit vorportionierten, einzelverpackten Pflastern und/oder eine Handschuhbox -, eine Benutzerschnittstelle - insbesondere eine Benutzerschnittstelle für Eingaben zur Festlegung der durchzuführenden Behandlung, eine Benutzerschnittstelle für Eingaben zur Festlegung eines durchzuführenden Behandlungsschrittes, eine Benutzerschnittstelle zur Ausgabe, etwa von Patientendaten, Behandlungsdaten oder Daten bezüglich eines tatsächlichen Bestands medizinischen Zubehör, und/oder eine Benutzerschnittstelle zur Steuerung des Behandlungswagens -, eine Ansteuerungsvorrichtung, ein Lesegerät - insbesondere für Patientendaten, Behandlungsdaten, Benutzerdaten, RFID-Kennungen und/oder Barcodes -, eine Entsorgungsvorrichtung, eine Ablageeinrichtung mit Ablagefläche, ein Kamera-Modul und/oder Laborzubehör. Weitere mögliche und bevorzugte Behandlungswagenmodule sind an anderer Stelle beschrieben.

Vorzugsweise weist das mobile Auswahlsystem, insbesondere das Steuerungssystem und/oder der Behandlungswagen, eine Zuordnungsvorschrift für Behandlungswagenmodule und eine Datenspeichervorrichtung für diese auf, wobei die Zuordnungsvorschrift für Behandlungswagenmodule eingerichtet ist, einem Behandlungswagenmodul einen Behandlungswagen zuzuordnen anhand von Zuordnungsdaten, welche auf der Datenspeichervorrichtung für diese Zuordnungsvorschrift gespeichert sind. Weiter bevorzugt kann dabei die Zuordnung des Behandlungswagenmoduls zu einem der Behandlungswagen durch das Verbinden des Behandlungswagenmoduls mit dem jeweiligen Behandlungswagen festgelegt werden, insbesondere indem das Behandlungswagenmodul oder der Behandlungswagen eine Sensoreinrichtung zur Erkennung des Behandlungswagens bzw. des Behandlungswagenmoduls aufweist und bei Erkennung entsprechende Zuordnungsdaten in die Datenspeichervorrichtung für die Zuordnungsvorschrift für Behandlungswagenmodule geschrieben werden. Aufgrund dieser Zuordnung, welche insbesondere mittels der Erkennung automatisiert werden kann, wird es ermöglicht, dass das mobile Auswahlsystem und insbesondere das Steuerungssystem medizinische Zubehöre auch auf Basis dieser Zuordnung auswählt und/oder eine Steuerung auf Basis dieser Zuordnung ausführt und insbesondere den wenigstens einen Herausgabemechanismus des Behandlungswagens auf Basis dieser Zuordnung steuert. So kann etwa, sofern der Behandlungswagen als Behandlungswagenmodul eine Dosiervorrichtung für Klebeband aufweist, ein Klebeband-Abschnitt von einem Klebeband ausgewählt und die Dosiervorrichtung für Klebeband zur Herausgabe eines solchen Klebeband-Abschnitts angesteuert werden und andernfalls ein vorab zugeschnittener, einzeln verpackter Klebebandabschnitt, welcher etwa in einem Schubfach des Behandlungswagens gelagert ist, ausgewählt werden und der wenigstens eine Herausgabemechanismus angesteuert werden, dieses Schubfach zu öffnen.

Gemäß einer bevorzugten Ausführungsform weist wenigstens eine erste der Lagereinrichtungen des Behandlungswagens ein Schubfach, insbesondere eine Schublade, auf oder besteht daraus, wobei das Schubfach genau einen Lagerbereich für eine Zubehörgattung aus der Mehrzahl an medizinischen Zubehören aufweist. Dabei kann in einer alternativen Variante das Schubfach auch in mehrere Lagerbereiche für verschiedene Zubehörgattungen unterteilt sein. Zudem weist wenigstens eine zweite der Lagereinrichtung des Behandlungswagens ein Schubfach oder ein Schrankfach auf oder besteht daraus. Dieses kann wiederum genau einen Lagerbereich für eine Zubehörgattung oder alternativ mehrere Lagerbereiche für verschiedene Zubehörgattungen aufweisen. Hierbei weist der Behandlungswagen insgesamt wenigstens zwei Lagerbereiche je für eine Zubehörgattung auf, so dass eine Lagerung und damit Auswahl zwischen den medizinischen Zubehören der beiden Zubehörgattungen ermöglicht wird. Nach dem Verständnis dieser Ausführungsform besteht die Mehrzahl an medizinischen Zubehören dabei nur aus den Zubehörgattungen, für welche die erste oder die zweite Lagereinrichtung Lagerbereiche aufweist, oder alternativ der Behandlungswagen weist noch weitere Lagereinrichtungen für die weiteren Zubehörgattungen der Mehrzahl an medizinischen Zubehören, die der ersten oder der zweiten Lagereinrichtung entsprechen, auf und/oder bei einem mobilen Auswahlsystem mit mehreren Behandlungswagen oder zusätzlichen Lagerbereichen, die nicht Teil eines Behandlungswagens sind, ist jedem der Behandlungswagen oder zusätzlichen Lagerbereichen ein Teil aus der Mehrzahl an medizinischen Zubehören mittels der Zubehördatenbank zugeordnet und dort jeweils entsprechend gelagert.

In einer bevorzugten voll-automatisierten Variante weist der Behandlungswagen einen weiteren Herausgabemechanismus auf, wobei der wenigstens eine Herausgabemechanismus zur Herausgabe von medizinischen Zubehören aus dem Schubfach der ersten Lagereinrichtung und der weitere Herausgabemechanismus zur Herausgabe von medizinischen Zubehören aus dem Schubfach bzw. Schrankfach der zweiten Lagereinrichtung eingerichtet ist. Auf diese vorteilhafte Weise können die medizinischen Zubehöre aller Zubehörgattungen, die in dem Behandlungswagen gelagert sind, automatisch herausgegeben werden.

Gemäß einer alternativen und bevorzugten semi-automatisierten Variante ist der Behandlungswagen nicht eingerichtet, medizinischen Zubehöre aus der zweiten Lagereinrichtung automatisiert herauszugeben, und weist insbesondere nicht den weiteren Herausgabemechanismus auf. Auf diese vorteilhafte Weise können die medizinischen Zubehöre der Zubehörgattungen, welche in der ersten Lagereinrichtung gelagert sind, automatisch herausgegeben werden, während die medizinischen Zubehöre der Zubehörgattungen, welche in der zweiten Lagereinrichtung gelagert sind, nicht automatisiert und/oder unabhängig von der Steuerung durch das Steuerungssystem entnommen werden können. Dies kann die technische Umsetzung und/oder die Handhabung des Behandlungswagens und damit des mobilen Auswahlsystems vereinfachen. So kann insbesondere das medizinische Zubehör einer Zubehörgattung, welches nur gemäß der Auswahl herausgegeben werden soll - etwa Kanülen -, in der ersten Lagereinrichtung gelagert werden, während medizinische Zubehöre anderer Zubehörgattungen - etwa Desinfektionsmittel, Tupfer, Pflaster und/oder Einmalhandschuhe - in der zweiten Lagereinrichtung gelagert werden. Auch lässt sich zusammen mit einer Benutzerautorisierung der Zugriff auf kritische medizinische Zubehöre, wie etwa Kanülen, auf autorisierte Benutzer einschränken, während andere medizinische Zubehöre, wie etwa Desinfektionsmittel, Tupfer, Pflaster und/oder Einmalhandschuhe, allgemein bereitgestellt werden.

Vorzugsweise kann wenigstens einer der Herausgabemechanismen einen motorisierten Seilzug aufweisen oder daraus bestehen, mit welchem das Schubfach zur Herausgabe von medizinischen Zubehören herausgefahren und anschließend wieder hereingefahren werden kann.

Gemäß einer bevorzugten Ausführungsform weist der Behandlungswagen eine, insbesondere nur eine, Lagereinrichtung mit wenigstens zwei Lagerbereichen je für eine Zubehörgattung auf. Insbesondere kann diese Lagereinrichtung ein Schubfach aufweisen oder daraus bestehen, welches in wenigstens zwei Lagerbereiche unterteilt ist.

Ein Vorteil einer Bündelung von mehreren Lagerbereichen in einer Lagereinrichtung, insbesondere einem Schubfach, kann insbesondere darin liegen, dass die Anzahl der Lagereinrichtungen und damit insbesondere die Komplexität des Herausgabemechanismus und/oder die Anzahl der für die Herausgabe eines Zubehörsets erforderlichen Schritte, insbesondere Bewegungen, reduziert werden können. Insbesondere können so durch Herausfahren eines Schubfachs medizinische Zubehöre eines Teils, vorzugsweise aller, Zubehörgattungen, welche in diesem Schubfach gelagert sind, herausgegeben werden.

Ein Vorteil einer Aufteilung von mehreren Lagerbereichen auf mehrere Lagereinrichtungen, vorzugsweise auf mehrere Schubfächer, kann insbesondere darin liegen, dass es ermöglicht wird, zur Herausgabe der medizinischen Zubehöre nur die medizinischen Zubehöre des Zubehörsets zugänglich zu machen und insbesondere nur jene Schubfächer herauszufahren, welche Lagerbereiche für die Zubehörgattungen der medizinischen Zubehöre des Zubehörsets aufweisen.

Gemäß einer bevorzugten Ausführungsform des Behandlungswagens, bei welcher der Behandlungswagen wenigstens ein Schubfach aufweist und eines der Schubfächer wenigstens zwei Lagerbereiche aufweist, kann das Steuerungssystem eingerichtet sein, den Herausgabemechanismus für dieses Schubfach so zu steuern, dass der Herausgabemechanismus das Schubfach nur soweit aus einer Aufnahmeeinrichtung für das Schubfach heraus fährt, dass derjenige der wenigstens zwei Lagerbereiche, in dem medizinische Zubehöre des Zubehörsets gelagert sind, aus der Aufnahmeeinrichtung herausgefahren wird, während weitere bezüglich der Bewegungsrichtung beim Herausfahren hinter diesem Lagerbereich angeordnete Lagerbereiche der wenigstens zwei Lagerbereiche nicht aus der Aufnahmeeinrichtung herausgefahren werden. Auf diese vorteilhafte Weise lässt sich einerseits der Zugriff auf medizinische Zubehöre einschränken und andererseits die Anzahl der Lagereinrichtungen, welche zum Lagern für eine bestimmte Anzahl an Zubehörgattungen benötigt werden, reduzieren.

Gemäß einer bevorzugten Ausführungsform weist der Behandlungswagen eine desinfizierbare Ablagefläche auf. Insbesondere kann die Ablagefläche einen Herausgabebereich aufweisen oder daraus bestehen. Ein Vorteil der Ablagefläche kann insbesondere darin liegen, dass vor der Behandlung zumindest ein Teil oder vorzugsweise alle medizinischen Zubehöre des Zubehörsets auf der Ablagefläche abgelegt werden können, womit sie für die Behandlung bereitstehen, ohne dass während der Behandlung eine Herausgabe und/oder eine Betätigung zur Herausgabe und/oder Entnahme eines solchen medizinischen Zubehörs erforderlich ist. Dies kann insbesondere die Hygiene verbessern.

Auch kann bei einer Ausführungsform mit Ablagefläche der wenigstens eine Herausgabemechanismus oder ein weiterer Herausgabemechanismus des Behandlungswagens eine Aussonderungsvorrichtung aufweisen oder daraus bestehen, welche für wenigstens eine der Lagereinrichtungen eingerichtet ist, jene medizinische Zubehöre, für deren Zubehörgattungen diese Lagereinrichtung Lagerbereiche aufweist und welche durch die Zubehörsetparameter, auf denen die Steuerung basiert, gekennzeichnet sind, aus den jeweiligen Lagerbereichen zu entnehmen und auf die Ablagefläche bzw. an dem Herausgabebereich abzugeben. Auf diese vorteilhafte Weise können diese medizinischen Zubehöre aus den jeweiligen Lagerbereichen ausgesondert werden und insbesondere so bereitgestellt werden, ohne dass der Lagerbereich für die jeweilige Zubehörgattung zugänglich gemacht werden muss, und/oder, sodass, insbesondere während der Behandlung, keine Betätigung zur Herausgabe und/oder manuelle Entnahme erforderlich ist. Dies kann insbesondere die Hygiene verbessern. Dabei kann das Aussondern vor der Behandlung erfolgen. Auch kann das Aussondern während der Behandlung und vorzugsweise in Abhängigkeit von einem jeweils durchzuführenden Behandlungsschritt der Behandlung erfolgen. Insbesondere kann eine derartige auf die Behandlungsschritte abgestimmte Aussonderung der jeweils erforderlichen medizinischen Zubehöre das Durchführen der durchzuführenden Behandlung weiter vereinfachen.

Gemäß einer bevorzugten Ausführungsform ist das mobile Auswahlsystem und insbesondere der Behandlungswagen bzw. einer der Behandlungswagen für eine Assistenzfunktion eingerichtet. Eine solche Assistenzfunktion unterstützt einen Benutzer, insbesondere den Benutzer des Behandlungswagens und/oder eine an der Behandlung beteiligte Person, bei der Durchführung der durchzuführenden Behandlung und speziell, wenn die durchzuführende Behandlung mehrere Behandlungsschritte und/oder Teilbehandlungen aufweist, bei der jeweiligen Durchführung der Behandlungsschritte.

Insbesondere ist dazu das mobile Auswahlsystem, vorzugsweise das Steuerungssystem, eingerichtet, die jeweils durchzuführende Behandlung und speziell den jeweils durchzuführenden Behandlungsschritt zu bestimmen. Zur Bestimmung des durchzuführenden Behandlungsschritts kann in einer Variante das mobile Auswahlsystem eine Benutzerschnittstelle aufweisen, die für eine oder mehrere Eingaben von dem Benutzer eingerichtet ist, durch die die durchzuführende Behandlung und speziell der durchzuführende Behandlungsschritt festgelegt wird und auf Basis derer das Steuerungssystem infolge dieser Eingaben die Behandlungskennung der durchzuführenden Behandlung und speziell eine dem durchzuführenden Behandlungsschritt zugeordnete Behandlungsschrittkennung bestimmt. In einer bevorzugten zusätzlichen oder alternativen Variante kann zur Bestimmung des durchzuführenden Behandlungsschritts das mobile Auswahlsystem, insbesondere einer der Behandlungswagen, ein Erkennungssystem für Behandlungen aufweisen, das mittels einer auf der Messung von Strahlung, insbesondere Licht, von Ultraschall, von Gewicht und/oder von Trägheit basierenden Sensorik eine an der Behandlung beteiligte Person, den Patienten, ein medizinisches Zubehör, insbesondere des Zubehörsets, und/oder deren Anordnung und/oder Bewegung zueinander erfasst und basierend darauf die durchzuführende Behandlung und/oder den als nächstes durchzuführenden Behandlungsschritt bestimmt.

Vorzugsweise ist für die Assistenzfunktion eine der Benutzerschnittstellen des mobilen Auswahlsystems eingerichtet, Assistenzinformationen auszugeben, oder das mobile Auswahlsystem weist eine zusätzliche, dazu eingerichtete Benutzerschnittstelle auf. Vorzugsweise weist einer der Behandlungswagen diese Benutzerschnittstelle auf, wobei diese insbesondere als Behandlungswagenmodul ausgebildet sein kann. Solche Assistenzinformationen können insbesondere akustische oder visuelle Instruktionen über die durchzuführende Behandlung, die Schritte der Behandlung, den durchzuführenden Behandlungsschritt und/oder die medizinischen Zubehöre sein und/oder darüber sein, wie die Behandlung oder der Behandlungsschritt jeweils durchzuführen ist und/oder wie ein bestimmtes medizinisches Zubehör, insbesondere für die Behandlung oder den Behandlungsschritt, zu verwenden ist. Vorzugsweise weist das Steuerungssystem eine Datenspeichervorrichtung für Assistenzinformationen auf und ist eingerichtet ein Assistenzverfahren auszuführen, welches wenigstens die folgenden Verfahrensschritte aufweist. In einem Verfahrensschritt des Assistenzverfahrens werden die durchzuführende Behandlung, der durchzuführende Behandlungsschritt und/oder ein medizinisches Zubehör dafür mittels des Erkennungssystems für Behandlungen bestimmt. In einem weiteren Verfahrensschritt des Assistenzverfahrens werden damit korrespondierende Assistenzinformationen aus der Datenspeichervorrichtung für Assistenzinformationen gelesen und an der Benutzerschnittstelle für Assistenzfunktionen ausgegeben.

Auch vorzugsweise kann das Steuerungssystem eingerichtet sein, ein Assistenzverfahren auszuführen, welches wenigstens den Verfahrensschritt aufweist: Bestimmen eines oder mehrere Assistenzparameter, welche die durchzuführende Behandlung, den durchzuführenden Behandlungsschritt und/oder ein medizinisches Zubehör dafür kennzeichnen, mittels des Erkennungssystems für Behandlungen. Zudem ist in einer bevorzugten Variante das Steuerungssystem eingerichtet, den wenigstens einen Herausgabemechanismus auf Basis der Zubehörsetparameter und der Assistenzparameter so zu steuern, dass jene medizinischen Zubehöre des Zubehörsets herausgegeben werden, welche für den durchzuführenden Behandlungsschritt erforderlich sind und/oder welche nach dem aktuell verwendeten medizinischen Zubehör zu verwenden sind. In einer bevorzugten alternativen oder zusätzlichen Variante ist der wenigstens eine Herausgabemechanismus eingerichtet, die medizinischen Zubehöre abhängig von den Assistenzparametern herauszugeben.

Insbesondere nachfolgend werden bevorzugte Weiterbildungen des mobilen Auswahlsystems und seiner Komponenten beschrieben, die jeweils, soweit dies technisch möglich ist und nicht ausdrücklich ausgeschlossen wird, nach Wunsch des Fachmanns miteinander kombiniert werden können. Entsprechend können die bevorzugten Weiterbildungen insbesondere auch mit bevorzugten Ausführungsformen kombiniert werden.

Gemäß einer bevorzugten Weiterbildung weist eine der Lagereinrichtungen ein Schubfach, insbesondere eine Schublade, mit einem oder mehreren Lagerbereichen je für eine Zubehörgattung aus der Mehrzahl an medizinischen Zubehören auf oder besteht daraus. Zudem ist der wenigstens eine Herausgabemechanismus oder ein weiterer Herausgabemechanismus des Behandlungswagens für diese Lagereinrichtung eingerichtet, das Schubfach zu öffnen. Dazu weist in einer bevorzugten Variante der Herausgabemechanismus, das Schubfach oder die Lagereinrichtung eine Verriegelungseinrichtung für das Schubfach, vorzugsweise etwa ein Riegel und ein Gegenstück oder eine magnetische Verriegelung, auf und der Herausgabemechanismus ist eingerichtet, die Verriegelungseinrichtung zu entriegeln. In einer bevorzugten zusätzlichen oder alternativen Variante ist der Herausgabemechanismus eingerichtet, das Schubfach, etwa mittels eines motorisierten Seilzugs oder Gewindes oder nach Entriegelung mittels einer Lagerung das Schubfach in Zusammenwirkung mit der Schwerkraft, herauszufahren.

Ein Vorteil des Schubfachs kann insbesondere darin liegen, dass verschiedene medizinische Zubehöre, insbesondere sortiert nach ihren Zubehörgattungen, in den Lagerbereichen des Schubfachs gelagert werden können und nach dem Herausgeben aus den Lagerbereichen, insbesondere gezielt von einem Benutzer, entnommen werden können. Gegenüber einem bloßen Anzeigen, wo das herauszugebende Zubehör gelagert ist, und/oder einem Öffnen des Schubfachs ohne Herausfahren lässt sich durch die Herausgabe mittels des Herausfahrens des Schubfachs die Auswahl der medizinischen Zubehöre für die durchzuführende Behandlung verbessern und insbesondere der Benutzungskomfort, die Hygiene und/oder die Arbeitseffizienz steigern, da zusätzliche Arbeitsschritte des Benutzers zum Auswählen der medizinischen Zubehöre und/oder zum Öffnen des Schubfachs vermieden werden können.

Ein Vorteil einer Variante, bei welcher die Lagereinrichtung genau einen Lagerbereich für medizinisches Zubehör genau einer Zubehörgattung aufweist, kann insbesondere darin liegen, dass beim Öffnen des Schubfachs nur medizinisches Zubehör dieser Zubehörgattung zugänglich gemacht wird, womit insbesondere die Behandlungssicherheit erhöht und/oder die gezielte Entnahme eines geeigneten medizinischen Zubehörs vereinfacht und/oder beschleunigt werden kann.

Ein Vorteil einer Variante, bei welcher die Lagereinrichtungen wenigstens zwei Lagerbereiche aufweist, kann insbesondere darin liegen, dass die Anzahl der Lagereinrichtungen und damit insbesondere die Komplexität des Herausgabemechanismus und/oder die Anzahl der für die Herausgabe eines Zubehörsets erforderlichen Arbeitsschritte, insbesondere Bewegungen, reduziert werden können.

Gemäß einer bevorzugten Weiterbildung weist eine der Lagereinrichtungen ein Abdeckungssystem für wenigstens einen Lagerbereich der Lagereinrichtung auf. Das Abdeckungssystem ist eingerichtet, bei der Herausgabe von medizinischen Zubehören aus der Lagereinrichtung diesen Lagerbereich abzudecken, sofern kein medizinisches Zubehör aus diesem Lagerbereich herausgegeben werden soll, und, andernfalls, diesem Lagerbereich freizugeben. Gemäß einer bevorzugten Variante weist das Abdeckungssystem ein Abdeckungselement für diesen Lagerbereich auf. Gemäß einer bevorzugten und alternativen oder zusätzlichen Variante, bei welcher die Lagereinrichtung ein Schubfach mit wenigstens zwei Lagerbereichen aufweist, ist eine Einfassung oder Aufnahmeeinrichtung für das Schubfach ein Bestandteil des Abdeckungssystems und das Steuerungssystem eingerichtet, das Schubfach nur soweit aus der Einfassung oder Aufnahmeeinrichtung herauszufahren, dass derjenige der wenigstens zwei Lagerbereiche, in dem das herauszugebende medizinische Zubehör gelagert ist, herausgefahren wird, während weitere bezüglich der Bewegungsrichtung beim Herausfahren hinter diesem Lagerbereich angeordnete Lagerbereiche nicht aus der Einfassung bzw. Aufnahmeeinrichtung herausgefahren werden. Auf diese vorteilhafte Weise lässt sich der Zugriff auf medizinische Zubehöre einschränken. Auch lässt sich gegenüber einer Zugriffseinschränkung mittels jeweiliger Lagereinrichtungen für die einzelnen Zubehörgattungen die Anzahl der Lagereinrichtungen, welche zum Lagern für eine bestimmte Anzahl an Zubehörgattungen benötigt werden, reduzieren.

Gemäß einer bevorzugten Weiterbildung weist der wenigstens eine Herausgabemechanismus oder ein weiterer Herausgabemechanismus des Behandlungswagens eine Aussonderungsvorrichtung für eine der Lagereinrichtungen auf oder besteht daraus. Dabei ist die Aussonderungsvorrichtung zur Aussonderung von medizinischen Zubehören aus wenigstens einem der Lagerbereiche dieser Lagereinrichtung eingerichtet. Zudem ist das Steuerungssystem eingerichtet, die Aussonderungsvorrichtung auf Basis der Zubehörsetparameter so zu steuern, dass die Aussonderungsvorrichtung wenigstens ein medizinisches Zubehör aus dem wenigstens einen Lagerbereich dieser Lagereinrichtung entnimmt und an einem Herausgabebereich abgibt, wenn die Zubehörsetparameter ein solches medizinisches Zubehör kennzeichnen.

Im Sinne der Erfindung ist eine "Aussonderungsvorrichtung" eine Vorrichtung, welche eingerichtet ist, eine Sache, insbesondere ein medizinisches Zubehör, an einem Ausgangsort, insbesondere aus einer Lagereinrichtung und/oder aus einem Lagerbereich einer Lagereinrichtung, zu entnehmen und an einem Zielort, insbesondere einem Herausgabebereich, abzugeben.

Zudem weist die Aussonderungsvorrichtung vorzugsweise Steuermittel, insbesondere Steuereinrichtungen, auf, welche den Ausgangsort oder den Zielort steuern. Mittels des Steuerns des Ausgangsorts lässt sich eine bestimmte Sache aus einer Mehrzahl von Sachen, die insbesondere in einer Lagereinrichtung oder einem Lagerbereich einer Lagereinrichtung gelagert sein können, entnehmen. Daneben lässt sich mittels des Steuerns des Zielorts die auszusondernde Sache an einem bestimmten Zielort, insbesondere an einer bestimmten Position in einem Herausgabebereich, abgeben. Vorzugsweise leitet ein solches Steuermittel die auszusondernde Sache selbst in eine bestimmte Richtung. Auch bevorzugt und alternativ oder zusätzlich verändert, beeinflusst oder steuert ein solches Steuermittel ein Leitmittel so, dass dieses Leitmittel die auszusondernde Sache von einem bestimmten Ausgangsort und/oder zu einem bestimmten Zielort leitet. Solche Steuermittel bzw. Steuereinrichtungen sind insbesondere variable Magnetfelder oder elektrische Felder, ein in Stärke und/oder Richtung steuerbarer Luftstrom, Weichen - etwa wenn als Leitmittel Schienen dienen -, Klappen und/oder Stellglieder.

Insbesondere kann die Aussonderungsvorrichtung auch als Greifeinrichtung ausgebildet sein, wobei die Leiteinrichtung eine oder mehrere Greifglieder der Greifeinrichtung und die Steuereinrichtung eine Positionierungs- und/oder Orientierungseinrichtung der Greifeinrichtung - etwa Teleskopstangen und/oder Gelenke und Gelenkarme - aufweist oder daraus besteht.

Die Weiterbildung mit Aussonderungsvorrichtung ermöglicht es, wenigstens ein medizinisches Zubehör aus dem wenigstens einen Lagerbereich auszusondern. Auf diese vorteilhafte Weise kann ein solches medizinisches Zubehör insbesondere so herausgegeben und/oder bereitgestellt werden, dass, insbesondere während der Behandlung, keine Betätigung zur Herausgabe und/oder eine manuelle Entnahme erforderlich ist. Auch lässt sich insbesondere vermeiden, dass zur Herausgabe der Lagerbereich für die Zubehörgattung des medizinischen Zubehörs zugänglich gemacht werden muss. Damit lässt sich die Hygiene verbessern, der Benutzungskomfort steigern und/oder die Arbeitseffizienz erhöhen.

Gemäß einer bevorzugten Variante der Weiterbildung mit Aussonderungsvorrichtung ist die Aussonderungsvorrichtung als automatische Spendervorrichtung für medizinische Zubehöre wie Einmalhandschuhe, Kanülen, Spritzen, Tupfer, Tücher oder vorportionierte Pflaster oder Klebebandabschnitte ausgebildet. Solche medizinische Zubehöre können insbesondere einzeln verpackt, etwa bei Pflastern, Tupfern oder Kanülen, oder zu Einheiten von je einer vorbestimmten Anzahl größer 1 verpackt, etwa ein Paar Einmalhandschuhe, sein. Für ein solches verpacktes medizinisches Zubehör kann die automatische Spendervorrichtung vorzugsweise eine Schneideinrichtung zum Aufschneiden oder eine Reißeinrichtung zum Aufreißen der Verpackung des medizinischen Zubehörs aufweisen.

Auch kann der Behandlungswagen gemäß einer bevorzugten Weiterbildung eine oder mehrere manuelle Spendervorrichtungen für medizinische Zubehöre aufweisen. Insbesondere wird eine solche manuelle Spendervorrichtung nicht von dem Steuerungssystem gesteuert, sondern manuell betätigt. Gemäß einer bevorzugten Variante weist eine manuelle Spendervorrichtung einen Schacht zum Lagern von medizinischen Zubehören einer bestimmten Zubehörgattung auf. Wenn die manuelle Spendervorrichtung für ihre übliche Verwendung orientiert ist, zeigt die Längsachse des Schachts zumindest soweit entlang der Gravitationskraft, dass darin gelagerte medizinische Zubehöre nach unten fallen oder gleiten können. Dabei weist die manuelle Spendervorrichtung des Weiteren einen Trägerteil und der Schacht eine seitliche Öffnung zur Entnahme von medizinischen Zubehören auf, wobei der Trägerteil eingerichtet ist, ein unterstes medizinisches Zubehör so zu tragen, dass das unterste medizinische Zubehör im Schacht und bei der Öffnung getragen wird, über die Öffnung entnehmbar ist sowie die Öffnung für weitere, insbesondere über dem untersten medizinischen Zubehör angeordnete, medizinische Zubehöre blockiert und ein Nach-Unten-Fallen oder Gleiten der weiteren medizinischen Zubehöre verhindert.

Gemäß einer bevorzugten Weiterbildung weist der Behandlungswagen eine Dosiervorrichtung mit einer der Lagereinrichtungen, welche wenigstens einen Lagerbereich für wenigstens ein medizinisches Zubehör einer dosierbaren Zubehörgattung aufweist, und mit einer Aussonderungsvorrichtung zur teilweisen Entnahme des wenigstens einen medizinischen Zubehörs auf. Vorzugsweise ist die Aussonderungsvorrichtung dabei auch ein Bestandteil des Herausgabemechanismus oder der Herausgabemechanismus als die Aussonderungsvorrichtung ausgebildet. Das Auswahlverfahren der bevorzugten Weiterbildung weist des Weiteren die folgenden Verfahrensschritte auf. In einem Verfahrensschritt wird überprüft, ob die Zubehörsetparameter ein medizinisches Zubehör dieser dosierbaren Zubehörgattung kennzeichnen. In einem weiteren Verfahrensschritt werden, falls dies der Fall ist, einer oder mehrere Dosierparameter bestimmt, welche das dosierbare medizinische Zubehör und die für das Zubehörset benötigte Dosis kennzeichnen. Zudem ist das Steuerungssystem eingerichtet, die Aussonderungsvorrichtung der Dosiervorrichtung auf Basis der Dosierparameter und vorzugsweise der Zubehörsetparameter so zu steuern, dass die Aussonderungsvorrichtung einen Teil des wenigstens einen dosierbaren medizinischen Zubehörs, welcher der benötigten Dosis entspricht, aus dem wenigstens einen Lagerbereich der Dosiervorrichtung entnimmt und an einem Herausgabebereich abgibt. Auf diese vorteilhafte Weise kann von einem dosierbaren medizinischen Zubehör jeweils der benötigte Teil entnommen werden. Insbesondere lässt sich auf diese Weise vermeiden, dass von einer Art an medizinischem Zubehör eine Vielzahl an unterschiedlichen Dosierungen bevorratet werden muss.

Gemäß einer bevorzugten Variante wird zum Entnehmen von dem dosierbaren medizinischen Zubehör ein Teil davon abgetrennt, insbesondere abgeschnitten. Vorzugsweise weist diese Variante eine Schneideinrichtung und im Lagerbereich eine Halteeinrichtung für das dosierbare medizinische Zubehör auf. Insbesondere bei einer Dosiervorrichtung für Klebebandrollen kann diese als Schneideinrichtung ein Schneidmesser mit einem Aktuator, als Halteeinrichtung einen Klebebandrollen-Halter für eine Klebebandrolle und des Weiteren eine Fördereinrichtung für Klebeband aufweisen. Auf diese vorteilhafte Weise lässt sich von dem dosierbaren medizinischen Zubehör, insbesondere von dem Klebeband der Klebebandrolle, je nach Bedarf ein Teil abtrennen, womit verschiedene Dosierungen des dosierbaren medizinischen Zubehörs bereitgestellt werden können, ohne dass diese einzeln bevorratet werden müssen. Insbesondere sind vorportionierte - also insbesondere vorab zugeschnittene - medizinische Zubehöre üblicherweise teurer als ein medizinisches Zubehör, welches in diese vorportionierten medizinischen Zubehöre aufgetrennt werden kann. Folglich lassen sich durch das Abtrennen insbesondere Kosten reduzieren. Insbesondere kann auch die Anzahl der Lagerbereiche reduziert und/oder das Aussondern gegenüber einem Aussondern aus einer Vielzahl an Lagerbereichen für eine Vielzahl von Dosierungen technisch vereinfacht werden.

Gemäß einer bevorzugten und alternativen Variante für ein fließfähiges, flüssiges, pastöses, pulverförmiges oder körniges, medizinisches Zubehör weist die Lagereinrichtung der Dosiervorrichtung ein Behältnis mit dem wenigstens einen Lagerbereich auf, welches zur Aufnahme des fließfähigen medizinischen Zubehörs eingerichtet ist, und die Aussonderungsvorrichtung ist eingerichtet, eine gemäß der Dosierparameter vorbestimmte Menge des fließfähigen medizinischen Zubehörs aus dem Behältnis fließen zu lassen, insbesondere abzupumpen. Vorzugsweise weist die Aussonderungsvorrichtung dazu eine Pumpeinrichtung und/oder ein, insbesondere elektrisch, steuerbares Ventil auf. Insbesondere sind solche fließfähigen medizinischen Zubehöre besonders zum Dosieren geeignet. Ein Vorteil des Dosierens durch Fließen-Lassen des fließfähigen medizinischen Zubehörs kann insbesondere in der technisch einfachen Realisierung, d.h. insbesondere einer Realisierung mit wenigen und/oder bewährten Bauteilen - etwa Pumpen und/oder Ventilen -, und/oder in der Zuverlässigkeit einer solchen Realisierung liegen. Insbesondere kann eine Dosiervorrichtung dieser Variante ein Desinfektionsmittel oder ein Reinigungsmittel dosiert herausgeben, wobei die herausgegebene Menge insbesondere fest vorbestimmt oder abhängig von der durchzuführenden Behandlung oder den durchzuführenden und/oder die Arbeitseffizienz steigern lässt.

Vorzugsweise kann der Behandlungswagen eine Kombination aus einer Aussonderungsvorrichtung für Tupfer oder Tücher und einer Dosiervorrichtung für Desinfektionsmittel aufweisen, wobei die Dosiervorrichtung eine Dosis Desinfektionsmittel auf einen auszusondernden oder ausgesonderten Tupfer bzw. Tuch appliziert.

Der Behandlungswagen weist eine desinfizierbare Ablagefläche oder eine Ablageeinrichtung mit einer desinfizierbaren Ablagefläche auf. Dabei ist die Ablagefläche für die Ablage von medizinischen Zubehören eingerichtet und/oder weist einen oder mehrere Herausgabebereiche für das Aussondern von medizinischen Zubehören auf.

Ein Vorteil der desinfizierbaren Ablagefläche kann insbesondere darin liegen, dass diese desinfiziert werden kann, womit insbesondere sich die Hygiene auch bei längerer Verwendung des Behandlungswagens sicherstellen lässt und/oder die Ablagefläche bzw. die Ablageeinrichtung für mehrere Behandlungen verwendet werden kann.

Ein Vorteil der für die Ablage von medizinischen Zubehören eingerichteten Ablagefläche kann insbesondere darin liegen, dass vor der Behandlung zumindest ein Teil oder vorzugsweise alle medizinischen Zubehöre des Zubehörsets auf der Ablagefläche abgelegt werden können, womit sie für die Behandlung bereitstehen, ohne dass während der Behandlung eine Herausgabe und/oder eine Betätigung zur Herausgabe und/oder Entnahme eines solchen medizinischen Zubehörs erforderlich ist. Dies kann insbesondere die Arbeitseffizienz steigern, die Behandlungssicherheit erhöhen und/oder, insbesondere zusammen mit der Desinfizierbarkeit der Ablagefläche, die Hygiene verbessern.

Ein Vorteil der Ablagefläche mit einem oder mehreren Herausgabebereichen kann insbesondere darin liegen, dass ein medizinisches Zubehör, wenn es an einen dieser Herausgabebereiche ausgesondert wird, auf der Ablagefläche abgelegt und die Behandlungssicherheit und/oder die Arbeitseffizienz verbessern lässt. Vorzugsweise weist die Ablagefläche mehrere Herausgabebereiche auf und mehrere Aussonderungsvorrichtungen des Behandlungswagens sind eingerichtet, medizinische Zubehöre jeweils an einem bestimmten Herausgabebereich auszusondern, so dass ein medizinisches Zubehör einer bestimmten Zubehörgattung immer an einem bestimmten Herausgabebereich ausgesondert und auf diese Weise dort bereitgestellt wird. Auf diese vorteilhafte Weise lassen sich bestimmte Zubehörgattungen bestimmten Herausgabebereichen zuordnen, sodass insbesondere die Position von medizinischen Zubehören einer bestimmten Zubehörgattung auf der Ablagefläche gleich bleibt und/oder sich medizinische Zubehöre verschiedener Zubehörgattungen bereits aufgrund ihrer verschiedenen Positionen auf der Ablagefläche unterscheiden lassen, womit sich insbesondere die an der Behandlung beteiligten Personen entlasten lassen, sich die Arbeitseffizienz steigern und/oder sich die Behandlungssicherheit erhöhen lässt.

Der Behandlungswagen weist eine Desinfektionsvorrichtung für die Ablagefläche des Behandlungswagens auf.

Gemäß einer bevorzugten Variante ist das Steuerungssystem eingerichtet, die Desinfektionsvorrichtung für die Ablagefläche so zu steuern, dass die Desinfektionsvorrichtung die Ablagefläche, insbesondere vor, während und/oder nach der durchzuführenden Behandlung und/oder vor, während und/oder nach bestimmten Behandlungsschritten der durchzuführenden Behandlung, desinfiziert.

Der Behandlungswagen weist gemäß einer bevorzugten Weiterbildung eine Verbindungsvorrichtung für Behandlungswagenmodule auf. Dabei ist die Verbindungsvorrichtung eingerichtet, eine formschlüssige, kraftschlüssige und/oder stoffschlüssige Verbindung zwischen dem Behandlungswagen und einem oder mehreren Behandlungswagenmodulen auszubilden.

Im Sinne der Erfindung ist ein "Behandlungswagenmodul" eine Vorrichtung für einen Behandlungswagen, welches von dem Behandlungswagen abtrennbar ist und für den Behandlungswagen eine oder mehrere Funktionen bereitstellt. Insbesondere kann das Behandlungswagenmodul mit dem Behandlungswagen lösbar, vorzugsweise formschlüssig oder kraftschlüssig, verbunden werden und, zumindest wenn diese miteinander verbunden sind, eine oder mehrere Funktionen des Behandlungswagens übernehmen und/oder den Funktionsumfang des Behandlungswagens erweitern. Vorzugsweise weist ein solches Behandlungswagenmodul eine oder mehrere Verbindungsstellen für eine lösbare, insbesondere mechanische, Verbindung mit dem Behandlungswagen, insbesondere mit einer Verbindungsvorrichtung des Behandlungswagens, auf.

Im Sinne der Erfindung ist eine "Verbindungsvorrichtung für Behandlungswagenmodule" eine Vorrichtung zur lösbaren Verbindung eines oder mehrerer Behandlungswagenmodulen mit einem Behandlungswagen. Vorzugsweise ist die Verbindungseinrichtung ein Bestandteil des Behandlungswagens und mit diesem mechanisch verbunden. Die lösbare Verbindung kann insbesondere eine formschlüssige oder kraftschlüssige Verbindung sein, wobei diese vorzugsweise eine mechanische Verbindung ist. Vorzugsweise weist die Verbindungsvorrichtung eine oder mehrere Verbindungseinrichtungen je für ein Behandlungswagenmodul auf. Dabei ist eine solche Verbindungseinrichtung so eingerichtet und insbesondere so geformt, eine lösbare, insbesondere mechanische, Verbindung mit einer oder mit mehreren Verbindungsstellen des jeweiligen Behandlungswagenmoduls auszubilden, insbesondere auszuformen. Weiter bevorzugt weist wenigstens eine der Verbindungseinrichtungen eine Aufnahmeeinrichtung auf oder besteht daraus, wobei die Aufnahmeeinrichtung eingerichtet ist, ein Behandlungswagenmodul - insbesondere ein Behandlungswagenmodul, welches mit dieser Aufnahmeeinrichtung korrespondiert - ganz oder teilweise aufzunehmen und damit die lösbare Verbindung zu dem Behandlungswagen auszubilden.

Ein Vorteil des modularen Aufbaus des Behandlungswagens kann insbesondere darin liegen, dass sich der Behandlungswagen mittels entsprechender Behandlungswagenmodule an die jeweiligen Anforderungen anpassen lässt. Auch lässt sich ein solcher Behandlungswagen durch Austausch von Behandlungswagenmodulen auf einfache Weise reparieren und/oder modernisieren, womit insbesondere die Einsatzdauer verlängert werden kann.

Gemäß einer bevorzugten Weiterbildung weist der Behandlungswagen ein Bussystem zur Ausbildung einer Datenverbindung mit und insbesondere zur Steuerung von wenigstens einem der Behandlungswagenmodule, dem wenigstens einen Herausgabemechanismus oder einem weiteren Herausgabemechanismus, der Aussonderungsvorrichtung und/oder der Dosiervorrichtung auf. Ein solches Bussystem kann insbesondere ein Universal Serial Bus (USB) oder ein Controller Area Network Bus (CAN-Bus) sein.

Gemäß einer bevorzugten Weiterbildung ist die Benutzerschnittstelle, wenigstens eine der Lagereinrichtungen, einer der Herausgabemechanismen oder ein Teil davon, das Steuerungssystem oder ein Teil davon, ein Schubfach einer der Lagereinrichtungen, die Aussonderungsvorrichtung, die Dosiervorrichtung und/oder die Ablageeinrichtung als Behandlungswagenmodul ausgebildet.

Vorzugsweise weist ein Behandlungswagenmodul eine Kombination mit der Benutzerschnittstelle, wenigstens einer der Lagereinrichtungen, einem der Herausgabemechanismen oder einem Teil davon, dem Steuerungssystem oder einem Teil davon, einem Schubfach einer der Lagereinrichtungen, der Aussonderungsvorrichtung, der Dosiervorrichtung und/oder der Ablageeinrichtung und/oder mit weiteren Vorrichtungen auf.

Gemäß einer bevorzugten Weiterbildung weist der Behandlungswagen eine Verbindungsvorrichtung für eine von dem Behandlungswagen abtrennbare Fahrvorrichtung auf. Insbesondere kann die abtrennbare Fahrvorrichtung als Behandlungswagenmodul ausgebildet sein. Auch ist in einer breiten Auslegung die abtrennbare Fahrvorrichtung kein Bestandteil des Behandlungswagens. Vorzugsweise kann der Behandlungswagen jedoch die abtrennbare Fahrvorrichtung aufweisen.

Im Sinne der Erfindung ist eine "Fahrvorrichtung" eine Vorrichtung, welche eingerichtet ist, eine weitere Vorrichtung, insbesondere einen Behandlungswagen, beweglich auf einer Fahrbahn, insbesondere einem Untergrund, zu lagern. Vorzugsweise wird dazu die Fahrvorrichtung mit der weiteren Vorrichtung mechanisch verbunden. Dabei kann diese Verbindung entweder dauerhaft sein; oder die Verbindung lösbar, die Fahrvorrichtung also eine abtrennbare Fahrvorrichtung sein. Zum beweglichen Lagern auf dem Untergrund, etwa einer Bodenfläche oder einer Straße, kann die Fahrvorrichtung vorzugsweise Räder, Rollen, Bänder oder Ketten aufweisen. Vorzugsweise kann die Fahrvorrichtung eine aktive Fahrvorrichtung sein und dazu einen Antrieb - insbesondere einen Motor, der mit wenigstens einem der Räder mechanisch gekoppelt ist - aufweisen. Bevorzugt und alternativ kann eine solche Fahrvorrichtung eine passive Fahrvorrichtung sein, also keinen Antrieb aufweisen. Weiter bevorzugt kann eine solche Fahrvorrichtung eine autonome Fahrvorrichtung sein, welche einen Antrieb und eine Steuerungseinrichtung aufweist und eingerichtet ist, von einem Startort zu einem Zielort autonom, d.h. insbesondere ohne Steuerung durch einen Menschen, zu fahren.

Gemäß einer bevorzugten Weiterbildung ist die Benutzerschnittstelle mit dem Behandlungswagen unlösbar verbunden. Dabei ist diese Verbindung vorzugsweise eine unlösbare formschlüssige, kraftschlüssige oder stoffschlüssige Verbindung. Auch vorzugsweise ist die Benutzerschnittstelle mit dem Behandlungswagen integral verbunden. Auf diese vorteilhafte Weise lässt sich sicherstellen, dass die Benutzerschnittstelle am Ort des Behandlungswagens und damit der Behandlungswagen insbesondere bedienbar ist.

Gemäß einer bevorzugten Weiterbildung ist die Benutzerschnittstelle mit dem Behandlungswagen lösbar, insbesondere lösbar formschlüssig oder lösbar kraftschlüssig, verbunden oder verbindbar. Ein Vorteil der lösbaren Verbindung der Benutzerschnittstelle kann insbesondere darin liegen, dass die Benutzerschnittstelle von dem Behandlungswagen abgetrennt werden kann und insbesondere unabhängig von dem Behandlungswagen gereinigt werden kann und/oder, etwa bei Defekt, durch eine andere entsprechende Benutzerschnittstelle ersetzt werden kann. Auch wird es insbesondere ermöglicht, den Behandlungswagen und die Benutzerschnittstelle als eine logistische Einheit zusammenzufassen, solange diese miteinander lösbar verbunden sind.

Gemäß einer bevorzugten Weiterbildung ist die Benutzerschnittstelle, während diese zumindest für Eingaben eingerichtet ist, infolge derer das Steuerungssystem die Behandlungskennung der durchzuführenden Behandlung bestimmt, an einem geographisch anderen Ort als dem Ort des Behandlungswagens positioniert oder positionierbar. Auf diese vorteilhafte Weise lässt sich der Behandlungswagen unabhängig von dem Ort der Benutzerschnittstelle bedienen. So kann etwa die Benutzerschnittstelle vor oder während der Behandlung an einem Ort positioniert werden, welcher zur Bedienung des Behandlungswagens während der Behandlung besonders geeignet ist.

Gemäß einer bevorzugten Weiterbildung weist das mobile Auswahlsystem wenigstens einen weiteren Behandlungswagen auf. Dabei gelten die möglichen Vorteile sowie Ausführungsformen, Weiterbildungen oder Varianten des Behandlungswagens des mobilen Auswahlsystems entsprechend für den wenigstens einen weiteren Behandlungswagen.

Gemäß einer bevorzugten Weiterbildung, insbesondere bei einer bevorzugten Weiterbildung des mobilen Auswahlsystems mit einem oder mehreren Behandlungswagen und/oder mit einem oder mehreren Behandlungswagenmodulen, ist wenigstens eines der Behandlungswagenmodule bzw. das Behandlungswagenmodul wenigstens einem der Behandlungswagen zuordenbar.

Ein Vorteil des zuordenbaren Behandlungswagenmoduls kann insbesondere darin liegen, dass dem Behandlungswagen - insbesondere bei einem mobilen Auswahlsystem mit nur einem Behandlungswagen oder einer Ausführungsform, Weiterbildung oder Variante des Behandlungswagens - bzw. einem der Behandlungswagen - insbesondere bei einem mobilen Auswahlsystem mit mehreren Behandlungswagen - dieses zuordenbare Behandlungswagenmodul zugeordnet werden kann, womit insbesondere bestimmt werden kann, ob dieser Behandlungswagen dieses zuordenbare Behandlungswagenmodul aufweist. Dabei ist in einer bevorzugten Variante das Steuerungssystem eingerichtet, einen Behandlungswagen abhängig davon zu steuern, welche zuordenbare Behandlungswagenmodule diesem Behandlungswagen zugeordnet sind und/oder welche Behandlungswagenmodule dieser aufweist.

Umgekehrt kann, insbesondere bei einem mobilen Auswahlsystem mit mehreren Behandlungswagen, ein Vorteil des zuordenbaren Behandlungswagenmoduls darin liegen, dass einer der Behandlungswagen dem zuordenbaren Behandlungswagenmodul zugeordnet werden kann, womit insbesondere bestimmt werden kann, ob und welcher der Behandlungswagen dieses zuordenbare Behandlungswagenmodul aufweist. Dabei ist in einer bevorzugten Variante dieses zuordenbare Behandlungswagenmodul auf vorteilhafte Weise so eingerichtet, dass sich dessen Funktionen an den Behandlungswagen, welcher dieses Behandlungswagenmodul aufweist, anpassen.

Gemäß einer bevorzugten Weiterbildung, bei welcher wenigstens ein Behandlungswagenmodul einen Behandlungswagen zuordenbar ist, weist das mobile Auswahlsystem, insbesondere das Steuerungssystem, eine Zuordnungsvorschrift für Behandlungswagenmodule und eine Datenspeichervorrichtung für diese auf. Dabei ist die Zuordnungsvorschrift eingerichtet, anhand von Zuordnungsdaten, welche auf dieser Datenspeichervorrichtung gespeichert sind, einem Behandlungswagenmodul, welches mittels einer Behandlungswagenmodulkennung gekennzeichnet ist, einen Behandlungswagen, welcher mittels einer Behandlungswagenkennung gekennzeichnet ist, zuzuordnen. Ein Vorteil der Zuordnungsvorschrift für Behandlungswagenmodul kann insbesondere darin liegen, dass die Zuordnung zwischen Behandlungswagenmodulen und Behandlungswagen anhand von Zuordnungsdaten, die in der Datenspeichervorrichtung für diese Zuordnungsvorschrift gespeichert sind, festgelegt werden kann. Vorzugsweise kann der Speicherinhalt dieser Datenspeichervorrichtung veränderbar sein, also die Datenspeichervorrichtung zumindest einen beschreibbaren, insbesondere wiederbeschreibbaren, Speicherbereich aufweisen, so dass sich die Zuordnung durch Verändern der Zuordnungsdaten verändern lässt. Dies ermöglicht es insbesondere die Zuordnungen der Zuordnungsvorschrift an die tatsächlichen, physischen Gegebenheiten - insbesondere ohne mechanische Umrüstungen und/oder zumindest im Wesentlichen verschleißfrei - anzupassen.

Gemäß einer bevorzugten Weiterbildung, bei welcher wenigstens ein Behandlungswagenmodul einem Behandlungswagen zuordenbar ist, weist das mobile Auswahlsystem oder der Behandlungswagen eine Benutzerschnittstelle auf, die für eine oder mehrere Eingaben von einem Benutzer eingerichtet ist, durch die die Zuordnung des zuordenbaren Behandlungswagenmoduls zu dem Behandlungswagen festgelegt wird. Gemäß einer bevorzugten Variante ist dabei das Steuerungssystem eingerichtet, auf Basis dieser Eingaben Zuordnungsdaten zu bestimmen und in die Speichervorrichtungen für die Zuordnungsvorschrift für Behandlungswagenmodule zu schreiben, welche die Zuordnung des Behandlungswagenmoduls zu dem Behandlungswagen kennzeichnen.

Gemäß einer bevorzugten Weiterbildung, bei welcher wenigstens ein Behandlungswagenmodul einem Behandlungswagen zuordenbar ist, weist der Behandlungswagen ein Erkennungssystem für Behandlungswagenmodule auf, das mittels einer auf Messung von Strahlung, insbesondere elektromagnetischer Strahlung wie Licht oder Radiowellen, von Ultraschall, von elektrischen Kontaktierungen und/oder von zumindest im Wesentlichen statischen Magnetfeldern basierenden Sensorik Daten über ein Behandlungswagenmodul, das insbesondere benachbart zu diesem Erkennungssystem positioniert ist, erfasst und basierend darauf die Behandlungswagenmodulkennung dieses Behandlungswagenmoduls bestimmt und/oder dem Behandlungswagen dieses Behandlungswagenmodul zuordnet.

Vorzugsweise ist zum Zuordnen das Erkennungssystem zur Ausbildung einer Datenverbindung mit dem Steuerungssystem eingerichtet und/oder weist eine Datenverbindung mit dem Steuerungssystem auf, wobei das Erkennungssystem oder das Steuerungssystem eingerichtet ist, die Behandlungswagenkennung des Behandlungswagens zu bestimmen sowie auf Basis der Behandlungswagenkennung und der Behandlungswagenmodulkennung Zuordnungsdaten zu bestimmen und in die Speichervorrichtungen für die Zuordnungsvorschrift für Behandlungswagenmodule zu schreiben, welche die Zuordnung des Behandlungswagenmoduls zu dem Behandlungswagen kennzeichnen. In einer bevorzugten Variante weist das wenigstens eine zuordenbare Behandlungswagenmodul eine Kennungseinrichtung, insbesondere einen Barcode oder einen RFID-Tag, auf und das Erkennungssystem für Behandlungswagenmodul weist ein entsprechendes Lesegerät, insbesondere ein Barcodelesegerät bzw. ein RFID-Lesegerät, auf, womit sich das Behandlungswagenmodul und/oder die Behandlungswagenmodulkennung bestimmen lässt.

Gemäß einer bevorzugten Weiterbildung weist wenigstens ein Behandlungswagenmodul ein Erkennungssystem für Behandlungswagen auf. Dieses ist entsprechend dem Erkennungssystem für Behandlungswagenmodule eingerichtet, wobei ein Behandlungswagen und/oder dessen Behandlungswagenkennung basierend auf der Messung bestimmt werden. Dabei gelten die möglichen Vorteile sowie Ausführungsformen, Weiterbildungen oder Varianten des Erkennungssystems für Behandlungswagenmodule entsprechend für das Erkennungssystem für Behandlungswagen.

Gemäß einer bevorzugten Weiterbildung, bei welcher wenigstens ein Behandlungswagenmodul einem Behandlungswagen zuordenbar ist, wird die Zuordnung zwischen dem Behandlungswagenmodul und dem Behandlungswagen durch Herstellen der lösbaren Verbindung festgelegt. Ein Vorteil der Zuordnung mittels lösbarer Verbindung kann insbesondere darin liegen, dass die Zuordnung automatisiert werden kann und/oder neben dem Herstellen der lösbaren Verbindung keine zusätzlichen Arbeitsschritte erforderlich sind, wodurch insbesondere das Personal entlastet werden kann, Fehler vermieden werden können und/oder die Arbeitseffizienz gesteigert werden kann.

Vorzugsweise kann dabei die Zuordnung mechanisch, insbesondere durch Verbindung mit einem mechanischen Steuerelement des Behandlungswagens, und/oder durch Anschluss an ein Bussystem des Behandlungswagens erfolgen, was auf vorteilhafte Weise eine technisch einfache Realisierung erlaubt.

Auch vorzugsweise kann dabei die Zuordnung mittels eines Erkennungssystems für Behandlungswagenmodule erfolgen, welches eingerichtet ist, die Erkennung beim oder nach dem Herstellen der lösbaren Verbindung durchzuführen, was auf vorteilhafte Weise eine automatisierte Zuordnung erlaubt. Gemäß einer bevorzugten Variante müssen die Behandlungswagenmodule für diese Erkennung nicht speziell ausgestaltet sein oder weisen nur eine technisch einfache, bewährte und/oder kostengünstige Kennungseinrichtung - etwa einen Barcode oder einen RFID-Tag - auf, wodurch sich der technische Aufwand für die Erkennung wird auf das Erkennungssystem verlagern lässt, was insbesondere die Behandlungswagenmodule technisch vereinfachen, kostengünstiger und/oder zuverlässiger machen kann.

Gemäß einer bevorzugten Weiterbildung weist das mobile Auswahlsystem wenigstens eine weitere Benutzerschnittstelle auf. Dabei gelten die möglichen Vorteile sowie Ausführungsformen, Weiterbildungen oder Varianten der Benutzerschnittstelle des mobilen Auswahlsystems entsprechend für die wenigstens eine weitere Benutzerschnittstelle.

Gemäß einer bevorzugten Weiterbildung, bei welcher das mobile Auswahlsystem wenigstens einen weiteren Behandlungswagen aufweist, ist wenigstens eine der Benutzerschnittstellen dem Behandlungswagen und dem weiteren Behandlungswagen für Eingaben zuordenbar. Ein Vorteil der zuordenbaren Benutzerschnittstelle kann insbesondere darin liegen, dass diese einem der Behandlungswagen zugeordnet werden kann, womit insbesondere festgelegt werden kann, welcher der Behandlungswagen mit dieser Benutzerschnittstelle bedient wird und/oder für welchen der Behandlungswagen diese Benutzerschnittstelle Eingaben von einem Benutzer erfasst, durch die insbesondere eine durchzuführende Behandlung festgelegt wird, für welche dieser Behandlungswagen medizinische Zubehöre herausgibt.

Gemäß einer bevorzugten Weiterbildung, bei welcher wenigstens eine der Benutzerschnittstellen einem der Behandlungswagen zuordenbar ist, ist diese Benutzerschnittstelle mit einem der Behandlungswagen unlösbar verbunden und damit diesem Behandlungswagen zugeordnet. Ein Vorteil der Zuordnung mittels unlösbarer Verbindung kann insbesondere darin liegen, dass die Zuordnung auf technisch einfache Weise realisiert werden kann und/oder, insbesondere gegenüber einer anpassbaren Zuordnung oder lösbaren Verbindung, besonders robust und/oder zuverlässig ist.

Gemäß einer bevorzugten Weiterbildung, bei welcher das mobile Auswahlsystem wenigstens eine Benutzerschnittstelle aufweist, die mit einem der Behandlungswagen unlösbar verbunden ist und diesem damit zugeordnet ist, weist das mobile Auswahlsystem für jeden der Behandlungswagen wenigstens eine solche unlösbar verbundene Benutzerschnittstelle auf, welche damit dem jeweiligen Behandlungswagen zugeordnet ist.

Gemäß einer bevorzugten Weiterbildung, bei welcher wenigstens eine der Benutzerschnittstellen einem der Behandlungswagen zuordenbar ist, ist diese Benutzerschnittstelle mit einem der Behandlungswagen lösbar verbunden und damit diesem Behandlungswagen zugeordnet. Ein Vorteil der Zuordnung mittels lösbarer Verbindung kann insbesondere darin liegen, dass die Zuordnung auf technisch einfache Weise realisiert werden kann und/oder, insbesondere gegenüber einer Zuordnung mittels unlösbarer Verbindung, die Benutzerschnittstelle einem der Behandlungswagen durch lösbares Verbinden an die jeweiligen Anforderungen anpassbar zugeordnet werden kann.

Gemäß einer bevorzugten Weiterbildung, bei welcher wenigstens eine der Benutzerschnittstellen einem der Behandlungswagen zuordenbar ist, weist das Steuerungssystem dazu eine Zuordnungsvorschrift für Benutzerschnittstellen und eine Datenspeichervorrichtung für diese auf. Die Zuordnungsvorschrift ist eingerichtet, anhand von Zuordnungsdaten, welche auf dieser Datenspeichervorrichtung gespeichert sind, einer Benutzerschnittstelle, welche mittels einer Benutzerschnittstellenkennung gekennzeichnet ist, einem Behandlungswagen, welcher mittels einer Behandlungswagenkennung gekennzeichnet ist, zuzuordnen. Ein Vorteil der Zuordnungsvorschrift für Benutzerschnittstellen kann insbesondere darin liegen, dass die Zuordnung zwischen Benutzerschnittstellen und Behandlungswagen anhand von Zuordnungsdaten, die in der Datenspeichervorrichtung für die Zuordnungsvorschrift für Benutzerschnittstellen gespeichert sind, festgelegt werden kann. Vorzugsweise kann der Speicherinhalt der Datenspeichervorrichtung veränderbar sein, also die Datenspeichervorrichtung zumindest einen beschreibbaren, insbesondere wiederbeschreibbaren, Speicherbereich aufweisen, so dass sich die Zuordnung durch Verändern der Zuordnungsdaten verändern lässt. In einer bevorzugten Variante ist wenigstens eine der Benutzerschnittstellen für eine oder mehrere Eingaben von einem Benutzer eingerichtet, durch die die Zuordnung wenigstens einer der mittels der Zuordnungsvorschrift zuordenbaren Benutzerschnittstellen zu einem Behandlungswagen festgelegt wird, und das Steuerungssystem ist eingerichtet, auf Basis dieser Eingaben Zuordnungsdaten zu bestimmen und in die Speichervorrichtung für die Zuordnungsvorschrift zu schreiben, welche die Zuordnung dieser wenigstens einen zuordenbaren Benutzerschnittstelle zu dem Behandlungswagen kennzeichnen.

Gemäß einer bevorzugten Weiterbildung ist das Steuerungssystem eingerichtet, Benutzer und/oder Patienten, insbesondere an der Behandlung beteiligte Personen, eindeutig voneinander zu unterscheiden. Ein Patient oder Benutzer wird vom Steuerungssystem vorzugsweise eindeutig identifiziert. Bevorzugt können auch mehrere Benutzer oder mehrere Patienten gruppiert und die Patientengruppen und/oder Benutzergruppen vom Steuerungssystem eindeutig identifiziert werden. Zudem können nicht-identifizierte Patienten oder Benutzer vom Steuerungssystem insbesondere als eine unidentifizierte Gruppe zusammengefasst werden. Zur Identifizierung verarbeitet das Steuerungssystem vorzugsweise Identifizierungsdaten. Vorzugsweise weist das Steuerungssystem eine Datenspeichervorrichtung für Identifizierungsdaten auf und ist eingerichtet, ein Identifizierungsverfahren auszuführen, mit dem die Identität eines Patienten oder Benutzers überprüft und der Patient oder Benutzer identifiziert wird, falls Daten, die in der Datenspeichervorrichtung für Identifizierungsdaten gespeichert sind, mit dem Patienten oder Benutzer korrespondieren.

Identifizierungsdaten enthalten zum Beispiel einen Login-Text und einen Passwort-Text oder einen Datensatz zur Gesichtserkennung oder zum Iris-Scan oder zum Fingerabdruckscan, oder andere Daten oder biometrische Daten. Die Biometrie bietet den Vorteil, dass der Patient oder Benutzer die Identität ohne kognitive Leistung ermitteln kann, was besonders vorteilhaft ist, wenn zum Beispiel der Patient nicht ansprechbar ist oder sich aus anderen Gründen nicht aktiv an der Identifizierung beteiligen kann. Die Identifizierung kann ferner mittels RFID-Chips, insbesondere RFID-Tags, oder NFC-Chips oder über Gestenerkennung erfolgen.

Eine Identifizierung kann insbesondere per Direktzugriff vor Ort oder per Fernzugriff auf einen der Behandlungswagen oder auf eine Benutzerschnittstelle, welche insbesondere diesem Behandlungswagen zugeordnet sein kann, erfolgen.

Vorzugsweise ist das Steuerungssystem eingerichtet, das Identifizierungsverfahren für einen Patienten oder Benutzer bei Vorliegen eines vorbestimmten Identifizierungskriteriums auszuführen. Ein solches Identifizierungskriterium kann insbesondere eine Anfrage des Patienten oder Benutzers zur Identifizierung, etwa durch Betätigen einer Benutzerschnittstelle, sein. Auch kann ein solches Identifizierungskriterium die Anwesenheit eines Benutzers oder Patienten in der Nähe - insbesondere gleicher Raum oder Entfernung höchstens 5 m, vorzugsweise höchstens 1,5 m und weiter bevorzugt höchstens 0,5 m - eines Behandlungswagens oder einer Benutzerschnittstelle sein.

Die Identifizierung kann erfordern, dass der Patient oder Benutzer zuvor am System registriert worden ist, so dass dem System Vergleichsdaten für die Identifizierung zur Verfügung stehen, also insbesondere Identifizierungsdaten, welche den Patienten oder Benutzer kennzeichnen, auf der Datenspeichervorrichtung für Identifizierungsdaten gespeichert sind. Insbesondere kann das Steuerungssystem eingerichtet sein, einen Patienten oder Benutzer im System zu registrieren, wodurch ein dem Patienten eindeutig zuordenbarer Satz von Patientendaten bzw. dem Benutzer ein dem Benutzer eindeutig zuordenbarer Satz von Benutzerdaten zugeordnet wird, der eine Patientenkennung, welche den Patienten kennzeichnet, bzw. eine Benutzerkennung, welche den Benutzer kennzeichnet, enthält. Dieser Patientendatensatz bzw. Benutzerdatensatz kann in der Datenspeichervorrichtung für Identifizierungsdaten gespeichert sein.

Vorzugsweise werden im Identifizierungsverfahren Identifizierungsdaten von den Patienten oder Benutzer mittels einer Sensoreinrichtung für Identifizierungsdaten erfasst und diese erfassten Identifizierungsdaten mit Daten der Datenspeichervorrichtung für Identifizierungsdaten verglichen. Vorzugsweise ist die Sensoreinrichtung für Identifizierungsdaten als biometrisches Lesegerät, insbesondere als Iris-Scanner, Fingerabdruckscanner oder Kamera zur Gesichts- oder Gestenerkennung, ausgebildet. Insbesondere kann wenigstens eine der Benutzerschnittstellen eine solche Sensoreinrichtung für Identifizierungsdaten aufweisen. Alternativ oder zusätzlich kann eine der Benutzerschnittstellen für eine oder mehrere Eingaben von einem Benutzer oder Patienten eingerichtet sein, durch die ein Benutzer oder Patient identifizierbar ist. Dabei kann eine derartig eingerichtete Benutzerschnittstelle insbesondere ein Lesegerät für Kennungen, etwa vorzugsweise ein Barcode-Lesegerät, ein RFID-Lesegerät, ein Dokumenten-Lesegerät oder ein Magnetstreifen-Lesegerät, aufweisen oder daraus bestehen, wobei mittels dieses Lesegeräts die Eingaben zur Identifizierung eingelesen werden können. Auch kann eine derartig eingerichtete Benutzerschnittstelle eine Tastatur, eine Maus oder einen berührungsempfindlichen Bildschirm zur Eingabe von Text und/oder grafischen Symbolen, etwa einem Login-Text und einen Passwort-Text, einem Geheimcode oder einer Erkennungsgeste, aufweisen.

Gemäß einer bevorzugten Weiterbildung ist das Steuerungssystem eingerichtet, den Benutzer in Abhängigkeit von einer erfolgreichen Identifizierung am System anzumelden, eine Benutzerkennung zu bestimmen, das Auswahlverfahren auszuführen und/oder die Herausgabe von medizinischen Zubehören auf Zubehörgattungen zu beschränken, für welche der angemeldete Benutzer autorisiert ist, und/oder, falls der Benutzer nicht erfolgreich identifiziert worden ist, das Auswahlverfahren nicht auszuführen und/oder die Auswahl oder die Herausgabe von medizinischen Zubehören auf Zubehörgattungen für nicht-autorisierte Benutzer zu beschränken. Auf diese vorteilhafte Weise lässt sich die Sicherheit steigern. Gemäß einer bevorzugten Variante ist das Steuerungssystem zur Personalisierung an den angemeldeten Benutzer eingerichtet und insbesondere eingerichtet, Ausgaben an einer der Benutzerschnittstellen und/oder das Auswahlverfahren an den angemeldeten Benutzer anzupassen, womit sich insbesondere der Benutzungskomfort, die Arbeitseffizienz, die Behandlungssicherheit und/oder die Behandlungsqualität steigern lässt. Hierbei kann insbesondere das Bestimmen der Zubehörsetparameter so an den angemeldeten Benutzer angepasst werden, dass bei mehreren möglichen Zusammenstellungen der medizinischen Zubehöre für die durchzuführende Behandlung - also insbesondere, wenn für die durchzuführende Behandlung wenigstens zwei verschiedene Zubehörsets geeignet sind, - Zubehörparameter für jene medizinische Zubehöre bestimmt werden, welche der angemeldete Benutzer präferiert. Eine solche Präferenz kann insbesondere in der Zubehördatenbank, vorzugsweise als, insbesondere behandlungsspezifische, Zuordnung je zwischen einem Präferenzwert und einem jeweiligen Benutzer, gespeichert werden.

Gemäß einer bevorzugten Weiterbildung ist eine der Benutzerschnittstellen für eine oder mehrere Eingaben von dem, insbesondere angemeldeten, Benutzer eingerichtet, durch die ein zu behandelnder Patient festgelegt wird und auf Basis derer das Steuerungssystem infolge dieser Eingaben eine dem zu behandelnden Patienten zugeordnete Patientenkennung bestimmt. Zusätzlich oder alternativ kann der Patient auch mittels Identifizierung festgelegt werden und basierend darauf das Steuerungssystem die Patientenkennung des identifizierten, zu behandelnden Patienten bestimmen. Insbesondere kann der Patient auch ein Benutzer sein. Ferner weist das Auswahlverfahren des Weiteren den Verfahrensschritt Bestimmen der Patientenkennung des zu behandelnden Patienten auf. Zudem werden bei dem Verfahrensschritt Bestimmen der Zubehörsetparameter des Auswahlverfahrens, die Zubehörsetparameter auf Basis der Behandlungskennung und/oder der Patientenkennung bestimmt. Das Bestimmen des zu behandelnden Patienten kann auf vorteilhafte Weise insbesondere eine Personalisierung ermöglichen. Ein Vorteil des Bestimmens der Zubehörsetparameter auf Basis der Patientenkennung kann insbesondere darin liegen, dass aus der Mehrzahl an medizinischen Zubehören jene medizinische Zubehöre ausgewählt werden können, welche für den zu behandelnden Patienten besonders geeignet sind - sich also die Auswahl personalisieren lässt. Vorzugsweise werden hierbei beim Bestimmen der Zubehörsetparameter, wenn mehrere Zusammenstellungen der medizinischen Zubehöre für die durchzuführende Behandlung möglich sind - also insbesondere für die durchzuführende Behandlung wenigstens zwei verschiedene Zubehörsets geeignet sind -, die Zubehörparameter für jene medizinische Zubehöre bestimmt, welche für den durch die Patientenkennung gekennzeichneten, zu behandelnden Patienten besonders geeignet und/oder von diesem präferiert sind. Eine solche Eignung kann insbesondere in der Zubehördatenbank oder in einer Patientendatenbank gespeichert werden, wobei vorzugsweise die Datenbank, insbesondere behandlungsspezifisch, einem medizinischen Zubehör bezüglich eines Patienten bzw. Patientenkennung einen Eignungswert zuordnet.

Gemäß einer bevorzugten Weiterbildung kann das Steuerungssystem eingerichtet sein, die Behandlungskennung der durchzuführenden Behandlung auf Basis einer Patientenkennung des zu behandelnden Patienten zu bestimmen oder aus einer Vielzahl von möglichen Behandlungen nur jene Behandlungen bei der Festlegung der durchzuführenden Behandlung für diesen Patienten zuzulassen, welche dem zu behandelnden Patienten über seine Patientenkennung zugeordnet sind. Auf diese vorteilhafte Weise lässt sich die Behandlungssicherheit, die Behandlungsqualität und/oder die Arbeitseffizienz steigern.

Gemäß einer bevorzugten Weiterbildung weist das Steuerungssystem eine Behandlungsdatenbank auf, welche für Datensätze über eine Mehrzahl an Behandlungen eingerichtet und auf einer Datenspeichervorrichtung des Steuerungssystems gespeichert ist. Insbesondere kann ein solcher Datensatz eine bestimmte Behandlung zugeordnet sein und dazu vorzugsweise eine Behandlungskennung dieser Behandlung aufweisen. Auch kann ein solcher Datensatz Behandlungsdaten aufweisen. Auch kann ein solcher Datensatz, welcher einer bestimmten Behandlung, die mehrere Behandlungsschritte aufweist, zugeordnet ist, Daten bezüglich eines oder mehrerer Behandlungsschritte dieser Behandlung aufweisen. Dabei können die Behandlungsschritte insbesondere jeweils mit einer Behandlungsschrittkennung gekennzeichnet sein und der Datensatz eine oder mehrere Behandlungsschrittkennungen aufweisen.

Ferner kann ein solcher Datensatz Daten über ein medizinisches Zubehör bezüglich einer bestimmten Behandlung oder bezüglich eines bestimmten Behandlungschritts aufweisen. Insbesondere können solche Daten über ein medizinisches Zubehör betreffen: Informationen über das Aussehen des medizinischen Zubehörs; Informationen darüber, ob dieses medizinische Zubehör bei der bestimmten Behandlung oder dem bestimmten Behandlungsschritt verwendet wird; die Anordnung des medizinischen Zubehörs bei der Behandlung oder den Behandlungsschritt; und/oder Bewegungstrajektorien, welche mit den Bewegungen des medizinischen Zubehörs bei der Behandlung oder dem Behandlungsschritt korrespondieren.

Ferner kann ein solcher Datensatz Daten über eine Körperregion von Patienten aufweisen, die von einer bestimmten Behandlung oder einem bestimmten Behandlungsschritt betroffen ist und insbesondere verändert wird. Insbesondere können solche Daten über eine Körperregion betreffen: Informationen über das Aussehen eines Hautbereichs, Informationen darüber, ob diese Körperregion von der bestimmten Behandlung oder dem bestimmten Behandlungsschritt betroffen ist; eine Veränderung der Körperregion aufgrund der bestimmten Behandlung oder des bestimmten Behandlungschritts - vorzugsweise etwa eine Farbänderung der Körperregion, insbesondere der Haut -; und/oder Bewegungstrajektorien, welche mit den Bewegungen der Körperregionen bei der Behandlung oder den Behandlungsschritt korrespondieren.

Gemäß einer bevorzugten Weiterbildung weist das mobile Auswahlsystem des Weiteren ein Erkennungssystem für Behandlungen auf. Das Erkennungssystem für Behandlungen erfasst mittels einer auf Messung von Strahlung, insbesondere Licht, von Ultraschall, von Gewicht und/oder von Trägheit basierenden Sensorik eine an der Behandlung beteiligte Person, insbesondere den Patienten, ein medizinisches Zubehör, insbesondere ein medizinisches Zubehör des Zubehörsets oder ein medizinisches Zubehör für eine Behandlung, und/oder deren Anordnung und/oder deren Bewegung zueinander. Basierend darauf bestimmt das Erkennungssystem für Behandlungen vorzugsweise anhand der Zubehördatenbank und/oder der Behandlungsdatenbank, eine durchgeführte Behandlung und/oder einen durchgeführten Behandlungsschritt, insbesondere bezüglich der durchgeführten Behandlung.

Vorzugsweise ist das Erkennungssystem für Behandlungen als Behandlungswagenmodul ausgebildet oder ein Behandlungswagenmodul weist einen oder mehrere Bestandteile des Erkennungssystems, insbesondere dessen Sensorik, auf.

Vorzugsweise weist das mobile Auswahlsystem, insbesondere der Behandlungswagen, eine Entsorgungsvorrichtung für medizinische Zubehöre mit einem solchen Erkennungssystem für Behandlungen auf.

Gemäß einer bevorzugten Weiterbildung weist eine Entsorgungsvorrichtung für medizinische Zubehöre ein Erkennungssystem für Behandlungen auf, welches eingerichtet ist, medizinische Zubehöre zu erfassen, welche mittels der Entsorgungsvorrichtung entsorgt werden. Vorzugsweise weist die Entsorgungsvorrichtung für medizinische Zubehöre einen Entsorgungsbehälter mit einer Entsorgungsöffnung auf, wobei die Sensorik des Erkennungssystems so eingerichtet und insbesondere so angeordnet ist, dass diese ein medizinisches Zubehör, welches zur Entsorgung durch die Entsorgungsöffnung in den Entsorgungsbehälter gelangt, - insbesondere mittels optischer Erfassung oder mittels Erfassung des Gewichts- erfasst.

Vorzugsweise ist das Erkennungssystem eingerichtet, die durchgeführte Behandlung mittels der Behandlungsdatenbank und/oder der Zubehördatenbank auf Basis einer oder mehrerer bei der Entsorgung erfassten medizinischen Zubehöre zu bestimmen. Alternativ kann die durchgeführte Behandlung auch, vorzugsweise durch Eingaben des Benutzers, festgelegt und insbesondere deren Behandlungskennung bestimmt sein.

Zudem ist das Erkennungssystem vorzugsweise eingerichtet, den durchgeführten Behandlungsschritt mittels der Behandlungsdatenbank und/oder der Zubehördatenbank auf Basis einer oder mehrerer bei der Entsorgung erfassten medizinischen Zubehöre sowie vorzugsweise auf Basis der Behandlungskennung zu bestimmen. Insbesondere lässt sich der Suchraum für mögliche Behandlungsschritte durch die Behandlungskennung verkleinern, womit insbesondere die Bestimmung des Behandlungschritts vereinfacht und/oder robuster, d.h. insbesondere weniger fehleranfällig, realisiert werden kann.

Weiter bevorzugt ist das Erkennungssystem eingerichtet, anhand eines, insbesondere behandlungsspezifische oder zubehörspezifischen, Erfolgskriteriums und auf Basis der erfassten, entsorgten medizinischen Zubehöre zu bestimmen, ob der Behandlungsschritt erfolgreich durchgeführt worden ist. So kann insbesondere ein zerrissener Einmalhandschuh mit einem nicht-erfolgreichen Anziehen des Einmalhandschuhs korrespondieren. Auch kann insbesondere eine verbogene Nadel oder ein nichtflüssigkeitsgefüllter Katheter oder Infusionsschlauch mit einer nicht-erfolgreichen Verwendung davon korrespondieren. Auch kann die Zeitspanne zwischen dem Herausgeben des medizinischen Zubehörs und dessen Entsorgung ein Erfolgskriterium sein. So kann insbesondere ein Klebebandabschnitt, welcher höchstens 30 Sekunden, vorzugsweise höchstens 5 Sekunden, nach seinem Herausgeben entsorgt worden ist, mit einer nicht-erfolgreichen Verwendung - insbesondere einem fehlgeschlagenen Aufkleben auf einen Hautbereich oder einer misslungenen Fixierung eines Katheters oder einer Kanüle mit diesem Klebebandabschnitt - korrespondieren.

Vorzugsweise ist die Entsorgungsvorrichtung als Behandlungswagenmodul ausgebildet oder ein Behandlungswagenmodul weist einen oder mehrere Bestandteile der Entsorgungsvorrichtung, insbesondere den Entsorgungsbehälter und/oder die Sensorik des Erkennungssystems, auf oder besteht daraus.

Gemäß einer bevorzugten Weiterbildung ist das Steuerungssystem eingerichtet, ein Assistenzverfahren auszuführen. Das Assistenzverfahren weist wenigstens den Verfahrensschritt Bestimmen eines oder mehrerer Assistenzparameter, welche die durchzuführende Behandlung, einen durchzuführenden Behandlungsschritt der durchzuführenden Behandlung und/oder ein medizinisches Zubehör dafür kennzeichnen, auf. Vorzugsweise bestimmt das Steuerungssystem die durchzuführende Behandlung und/oder den durchzuführenden Behandlungsschritt mittels des Erkennungssystems für Behandlungen. Bevorzugt und alternativ oder zusätzlich bestimmt das Steuerungssystem die durchzuführende Behandlung und/oder den durchzuführenden Behandlungsschritt anhand von Eingaben eines Benutzers, welche die durchzuführende Behandlung und/oder den durchzuführenden Behandlungsschritt festlegen. Ein Vorteil der Assistenzparameter kann insbesondere darin liegen, dass darauf basierend eine Assistenzfunktion ausgeführt und/oder realisiert werden kann, welche insbesondere den Benutzer oder den Patienten bei der Behandlung unterstützt und so die Arbeitseffizienz, die Behandlungssicherheit, die Behandlungsqualität, den Benutzungskomfort und/oder die Patientenzufriedenheit steigern kann.

Gemäß einer bevorzugten Variante weist das mobile Auswahlsystem, insbesondere das Steuerungssystem, eine Datenspeichervorrichtung für Assistenzinformationen auf und/oder die Behandlungsdatenbank ist für Datensätze mit Assistenzinformationen bezüglich einer Behandlung oder eine Behandlungsschritt eingerichtet. Zudem ist eine der Benutzerschnittstellen für die Ausgabe von Assistenzinformationen eingerichtet und/oder das mobile Auswahlsystem, insbesondere einer der Behandlungswagen, weist eine zusätzliche Benutzerschnittstelle für Assistenzinformationen auf. Außerdem weist das Assistenzverfahren des Weiteren einen Verfahrensschritt auf, bei welchem auf Basis der Assistenzparameter Assistenzinformationen, welche mit den Assistenzparametern korrespondieren, aus der Datenspeichervorrichtung für Assistenzinformationen bzw. aus der Behandlungsdatenbank gelesen werden, und einen Verfahrensschritt, bei welchem das Steuerungssystem diese Assistenzinformationen mittels der Benutzerschnittstelle für Assistenzinformationen ausgibt.

Im Sinne der Erfindung können "Assistenzinformationen" insbesondere akustische oder visuelle Instruktionen über die durchzuführende Behandlung, die Behandlungsschritte einer Behandlung und/oder deren Abfolge, den jeweils durchzuführenden Behandlungsschritt und/oder die medizinischen Zubehöre sein. Auch können solche Assistenzinformationen Instruktionen darüber sein, wie die Behandlung oder der Behandlungsschritt jeweils durchzuführen ist und/oder wie ein bestimmtes medizinisches Zubehör, insbesondere für die Behandlung oder den Behandlungsschritt, zu verwenden ist. Auch können solche Assistenzinformationen Informationen über den zu behandelnden Patienten sein. Solche Patienteninformationen können insbesondere Informationen über Patientenmerkmale sein und/oder als Patientendaten codiert sein. Vorzugsweise können so bei einem Dialysepatienten das Vorhandensein einer arteriovenösen Fistel oder eines Prothesenshunts und/oder die zuletzt verwendeten Einstichstellen für den Gefäßzugang und/oder empfohlene Einstichstellen gespeichert sein; und des Weiteren die Assistenzfunktionen Informationen bzw. Instruktionen darüber sein, wo bzw. wie bei dem Patienten der Gefäßzugang zum Blutkreislauf für die Dialyse hergestellt werden soll, insbesondere an welcher Stelle eine Kanüle eingestochen und/oder ein Katheter gesetzt werden soll.

Vorzugsweise weist das mobile Auswahlsystem, insbesondere das Steuerungssystem, eine Patientendatenbank für die Patienteninformationen und eine Datenspeichervorrichtung für diese auf.

Ein Vorteil der Ausgabe von spezifischen Assistenzinformationen - also insbesondere dieser Assistenzfunktion - kann insbesondere darin liegen, dass eine an der Behandlung beteiligte Person damit unterstützt werden kann, wodurch sich insbesondere Fehler vermeiden lassen, die Behandlungsqualität gesteigert werden kann und/oder es ermöglicht wird, dass der Patient - als eine an der Behandlung beteiligte Person und geleitet durch die Assistenzinformationen - die Behandlung oder Behandlungsschritte selbst durchführen kann. Durch diese aktive Teilhabe des Patienten lässt sich insbesondere die Patientenzufriedenheit erhöhen und/oder das Personal entlasten.

Gemäß einer bevorzugten Variante ist das Steuerungssystem eingerichtet, den wenigstens einen Herausgabemechanismus wenigstens auf Basis der Zubehörsetparameter und der Assistenzparameter so zu steuern, dass jene medizinische Zubehöre des Zubehörsets herausgegeben werden, welche für den durchzuführenden Behandlungsschritt erforderlich sind und/oder welche nach dem aktuell verwendeten medizinischen Zubehör zu verwenden sind. Bevorzugt und alternativ oder zusätzlich ist der wenigstens eine Herausgabemechanismus eingerichtet, die medizinischen Zubehöre abhängig von den Assistenzparametern herauszugeben. Bevorzugt und alternativ oder zusätzlich ist der wenigstens eine Herausgabemechanismus eingerichtet, gemäß der Steuerung des Steuerungssystems und/oder auf Basis der Assistenzparameter jene Lagerbereiche anzuzeigen, in welchem die medizinischen Zubehöre des Zubehörsets lagern, die für den durchzuführenden Behandlungsschritt erforderlich sind und/oder die nach dem aktuell verwendeten medizinischen Zubehör zu verwenden sind. Ein Vorteil der, insbesondere automatisierten, Herausgabe auf Basis der Assistenzparameter - also insbesondere dieser Assistenzfunktion - kann insbesondere darin liegen, dass die benötigten medizinischen Zubehöre abhängig von dem Verlauf der Behandlung herausgegeben werden, womit sich insbesondere Verwechslungen und/oder Arbeitsschritte zum Herausnehmen oder Lokalisieren von medizinischen Zubehören vermeiden lassen. Außerdem lässt sich durch die Anzeige der Lagerbereiche das Lokalisieren von medizinischen Zubehören bzw. von Lagerbereichen mit den benötigten medizinischen Zubehören erleichtern.

Gemäß einer bevorzugten Variante mit einer Entsorgungsvorrichtung für medizinische Zubehöre weist die Entsorgungsvorrichtung eine verschließbare Entsorgungsöffnung und einen Verschlussmechanismus für die Entsorgungsöffnung auf. Dabei ist das Steuerungssystem eingerichtet, den Verschlussmechanismus so zu steuern, dass diese die Entsorgungsöffnung abhängig von den Assistenzparametern, insbesondere für bestimmte Behandlungsschritte, öffnet und/oder abhängig von den Assistenzparametern, insbesondere nach der Behandlung oder für den Transport, verschließt. Hierbei ist die Assistenzfunktion insbesondere das automatisierte Öffnen und/oder Schließen der Entsorgung öffnen. Auf diese vorteilhafte Weise lässt sich der Benutzungskomfort steigern und/oder die Sicherheit erhöhen.

Gemäß einer bevorzugten Weiterbildung weist das mobile Auswahlsystem, insbesondere einer der Behandlungswagen, eine Aufzeichnungsvorrichtung mit einer Bildsensoreinrichtung auf, welche eingerichtet ist, eines oder mehrere Bilder, insbesondere eine Bildersequenz oder einen Videofilm, vor nach oder während der Behandlung mittels der Bildsensoreinrichtung zu erfassen und mittels einer Datenspeichervorrichtung für Bildaufzeichnungen aufzuzeichnen und/oder zur Speicherung mittels einer Datenverbindung an das Steuerungssystem zu übertragen. Auf diese vorteilhafte Weise lässt sich die Behandlung überwachen und/oder, etwa zur Evaluierung oder Qualitätsverbesserung, später nachvollziehen. Außerdem kann ein Patient, welcher die Behandlung oder Schritte davon insbesondere in Heimanwendung durchführt, bei der Behandlung von einem medizinischen Personal, das nicht am Ort des Patienten ist, überwacht und, vorzugsweise mit einer Kommunikationsverbindung in Gegenrichtung, unterstützt werden. Gemäß einer bevorzugten Variante ist die Aufzeichnungsvorrichtung als Behandlungswagenmodul ausgebildet oder ein Behandlungswagenmodul weist einen oder mehrere Bestandteile der Aufzeichnungsvorrichtung, insbesondere die Bildsensoreinrichtung, auf. Insbesondere kann eine solche Bildsensoreinrichtung ein Kamera-Modul sein.

Gemäß einer bevorzugten Weiterbildung weist das mobile Auswahlsystem, insbesondere einer der Behandlungswagen, ein Vorratsüberwachungssystem für den Behandlungswagen bzw. für den einen der Behandlungswagen auf. Das Vorratsüberwachungssystem ist eingerichtet, für wenigstens einen der Lagerbereiche die Anzahl an vorrätigen medizinischen Zubehören der dort gelagerten Zubehörgattung zu bestimmen. Zudem ist das Steuerungssystem eingerichtet, ein Bestandsbestimmungsverfahren mit dem Verfahrensschritt Bestimmen eines tatsächlichen Bestands an vorrätigen medizinischen Zubehören der Mehrzahl an medizinischen Zubehören, insbesondere wenigstens bezüglich des wenigstens einen Lagerbereichs, mittels des Vorratsüberwachungssystems auszuführen.

Vorzugsweise werden Daten bezüglich der in dem Lagerbereich gelagerten medizinischen Zubehören mittels einer auf Messung von Strahlung, insbesondere Licht, und/oder von Gewicht basierenden Sensorik des Vorratsüberwachungssystems erfasst und basierend darauf die Anzahl bestimmt. Bevorzugt und zusätzlich oder alternativ wird die Anzahl durch Zählen des laufenden Verbrauchs der medizinischen Zubehöre dieser Zubehörgattung bestimmt - d.h. insbesondere bei dem Herausgeben die Anzahl beginnend von einem Ausgangswert jeweils um die Zahl der herausgegebenen medizinischen Zubehöre dieser Zubehörgattung reduziert.

Vorzugsweise ist das Vorratsüberwachungssystem eingerichtet, wenigstens für alle Lagerbereiche, für welche ein Herausgabemechanismus zur Herausgabe von medizinischen Zubehören aus diesen eingerichtet ist, jeweils die Anzahl an vorrätigen medizinischen Zubehören zu bestimmen, und das Steuerungssystem ist eingerichtet, den tatsächlichen Bestand wenigstens bezüglich dieser Lagerbereiche zu bestimmen.

Ein Vorteil der Bestimmung des tatsächlichen Bestands mittels des Vorratsüberwachungssystems kann insbesondere darin liegen, dass es ermöglicht wird, die Auswahl und Herausgabe von medizinischen Zubehören an den tatsächlichen Bestand anzupassen, womit insbesondere die Behandlungssicherheit, Effizienz und/oder Behandlungsqualität gesteigert werden kann, und/oder - insbesondere gegenüber einer manuellen Bestimmung des tatsächlichen Bestands - das Personal entlastet und/oder Fehlerquellen reduziert werden können.

Gemäß einer bevorzugten Weiterbildung ist eine der Lagereinrichtungen eingerichtet, mit einem Zubehörträger, welcher einen oder mehrere Lagerbereiche, die jeweils mit medizinischen Zubehören von jeweils einer Zubehörgattung bestückt sind, aufweist, bestückt zu werden, d.h. insbesondere diesen Zubehörträger formschlüssig aufzunehmen. Auf diese vorteilhafte Weise lässt sich der Bestand an medizinischen Zubehören bei dieser Lagereinrichtung durch Bestücken mit dem Zubehörträger auffüllen bzw. erneuern und sich insbesondere so vermeiden, dass die medizinischen Zubehöre der Zubehörgattungen einzeln in den Lagerbereichen angeordnet werden müssen. Insbesondere kann dabei der Zubehörträger von einem Bestückungssystem, insbesondere des mobilen Auswahlsystems, bestückt werden.

Ein untergeordneter Aspekt der Erfindung betrifft ferner ein Verfahren zur Bestückung eines Behandlungswagens, aufweisend die folgenden Verfahrensschritte: - Bestimmen eines Sollwertes bezüglich der Anzahl an medizinischen Zubehören einer Zubehörgattung und eines Istwertes bezüglich des tatsächlichen Bestands dieser medizinischen Zubehöre in dem Behandlungswagen, insbesondere in einem Lagerbereich einer Lagereinrichtung des Behandlungswagens; - Falls der Istwert kleiner dem Sollwert ist, Ausgeben eines Signals für Zubehörmangel, welches einen Mangel an medizinischen Zubehören dieser Zubehörgattung kennzeichnet.

Vorzugsweise wird das Signal an einer Benutzerschnittstelle ausgegeben und ein Benutzer des Behandlungswagens wird auf diese vorteilhafte Weise bei dem Bestücken des Behandlungswagens unterstützt. Vorzugsweise werden dabei zusätzlich Assistenzinformationen mit Instruktionen bezüglich der zu bestückenden medizinischen Zubehöre, deren Bevorratung in einem externen Lager, und/oder des Lagerbereichs, der diesen bestückt medizinischen Zubehören werden soll, ausgegeben.

Bevorzugt und alternativ oder zusätzlich wird das Signal ausgegeben mittels einer Datenverbindung zu einem Bestückungssystem. Dabei ist dieses Bestückungssystem eingerichtet einen Zubehörträger auf Basis dieses Signals zu bestücken und/oder, den Behandlungswagen, sofern dieser benachbart zu dem Bestückungssystem angeordnet ist, mit den medizinischen Zubehören zu bestücken, für deren Zubehörgattungen der Mangel signalisiert worden ist. Vorzugsweise kann der Behandlungswagen dazu mit dem Zubehörträger bestückt werden.

Ein zweiter Aspekt der Erfindung betrifft einen Behandlungswagen für ein mobiles Auswahlsystem gemäß dem ersten Aspekt der Erfindung. Der Behandlungswagen weist eine oder mehrere Lagereinrichtungen, wobei die Lagereinrichtungen wenigstens zwei Lagerbereiche für medizinische Zubehöre je von einer Zubehörgattung aufweisen, und wenigstens einen Herausgabemechanismus zur Herausgabe von medizinischen Zubehören auf.

Die bereits vorausgehend genannten möglichen Vorteile sowie Ausführungsformen, Weiterbildungen oder Varianten des ersten Aspekts der Erfindung gelten entsprechend auch für den erfindungsgemäßen Behandlungswagen. Umgekehrt gelten nachfolgend genannte mögliche Vorteile sowie Ausführungsformen, Weiterbildungen oder Varianten eines Behandlungswagens gemäß einer Ausführungsform des zweiten Aspekts der Erfindung entsprechend auch für ein mobiles Auswahlsystem gemäß einer Ausführungsform des ersten Aspekts der Erfindung mit einem solchen Behandlungswagen.

Gemäß einer bevorzugten Weiterbildung weist der Behandlungswagen eine der Benutzerschnittstellen des mobilen Auswahlsystems auf, d.h. insbesondere der Behandlungswagen weist eine Benutzerschnittstelle auf, welche entsprechend einer Benutzerschnittstelle des ersten Aspekts der Erfindung - vorzugsweise für Eingaben zur Festlegung einer durchzuführenden Behandlung - eingerichtet ist. Zudem weist der Behandlungswagen eine Ansteuerungsvorrichtung auf, welche zumindest einen Teil des Steuerungssystems des mobilen Auswahlsystems und/oder eine Kommunikationseinrichtung zur Ausbildung einer Datenverbindung mit dem Steuerungssystem bzw. einem anderen Teil des Steuerungssystems aufweist. Dabei ist die Ansteuerungsvorrichtung für die Steuerung des wenigstens einen Herausgabemechanismus durch das Steuerungssystem eingerichtet, den wenigstens einen Herausgabemechanismus auf Basis der Zubehörsetparameter elektrisch anzusteuern. Dazu weist der Behandlungswagen vorzugsweise ein Bussystem auf und die Ansteuerungsvorrichtung ist zur Ausbildung einer Datenverbindung mit dem wenigstens einen Herausgabemechanismus über das Bussystem eingerichtet und/oder weist eine Datenverbindung mit dem wenigstens einen Herausgabemechanismus über das Bussystem auf. Insbesondere sind die Benutzerschnittstelle und die Ansteuerungsvorrichtung so mit dem Behandlungswagen mechanisch verbunden oder verbindbar, dass diese zusammen mit den übrigen Bestandteilen des Behandlungswagens transportiert werden können. Vorzugsweise sind die Benutzerschnittstelle und/oder die Ansteuerungsvorrichtung als ein Behandlungswagenmodul ausgebildet.

Dabei ermöglicht die Benutzerschnittstelle des Behandlungswagens auf vorteilhafte Weise, den Behandlungswagen zu bedienen, insbesondere ohne dass eine dem Behandlungswagen externe Benutzerschnittstelle erforderlich ist.

Ein Vorteil des Behandlungswagens mit Ansteuerungsvorrichtung kann insbesondere darin liegen, dass die Steuerung, welche von dem Steuerungssystem ausgeht, bei dem Behandlungswagen mittels der Ansteuerungsvorrichtung elektrisch, d.h. insbesondere mittels elektrischer Signale, erfolgt, wobei insbesondere sich die elektrische Ansteuerung, vorzugsweise mit elektrischen Signalleitungen, etwa als Bestandteil eines Bussystems, gegenüber einer mechanischen Ansteuerung technisch robuster und/oder kostengünstiger realisieren lassen.

Ein Vorteil einer bevorzugten Variante, bei welcher der Behandlungswagen das Steuerungssystem oder zumindest Bestandteile davon nicht aufweist und/oder dieses bzw. diese mit dem Behandlungswagen nicht mechanisch verbunden sind, kann insbesondere darin liegen, dass das Steuerungssystem oder Bestandteile davon an einem anderen Ort als dem des Behandlungswagens angeordnet sein können und den Behandlungswagen von dort aus und/oder ohne mechanische Verbindung steuern können. Auf diese vorteilhafte Weise lassen sich insbesondere das Gewicht des Behandlungswagens reduzieren, die Herstellungskosten senken und/oder teure oder empfindliche Komponenten des Steuerungssystems an einem Ort anordnen, welcher dafür besonders geeignet ist - etwa einem Steuerungsraum oder Server-Raum. Überdies kann ein solches Steuerungssystem eingerichtet sein, mehrere Behandlungswagen jeweils über deren Ansteuerungsvorrichtung zu steuern.

Gemäß einer bevorzugten Variante kann die Ansteuerungsvorrichtung auch Teile der Steuerung, insbesondere eine Regelung des wenigstens einen Herausgabemechanismus, ausführen und dazu insbesondere wenigstens einen Regelkreis aufweisen. Auf diese vorteilhafte Weise kann die Steuerung an diesen Behandlungswagen mittels der Ansteuerungsvorrichtung angepasst werden und/oder es wird eine schnelle Rückkopplung bei der Steuerung, insbesondere Regelung, ermöglicht, insbesondere im Vergleich zu einem an einem anderen Ort angeordneten Steuerungssystem, welches die Steuerung und insbesondere Regelung ausführt.

Ein Vorteil einer bevorzugten Variante, bei welcher die Ansteuerungsvorrichtung das gesamte Steuerungssystem aufweist, kann insbesondere darin liegen, dass ein solcher Behandlungswagen unabhängig von weiteren Bestandteilen eines mobilen Auswahlsystems betrieben werden kann. Auch kann bei einer solchen Variante die Ansteuerungsvorrichtung bzw. das Steuerungssystem, welches der Behandlungswagen aufweist, eingerichtet sein, wenigstens einen weiteren Behandlungswagen zu steuern.

Gemäß einer bevorzugten Weiterbildung weist der Behandlungswagen einen Energiespeicher zur Versorgung des Behandlungswagens mit Energie auf. Vorzugsweise weist der Energiespeicher eine unabhängige Spannungsquelle, insbesondere eine wiederaufladbare Batterie, zur Versorgung des Behandlungswagens mit elektrischer Energie auf oder besteht daraus. Auf diese vorteilhafte Weise ist der Behandlungswagen, zumindest zeitweise bis zur Erschöpfung des Energiespeichers, von einer externen Energieversorgung unabhängig. Auch lässt sich ein solcher Behandlungswagen mit eigenem Energiespeicher an Standorten einsetzen, welche keine externe Energieversorgung ermöglichen, und/oder ohne Installation, d.h. insbesondere Anschluss an eine externe Energieversorgung, am jeweiligen Standort betreiben.

Gemäß einer bevorzugten Weiterbildung weist der Behandlungswagen eine Anschlusseinrichtung zum Anschluss an eine externe Energieversorgung auf, mittels welcher der Behandlungswagen mit Energie versorgt wird. Vorzugsweise weist die Anschlusseinrichtung einen Stecker für eine Steckdose des Stromnetzes und insbesondere ein Stromkabel auf oder besteht daraus. Auf diese vorteilhafte Weise lässt sich die Energie zum Betrieb des Behandlungswagens extern bereitstellen, wodurch insbesondere Kosten reduziert werden können und/oder die Betriebsdauer verlängert werden kann, da sich insbesondere ein vorzugsweise zusätzlich vorhandener eigener Energiespeicher des Behandlungswagens während der externen Energieversorgung nicht erschöpft und weiter bevorzugt auflädt.

Ein dritter Aspekt der Erfindung betrifft ein Verfahren zur Auswahl von medizinischen Zubehören aus einer Mehrzahl an medizinischen Zubehören, insbesondere für eine Kanülierung von Blutgefäßen von Patienten und/oder für eine Dialysebehandlung. Dabei ist ein Teil des Verfahrens zur Auswahl von medizinischen Zubehören ein Verfahren zur Herausgabe dieser medizinischen Zubehöre aus einem Behandlungswagen mit mehreren Lagereinrichtungen. Dabei lagern die medizinischen Zubehöre der Mehrzahl an medizinischen Zubehören gemäß ihrer jeweiligen Zubehörgattungen sortiert in wenigstens zwei Lagerbereichen der Lagereinrichtungen. Das erfindungsgemäße Verfahren weist die folgenden Verfahrensschritte auf. In einem Verfahrensschritt wird eine oder werden mehrere Eingaben von einem Benutzer, durch die eine durchzuführende Behandlung festgelegt wird, mittels einer Benutzerschnittstelle erfasst, die in Datenverbindung mit einem Steuerungssystem steht. In einem weiteren Verfahrensschritt wird eine Behandlungskennung der durchzuführenden Behandlung auf Basis dieser Eingaben mittels des Steuerungssystems bestimmt. In einem weiteren Verfahrensschritt wird oder werden mittels des Steuerungssystems einer bzw. mehrere Zubehörsetparameter, welche ein medizinisches Zubehörset aus einem oder mehreren für die durchzuführende Behandlung geeigneten medizinischen Zubehören kennzeichnen, anhand einer Zubehördatenbank und wenigstens auf Basis der Behandlungskennung der durchzuführenden Behandlung bestimmt, wobei die Zubehördatenbank Datensätze über die Mehrzahl an medizinischen Zubehören aufweist und auf einer Datenspeichervorrichtung des Steuerungssystems gespeichert ist. In einem weiteren Verfahrensschritt, insbesondere des Verfahrens zur Herausgabe, wird ein Herausgabemechanismus, der zur Herausgabe von medizinischen Zubehören aus wenigstens einer der Lagereinrichtungen eingerichtet ist und der in Datenverbindung mit dem Steuerungssystem steht, auf Basis der Zubehörsetparameter gesteuert. In einem weiteren Verfahrensschritt, insbesondere des Verfahrens zur Herausgabe, werden mittels des Herausgabemechanismus jene medizinischen Zubehöre, für deren Zubehörgattungen die wenigstens eine der Lagereinrichtungen Lagerbereiche aufweist und welche durch die Zubehörsetparameter, auf denen die Steuerung des Herausgabemechanismus basiert, gekennzeichnet sind, herausgegeben.

Die bereits vorausgehend genannten möglichen Vorteile sowie Ausführungsformen, Weiterbildungen oder Varianten des ersten und/oder des zweiten Aspekts der Erfindung gelten entsprechend auch für das erfindungsgemäße Verfahren zur Auswahl von medizinischen Zubehören.

Im Sinne der Erfindung ist ein "medizinisches Zubehör" - oder kurz "Zubehör" - eine Einrichtung, ein Instrument, ein Material, ein Stoff oder eine Kombination daraus, welche zur Behandlung eines Patienten vorbestimmt und/oder verwendbar ist. Insbesondere kann ein medizinisches Zubehör bei der bestimmungsgemäßen Verwendung verbraucht werden. Alternativ kann ein medizinisches Zubehör bei der bestimmungsgemäßen Verwendung nicht verbraucht werden, d.h. für mehrere Behandlungen eines oder mehrerer Patienten verwendet werden.

Insbesondere kann ein medizinisches Zubehör ein Arzneimittel sein, welches üblicherweise bei bestimmungsgemäßer Verwendung zumindest im Wesentlichen pharmakologisch, metabolisch oder immunologisch wirkt. Bevorzugt weist ein solches Arzneimittel eine Darreichungsform, vorzugsweise etwa Kapseln, Pillen, Tabletten, Pasten, Salben, Injektionsdosen oder Arzneimittellösungen, mit einer für die Behandlung vorbestimmten Dosierung, Dosiereinrichtung, Verabreichungseinrichtung und/oder Aufbewahrungseinrichtung auf, vorzugsweise etwa Kapseln oder Tabletten in einer Blisterpackung, Spritzen mit einer Einfach- oder Mehrfachdosis eines injizierten Arzneimittels, Arzneimittelsäfte in Flaschen mit oder ohne Messbecher für die Dosierung oder Arzneimittelzubereitungen in einer Sprayflasche mit Sprühsystem.

Insbesondere kann ein medizinisches Zubehör auch ein Medizinprodukt sein, welches üblicherweise bei bestimmungsgemäßer Verwendung zumindest im Wesentlichen physisch, physikalisch oder physikochemisch wirkt und/oder mittels dessen ein Arzneimittel verabreicht wird. Solche Medizinprodukte sind insbesondere chirurgische Instrumente wie Wundhaken, Retraktoren, Spreizer, Skalpelle, Scheren oder Pinzetten. Auch sind solche Medizinprodukte insbesondere Nadeln, Spritzen, Kanülen, Seldinger-Drähte oder Katheter.

Auch Schutzausrüstung für den Patienten oder die an der Behandlung beteiligten Personen kann ein medizinisches Zubehör im Sinne der Erfindung sein. Solche Schutzeinrichtungen sind insbesondere Handschuhe zum chemischen Schutz oder Infektionsschutz, vorzugsweise Einmalhandschuhe aus Latex, Nitrilkautschuk oder Vinyl, ein medizinischer Mundschutz, medizinische Haarnetze oder Schutzbrillen.

Insbesondere können auch Desinfektionsmittel, Verbandmaterialien oder kosmetische Mittel ein medizinisches Zubehör im Sinne der Erfindung sein.

Medizinisches Zubehör im Sinne der Erfindung ist insbesondere eine Einrichtung, ein Instrument, ein Material, ein Stoff oder eine Kombination daraus zur Kanülierung eines Patienten, vorzugsweise etwa für eine Dialysebehandlung. Dies sind insbesondere Spritzen, Kanülen, Infusionsschläuche, Einmalhandschuhe, Desinfektionsmittel, Tupfer, Verbandmaterial, Pflaster, Tapes und Klebebänder.

Außerdem weist das medizinische Zubehör im Sinne der Erfindung räumliche Abmessungen und ein Gewicht auf, welche einen Transport und/oder eine Handhabung des medizinischen Zubehörs durch eine an der Behandlung beteiligte Person ermöglicht. So beträgt insbesondere das Gewicht eines solchen medizinischen Zubehörs höchstens 10 kg, vorzugsweise höchstens 1 kg, vorzugsweise höchstens 300 g, vorzugsweise höchstens 100 g und weiter bevorzugt höchstens 30 g. Die räumlichen Abmessungen eines solchen medizinischen Zubehörs haben insbesondere eine Länge von höchstens 1,5 m, vorzugsweise höchstens 1 m, vorzugsweise höchstens 0,5 m, vorzugsweise höchstens 30 cm, vorzugsweise höchstens 15 cm und weiter bevorzugt höchstens 5 cm, wobei entsprechendes für die Breite und Höhe gilt. Dabei versteht es sich insbesondere, dass ein solches medizinisches Zubehör, etwa ein Infusionsschlauch oder ein Tape durch Ausrollen, Einrollen oder Abtrennen, auch in seinen räumlichen Abmessungen veränderbar sein kann, wobei es für die Handhabung bzw. den Transport darauf ankommt, dass dazu ein solches medizinisches Zubehör in seinen räumlichen Abmessungen so veränderbar ist, dass dieses höchstens die oben angegebene Länge, Breite und Tiefe aufweist.

Vorzugsweise kann das medizinische Zubehör im Sinne der Erfindung eingerichtet und insbesondere so geformt sein, dass eine Behandlungsvorrichtung für eine Behandlung eines Patienten dieses medizinische Zubehör zur Behandlung verwenden, also insbesondere aufnehmen, betätigen, manipulieren, abgeben und/oder applizieren, kann.

Ein "medizinisches Zubehörset" - oder kurz "Zubehörset" - im Sinne der Erfindung ist eine Zusammenstellung mit einem oder vorzugsweise mehreren medizinischen Zubehören. Vorzugsweise ist ein medizinisches Zubehörset für eine bestimmte Behandlung und/oder einen bestimmten Patienten vorgesehen und weist dazu wenigstens ein medizinisches Zubehör auf, welches für diesen Patienten und/oder diese Behandlung erforderlich, besonders geeignet und/oder ausgewählt worden ist.

Unter einem "medizinischen Zubehörträger" - oder kurz "Zubehörträger" - ist im Sinne der Erfindung zumindest eine Vorrichtung zum Tragen und/oder zum Aufhängen eines medizinischen Zubehörsets zu verstehen.

Vorzugsweise kann der Zubehörträger eines, vorzugsweise wenigstens zwei, weiter vorzugsweise alle medizinischen Zubehöre des Zubehörsets aufweisen, also insbesondere mit diesen medizinischen Zubehören bestückt sein. Insbesondere kann der Zubehörträger zumindest mit einem Teil eines Zubehörsets vorbestückt sein, sodass dieser bereits zumindest diesen Teil des Zubehörsets aufweist. Auf diese Weise lassen sich Zubehörträger mit bestimmten, insbesondere häufig benötigten, Zubehörsets bereitstellen, insbesondere ohne dass diese jeweils einzeln spezifisch auf eine Anforderung oder Auswahl hin bestückt werden müssen. Auch kann ein solcher Zubehörträger dafür vorgesehen sein, noch mit einem weiteren Teil eines Zubehörsets bestückt zu werden, womit ein solcher Zubehörträger mit verschiedenen medizinischen Zubehörsets, insbesondere fertig, bestückt werden kann, wobei der Zubehörträger einerseits mit den jeweils spezifischen Zubehören des Zubehörsets einzeln bestückt wird während andererseits jener Teil des Zubehörsets oder einer Gruppe von medizinischen Zubehörsets, welcher diesen gemeinsam ist und/oder häufig benötigt wird, nicht einzeln spezifisch auf eine Anforderung oder Auswahl hin bestückt werden muss.

Auch vorzugsweise kann der Zubehörträger eines, vorzugsweise wenigstens zwei, weiter vorzugsweise alle medizinischen Zubehöre zur Bestückung einer Lagereinrichtung eines Behandlungswagens aufweisen, also insbesondere mit diesen medizinischen Zubehören bestückt sein. Auf diese vorteilhafte Weise kann der Bestand an medizinischen Zubehören dieser Lagereinrichtung durch Bestücken der Lagereinrichtung mit diesem bestückten Zubehörträger aufgefüllt bzw. erneuert werden.

Insbesondere kann ein Zubehörträger eine Aufnahmeeinrichtung, welche eingerichtet ist, eines, vorzugsweise wenigstens zwei und weiter vorzugsweise alle medizinischen Zubehöre des Zubehörsets aufzunehmen, insbesondere sich also mit diesen formschlüssig, kraftschlüssig oder stoffschlüssig lösbar zu verbinden und/oder diese zu umschließen und/oder wobei also diese medizinischen Zubehöre in der Aufnahmeeinrichtung angeordnet sind. Vorzugsweise weist ein Zubehörträger eine oder mehrere Halteeinrichtungen jeweils zur Aufnahme eines medizinischen Zubehörs des Zubehörsets auf. Insbesondere können dabei medizinische Zubehöre für eine bestimmte Behandlung auf eine bestimmte Weise, vorzugsweise auf Basis der Reihenfolge ihrer Verwendung bei der bestimmten Behandlung, angeordnet sein.

Insbesondere kann der Zubehörträger als medizinischer Zubehörbehälter ausgebildet sein, wobei dieser oder die Aufnahmeeinrichtung des Zubehörträgers einen Behälter aufweist und/oder daraus besteht, welcher eines, vorzugsweise wenigstens zwei und weiter vorzugsweise alle medizinischen Zubehöre aufnimmt, also insbesondere räumlich umschließt. Ein solcher Zubehörbehälter ist vorzugsweise kastenförmig geformt und wird insbesondere als Zubehörbox bezeichnet.

Vorzugsweise ist ein Zubehörträger eingerichtet und insbesondere so geformt, dass eine Behandlungsvorrichtung für die Behandlung, für welche der Zubehörträger bestimmt ist, oder eine an der Behandlung beteiligte Person diesen Zubehörträger verwenden, also insbesondere aufnehmen, öffnen, betätigen, manipulieren, abgeben und/oder medizinisches Zubehör des Zubehörsets, mit welchen der Zubehörträger bestückt ist, entnehmen, kann.

Insbesondere kann auch ein Zubehörträger selbst, speziell ein bestückter Zubehörträger, ein medizinisches Zubehör sein, welches insbesondere die medizinischen Zubehöre, mit welchen der Zubehörträger bestückt ist, zusammenfasst, für eine Behandlung bereitstellt und/oder ihre Funktionen bereitstellt.

Unter einer "Behandlung eines Patienten" ist im Sinne der Erfindung zumindest ein medizinisches, d.h. insbesondere therapeutisches oder diagnostisches, oder kosmetisches Verfahren zu verstehen, welches den Körper und/oder die Gesundheit des Patienten verändert oder mittels dessen der Gesundheitszustand des Patienten bestimmt wird. Eine Behandlung ist insbesondere eine Verabreichung von Arzneimitteln, eine Kanülierung, ein Blutreinigungsverfahren wie die Dialyse, eine Operation und/oder eine Untersuchung des Patienten.

Eine "Gruppe von Behandlungen" im Sinne der Erfindung können jeweils bestimmte Operationen, die Therapie einer bestimmten Erkrankung, die Eingangsuntersuchung eines Patienten oder eine Dialyse-Behandlung, welche wiederum Untergruppen, insbesondere Behandlung mittels Hämodialyse, Hämofiltration, Hämodiafiltration, Hämoperfusion oder Peritonealdialyse, aufweisen kann, sein.

Unter einer "an der Behandlung beteiligten Person" ist im Sinne der Erfindung insbesondere eine behandelnde Person, etwa ein Arzt oder eine Ärztin, oder eine Person, die die Behandlung unterstützt, etwa eine Pflegekraft, zu verstehen. Insbesondere kann auch der zu behandelnde Patient selbst eine an der Behandlung beteiligte Person oder behandelnde Person sein.

Eine "Gruppe von Patienten" im Sinne der Erfindung können insbesondere jeweils männliche oder weibliche Patienten, Patienten mit einer bestimmten chronischen Erkrankung, Patienten mit einer bestimmten Blutgruppe, Patienten mit einem bestimmten Hauttyp, Dialyse-Patienten mit arteriovenöser Fistel (AV-Fistel) - d.h. mit Cimino-Shunt-, Dialyse-Patienten mit einem Prothesenshunt (AV-Graft) - d.h. insbesondere mit Scribner-Shunt - oder Dialyse-Patienten, welche einen Zugang mittels zentralvenösen Katheter benötigen, sein.

Unter einer "Behandlungsvorrichtung" ist im Sinne der Erfindung zumindest eine Vorrichtung zu verstehen, welche eingerichtet ist, eine oder mehrere Behandlungen an einem Patienten durchzuführen und/oder zu unterstützen. Eine Behandlungsvorrichtung ist insbesondere ein Chirurgie-Roboter, eine Kanülierungsvorrichtung - insbesondere für einen vaskulären Zugang und/oder insbesondere eine Kanülierautomat -, eine Dialysevorrichtung oder eine Desinfektionsvorrichtung zur Desinfektion einer Körperregion eines Patienten.

Im Sinne der Erfindung ist eine "Lagereinrichtung" eine Einrichtung zum Lagern von Sachen, insbesondere von Einrichtungen, Instrumenten, Materialien oder Stoffen, insbesondere von medizinischen Zubehören oder Zubehörträgern. Dabei kann die Lagereinrichtung vorzugsweise die zu lagernden Sachen oder zumindest einen Teil davon, insbesondere in Zusammenwirkung mit einer die Lagereinrichtung aufweisenden Vorrichtung, aufnehmen, d.h. insbesondere räumlich umschließen oder zumindest teilweise umgeben.

Vorzugsweise weist die Lagereinrichtung, insbesondere in Zusammenwirkung mit der die Lagereinrichtung aufweisenden Vorrichtung, wenigstens einen Lagereinrichtungsteil mit einem Innenbereich auf, wobei der Lagereinrichtungsteil den Innenbereich in einem geschlossenen Zustand von der Umgebung der Lagereinrichtung räumlich und physisch trennt. Insbesondere können in dem Innenbereich zu lagernde Sachen angeordnet und damit gelagert werden und der Innenbereich in einem offenen Zustand räumlich und physisch von der Umgebung der Lagereinrichtung zugänglich sein.

Vorzugsweise weist die Lagereinrichtung wenigstens zwei Lagerbereiche auf, die räumlich voneinander getrennt sind. Auf diese Weise lassen sich verschiedene Sachen getrennt voneinander bei den wenigstens zwei Lagerbereichen lagern und insbesondere anhand der Lagerbereiche adressieren. Vorzugsweise ist ein solcher Lagerbereich als Innenbereich ausgebildet.

Vorzugsweise weist die Lagereinrichtung eine Klimatisierungseinrichtung zum Steuern - und insbesondere Regeln - der Lagerbedingungen auf, d.h. insbesondere zum Steuern und/oder Regeln der Luftfeuchtigkeit und/oder der Temperatur. Weitere Lagerbedingungen sind insbesondere ein Medium in der Lagereinrichtung bzw. in den Innenbereichen der Lagereinrichtung, vorzugsweise etwa Luft, ein Schutzgas, eine Lagerungsflüssigkeit oder ein Schüttgut für die Lagerung - wie Styroporkugeln zur Erschütterungsdämpfung -, das Vorhandensein bzw. Nicht-Vorhandensein bestimmter Chemikalien wie Säuren oder Basen sowie Strahlungseinflüsse wie sichtbares Licht, ultraviolettes Licht, Infrarotlicht, radioaktive Strahlung oder Schall. Vorzugsweise sind die Lagerbedingungen bei den Innenbereichen der Lagereinrichtung separat steuerbar bzw. regelbar.

Im Sinne der Erfindung ist eine "Datenbank" ein System zur elektronischen Datenverwaltung, welches insbesondere eine Datenbasis und einen Programmcode zum Verwalten der Datenbasis, d.h. insbesondere zum Speichern, Lesen, Abfragen, Ändern und/oder Löschen der Daten, insbesondere der Datensätze, der Datenbasis aufweist.

Vorzugsweise ist eine Datenverarbeitungsvorrichtung eingerichtet, den Programmcode von einer Datenspeichervorrichtung für die Datenbank zu laden, diesen auszuführen sowie zumindest teilweise die Datenbasis von einer Datenspeichervorrichtung für die Datenbank zu laden und die Datenbasis gegebenenfalls nach einer Änderung zu speichern. Insbesondere kann zur Abfrage der Datenbank eine, insbesondere elektronisch oder optisch codierte, Information an die Datenverarbeitungsvorrichtung gesendet werden, woraufhin mittels des Programmcodes jener Teil der Datenbasis bzw. jene Datensätze ausgegeben werden, welche mit der zugesandten Information korrespondieren. Insbesondere lassen sich so Datensätze abfragen und damit auslesen (oder bei einer datenverändernden Abfrage auch ändern), bei welchen ein Datenfeld des jeweiligen Datensatzes einen bestimmten Wert hat oder in einem bestimmten Wert im Bereich liegt. Vorzugsweise ist die Datenbank als relationale Datenbank, insbesondere als SQL-Datenbank ausgebildet.

Eine "Zubehördatenbank" im Sinne der Erfindung ist eine Datenbank für medizinische Zubehöre. Die Zubehördatenbank, insbesondere die Datenbasis, ist eingerichtet, Datensätze über medizinische Zubehöre aufzuweisen. Insbesondere kennzeichnet jeweils wenigstens ein Datensatz ein medizinisches Zubehör und/oder dessen Merkmale. Vorzugsweise ist dabei jedem in der Zubehördatenbank erfassten medizinischen Zubehör eine Zubehörkennung zugeordnet und die Zubehörkennung in den Datensätzen bezüglich des jeweiligen Zubehörs als elektronisch abrufbare und insbesondere abfragbare Information gespeichert. Auch vorzugsweise sind die Merkmale des Zubehörs entsprechend als elektronisch abrufbare und insbesondere abfragbare Information in wenigstens einem der Datensätze bezüglich des jeweiligen Zubehörs gespeichert.

Entsprechend ist eine "Patientendatenbank" im Sinne der Erfindung eine Datenbank für Patienten. Die Patientendatenbank, insbesondere ihre Datenbasis, ist eingerichtet, Datensätze über Patienten aufzuweisen. Insbesondere kennzeichnet jeweils wenigstens ein Datensatz einen Patienten- und/oder dessen Merkmale. Vorzugsweise ist dabei jedem in der Patientendatenbank erfassten Patienten eine Patientenkennung zugeordnet die Patientenkennung in den Datensätzen bezüglich des jeweiligen Patienten als elektronisch abrufbare und insbesondere abfragbare Information gespeichert. Auch vorzugsweise sind die Merkmale des Patienten entsprechend als elektronisch abrufbare und insbesondere abfragbare Informationen in wenigstens einem der Datensätze bezüglich des jeweiligen Patienten gespeichert. Vorzugsweise betrifft wenigstens ein Merkmal die Eignung eines medizinischen Zubehörs für die Behandlung des Patienten. Auch vorzugsweise betrifft wenigstens ein Patientenmerkmal das Geschlecht des Patienten, eine akute oder chronische Erkrankung des Patienten, Allergien des Patienten, sein Alter, seinen Namen, seine Blutgruppe, seinen Hauttyp und/oder durchgeführte Behandlungen. Auch vorzugsweise betrifft wenigstens ein Patientenmerkmal, insbesondere bei einem Dialysepatienten, die Möglichkeiten zum Gefäßzugang zum Blutkreislauf, insbesondere das Vorhandensein - und vorzugsweise die Eigenschaften - einer arteriovenösen Fistel oder eines Prothesenshunts, die zuletzt verwendeten Einstichstellen für den Gefäßzugang und/oder empfohlene Einstichstellen.

Entsprechend ist eine "Behandlungsdatenbank" im Sinne der Erfindung eine Datenbank für Behandlungen, welche vorzugsweise je durch eine Behandlungskennung gekennzeichnet sind. Vorzugsweise ist eine solche Behandlungsdatenbank, insbesondere ihre Datenbasis, eingerichtet Datensätze über Behandlungen und/oder über einen oder mehrere Behandlungsschritte wenigstens einer Behandlung aufzuweisen. Dabei sind die Behandlungsschritte vorzugsweise je durch eine Behandlungsschrittkennung gekennzeichnet.

Im Sinne der Erfindung ist eine "Kennung" eine Information, die Etwas, insbesondere eine Sache, einen Vorgang, ein Verfahren, ein Merkmal oder eine Person, kennzeichnet und die mittels einer Datenverarbeitungsvorrichtung verarbeitet werden kann, also insbesondere eine Signalfolge und/oder deren mechanische, elektrische oder optische Repräsentation in einem Datenspeicher. Dabei weist die Information vorzugsweise einen Teil auf, welcher die Information als Kennung einer bestimmten Art - insbesondere gegenüber weiteren Kennungen von anderer Art - kennzeichnet. Eine Kennung ist insbesondere eine Information über eine eindeutige Nummer, eine eindeutige Bezeichnung und/oder einen Namen, sofern dieser eindeutig ist oder mittels zusätzlicher Kennung eindeutig gemacht wird.

Eine "Behandlungskennung" im Sinne der Erfindung ist eine Kennung, welche eine bestimmte Behandlung oder eine bestimmte Gruppe von Behandlungen kennzeichnet. Insbesondere kann mittels der Behandlungskennung diese bestimmte Behandlung oder die Gruppe von Behandlungen, zu welcher eine bestimmte Behandlung zugeordnet ist, identifiziert werden. Eine Behandlungskennung ist insbesondere eine Information über eine Behandlungsnummer, eine eindeutige Bezeichnung für eine Behandlung oder eine Gruppe von Behandlungen oder ein eindeutiger oder eindeutig gemachter Name einer Behandlung. Dabei weist die Information vorzugsweise einen Teil auf, welcher die Information als Kennung einer Behandlung bzw. Gruppe von Behandlungen - insbesondere gegenüber anderen Kennungen, welche sich nicht auf eine Behandlung bzw. Gruppe von Behandlungen beziehen - kennzeichnet.

Entsprechend ist eine "Behandlungsschrittkennung" im Sinne der Erfindung eine Kennung, welche einen bestimmten Behandlungsschritt kennzeichnet.

Entsprechend ist eine "Patientenkennung" im Sinne der Erfindung eine Kennung, welche einen bestimmten Patienten oder eine bestimmte Gruppe von Patienten kennzeichnet. Eine solche Patientenkennung kann insbesondere eine Information über eine Patientennummer, eine (Kranken-) Versicherungsnummer, einen Namen des Patienten oder eine Ausweisnummer des Patienten aufweisen. Vorzugsweise ist diese Information auf einer Patientenkarte, einem elektronischen Datenspeicher und/oder auf einem Dokument für den Patienten, insbesondere als Barcode, Magnetstreifen, RFID-Tag oder alphanumerische Darstellung, gespeichert.

Entsprechend ist eine "Benutzerkennung" im Sinne der Erfindung eine Kennung, welche einen bestimmten Benutzer oder eine bestimmte Gruppe von Benutzern kennzeichnet.

Entsprechend ist eine "Zubehörkennung" im Sinne der Erfindung eine Kennung, welche ein bestimmtes medizinisches Zubehör kennzeichnet. Eine Repräsentation der Information dieser Zubehörkennung kann insbesondere auf oder mittels einer Verpackung des medizinischen Zubehörs, etwa als Barcode, RFID-Tag, gespeichert sein.

Entsprechend ist eine "Behandlungswagenkennung" im Sinne der Erfindung eine Kennung, welche einen bestimmten Behandlungswagen kennzeichnet. Vorzugsweise weist der Behandlungswagen einen, insbesondere elektronischen, Datenspeicher auf - vorzugsweise etwa einen Magnetstreifen, einen RFID-Tag, einen Barcode oder eine alphanumerische Darstellung -, in welchem seine Behandlungswagenkennung auslesbar gespeichert ist. Vorzugsweise ist der Datenspeicher integral mit dem Behandlungswagen verbunden, also insbesondere ein RFID-Tag mit dem Behandlungswagen verklebt oder ein Barcode bzw. eine alphanumerische Darstellung in den Behandlungswagen eingraviert oder auf diesen gedruckt.

Entsprechend ist eine "Behandlungswagenmodulkennung" im Sinne der Erfindung eine Kennung, welche ein bestimmtes Behandlungswagenmodul kennzeichnet.

Entsprechend ist eine "Benutzerschnittstellenkennung" im Sinne der Erfindung eine Kennung, welche eine bestimmte Benutzerschnittstelle kennzeichnet.

Ein Barcode kann auch ein zweidimensionaler Barcode, also ein so genannter "2D-Code" sein.

Im Sinne der Erfindung ist ein "Steuerungssystem" ein System, welches eine Datenverarbeitungsvorrichtung und/oder einen flüchtigen oder dauerhaften Datenspeicher, insbesondere eine Datenspeichervorrichtung, aufweist und eingerichtet ist, ein System mit dem Steuerungssystem, insbesondere das mobile Auswahlsystem, dessen Bestandteile und/oder eine oder mehrere dem System externe Vorrichtungen, d.h. insbesondere auch weitere Systeme oder Mechanismen, vorzugsweise etwa einen externen Behandlungswagen oder ein Bestückungssystem - zu steuern. Das Steuerungssystem ist vorzugsweise eingerichtet, das Auswahlverfahren und/oder das Assistenzverfahren auszuführen insbesondere durch einen für diesen Zweck geeignet ausgebildeten, von einer Datenverarbeitungsvorrichtung ausführbaren Programmcode.

Das Steuerungssystem kann durch eine einzelne Steuerungsvorrichtung gebildet sein. Vorzugsweise weist das Steuerungssystem eine Mehrzahl von Steuerungseinrichtungen auf, die eigenständige Vorrichtungen sein können oder die Bestandteile anderer Vorrichtungen des Systems sein können, insbesondere einer der Benutzerschnittstellen und/oder einer der Behandlungswagen. Insbesondere können diese Steuerungseinrichtungen teilweise oder alle in einem Netzwerk zum Datenaustausch organisiert sein. In dem Fall, dass eine der Benutzerschnittstellen eine eigene Steuerungseinrichtung aufweist und/oder einer der Behandlungswagen eine eigene Steuerungseinrichtung aufweist, können diese Steuerungseinrichtungen als Bestandteile des Steuerungssystems angesehen werden. Es ist aber auch möglich, dass das Steuerungssystem diese optional vorgesehenen Steuerungseinrichtungen nicht aufweist.

Insbesondere kann ein Behandlungswagen eine Ansteuerungsvorrichtung als eigene Steuerungseinrichtung aufweisen. Bevorzugt und alternativ oder zusätzlich ist die Ansteuerungsvorrichtung selbst ein Steuerungssystem, weist das Steuerungssystem auf oder weist Bestandteile des Steuerungssystems, insbesondere eine Steuerungseinrichtung, auf.

Das Steuerungssystem und/oder der Behandlungswagen bzw. einer der Behandlungswagen und/oder die Ansteuerungsvorrichtung und/oder die Benutzerschnittstelle bzw. eine der Benutzerschnittstellen können - insbesondere alle - in einer physischen Geräteeinheit integriert sein, können aber jeweils auch eine eigene physische Geräteeinheit sein. Eine physische Geräteeinheit kann insbesondere ein Modul, insbesondere ein Behandlungswagenmodul, sein, welches mit dem Steuerungssystem zumindest datenverbunden ist oder verbindbar ist.

Das Steuerungssystem und/oder eine der Benutzerschnittstellen und/oder die Ansteuerungsvorrichtung oder Bestandteile dieser Komponenten können auch zumindest teilweise durch Softwarefunktionen implementiert sein oder können insbesondere teilweise Programmcode implementieren. Ein Behandlungswagen kann eine Ansteuerungsvorrichtung aufweisen, die in Kombination mit Softwarefunktionen eine oder mehrere Funktionen des Steuerungssystems und/oder des Behandlungswagens jeweils zumindest teilweise implementiert. Entsprechend kann eine Benutzerschnittstelle eine Steuerungseinrichtung aufweisen, die in Kombination mit Softwarefunktionen eine oder mehrere Funktionen des mobilen Auswahlsystems und/oder des Steuerungssystems und/oder des Behandlungswagens jeweils zumindest teilweise implementiert.

Im Sinne der Erfindung erfolgt ein "Steuern", insbesondere das Steuern durch ein Steuerungssystem, vorzugsweise auf Basis einer Kennung, einer Eingangsgröße, eines oder mehrerer Parameter oder einer Kombination daraus. Auch kann das Steuern ein Regeln beinhalten. So kann etwa ein Steuerungssystem auf Basis von Zubehörsetparametern einen Herausgabemechanismus für eine Lagereinrichtung, welche als Schubfach ausgebildet ist, so steuern, dass der Herausgabemechanismus das Schubfach bis zu einem bestimmten Lagerbereich des Schubfachs heraus fährt, wobei beim Herausfahren die herausgefahrene Strecke regelmäßig erfasst wird und das Steuerungssystem den Herausgabemechanismus, neben den Zubehörsetparametern, auch mittels einer Regelungsschleife bezüglich der jeweils erfassten Strecke steuert.

Unter "eingerichtet" ist im Sinne der Erfindung zu verstehen, dass eine Vorrichtung nicht nur prinzipiell geeignet ist eine bestimmte Funktion zu erfüllen - etwa erst nachdem ein bestimmter Programmcode aufgespielt worden ist, also die Vorrichtung programmiert worden ist, oder die Vorrichtung in bestimmter Weise ausgeformt worden ist -, sondern die Vorrichtung bereits alle Mittel besitzt, um die Funktion tatsächlich zu erfüllen. Vorzugsweise ist dazu die Vorrichtung bereits mit einem Programmcode für diese Funktion programmiert und/oder bereits so geformt und/oder so angeordnet und/oder weist dazu bereits eine solche Konfiguration auf, dass die Vorrichtung die Funktion tatsächlich erfüllt.

Im Sinne der Erfindung ist unter "auf Basis" zumindest "bedingt durch" und/oder "in Abhängigkeit von" zu verstehen. Insbesondere kann das Bestimmen eines oder mehrerer zu bestimmender Parameter auf Basis einer Kennung, einer Eingangsgröße oder eines oder mehrerer vorhergehender Parameter oder eine Kombination daraus erfolgen. Dabei kann das Ergebnis, d.h. die bestimmten Parameter, von einem Teil der Basis, auf welcher das Bestimmen basiert, abhängen - insbesondere funktional, insbesondere linear oder polynomial -, während das Ergebnis von einem anderen Teil der Basis nicht abhängt. Auch kann die Abhängigkeit durch einem Teil der Basis bedingt werden, d.h. insbesondere, dass dieser Teil der Basis bedingt, von welchen Teilen der Basis das Ergebnis abhängt. Entsprechendes gilt für das Steuern oder Ansteuern, insbesondere auf Basis von Parametern, Kennungen oder Eingangsgrößen.

Unter einer "Datenverarbeitungsvorrichtung" im Sinne der Erfindung ist zumindest eine Vorrichtung zu verstehen, welche eingerichtet ist, Daten zu verarbeiten, d.h. insbesondere Daten zu empfangen, empfangene Daten zu speichern, gespeicherte Daten auszulesen, empfangene und/oder gespeicherte bzw. ausgelesene Daten mittels logischer und/oder mathematischer Operationen zu transformieren, transformierte Daten zu speichern und/oder transformierte bzw. ausgelesene Daten auszugeben. Vorzugsweise ist eine solche Datenverarbeitungsvorrichtung programmierbar, d.h. insbesondere dass die Verfahren zum Verarbeiten der Daten zumindest teilweise durch einen Programmcode bestimmt werden und dieser Programmcode zumindest teilweise veränderbar ist.

Vorzugsweise ist die Datenverarbeitungsvorrichtung ein handelsüblicher Computer. Weiter bevorzugt weist die Datenverarbeitungsvorrichtung wenigstens einen Datenprozessor - also eine Recheneinheit -, insbesondere einen Mikroprozessor, einen nicht-flüchtigen - d.h. insbesondere dauerhaften - Datenspeicher, insbesondere eine Festplatte, einen Read-Only-Memory (ROM) oder ein Laufwerk mit Datenträger, sowie wenigstens eine Hardwareschnittstelle auf. Auch vorzugsweise weist die Datenverarbeitungsvorrichtung einen flüchtigen elektrischen Datenspeicher, insbesondere als Arbeitsspeicher, auf, vorzugsweise einen Halbleiterspeicher, insbesondere mit integrierten Kondensatoren und/oder Flip-Flops (bistabile Multivibratoren) zur Datenspeicherung, etwa Dynamisches RAM oder statisches RAM.

Im Sinne der Erfindung ist eine "Datenspeichervorrichtung" eine Vorrichtung zum Speichern von Daten. Diese ist insbesondere zur Ausbildung einer Datenverbindung mit einer weiteren Vorrichtung, insbesondere einer Datenverarbeitungsvorrichtung, eingerichtet und/oder weist eine Datenverbindung mit der weiteren Vorrichtung auf, wobei mittels der Datenverbindung Daten von der weiteren Vorrichtung zur Datenspeichervorrichtung zum Speichern übertragen werden können und/oder zum Abrufen von gespeicherten Daten diese von der Datenspeichervorrichtung zur weiteren Vorrichtung übertragen werden können. Vorzugsweise weist die Datenspeichervorrichtung wenigstens einen nicht-flüchtigen Datenspeicher auf. Auch vorzugsweise weist die Datenspeichervorrichtung wenigstens einen flüchtigen elektrischen Datenspeicher auf.

Eine Kommunikationseinrichtung ist vorzugsweise eingerichtet für das Senden und/oder Empfangen von Daten, insbesondere für den Datenaustausch über eine durch die Kommunikationseinrichtung bereitgestellte Datenverbindung, insbesondere für eine Datenfernverbindung mit einem entfernten Gerät. Insbesondere wird das bezüglich eines Behandlungswagens entfernt angeordnete Gerät auch bezeichnet als "Remote-Gerät" oder externes Gerät, etwa externe Benutzerschnittstelle. Insbesondere wird eine Vorrichtung, die nicht Bestandteil eines Steuerungssystems ist, auch als externe Vorrichtung bezeichnet, etwa externer Behandlungswagen. Die Datenverbindung, insbesondere Datenfernverbindung, kann über ein beschränktes (insbesondere ein Intranet) oder weltweites Netzwerk aus Computern (insbesondere ein WAN und/oder das Internet) aufgebaut werden. Die Datenverbindung, insbesondere Datenfernverbindung, kann auch über eine drahtlose Verbindung, insbesondere Funkverbindung aufgebaut werden. Die Datenverbindung, insbesondere Datenfernverbindung, kann insbesondere über eine Mobilfunkverbindung aufgebaut werden.

Eine Datenverbindung verbindet insbesondere zwei datenverarbeitende Einheiten, insbesondere zwei Datenverarbeitungseinrichtungen oder -vorrichtungen, in der Weise, dass Daten zwischen den Einheiten ausgetauscht werden können, entweder unidirektional oder bidirektional. Die Datenverbindung kann leitungsgebunden realisiert sein oder drahtlos, insbesondere als Funkverbindung. Eine Datenfernverbindung verbindet insbesondere zwei datenverarbeitende Einheiten, insbesondere zwei Datenverarbeitungseinrichtungen, die entfernt voneinander angeordnet sind, die also insbesondere nicht Bestandteil desselben Geräts, insbesondere desselben Behandlungswagens, Benutzerschnittstelleneinrichtung oder Steuerungssystems sind, falls die genannten Geräte als separate Geräte ausgeführt sind. Eine Datenverbindung, insbesondere eine Datenfernverbindung, eines Geräts mit einem anderen Gerät wird vorzugsweise über eine direkte Verbindung der beiden Geräte realisiert, oder eine mittelbare Verbindung der beiden Geräte, so dass ein drittes Gerät zwischen die beiden Geräte geschaltet ist, um die Daten weiterzuvermitteln. Eine Datenfernverbindung kann insbesondere über ein Netzwerk aus Computern realisiert sein, bei denen die über die Datenfernverbindung verbundenen Geräte über das Netzwerk verbunden sind. Das Netzwerk kann ein beschränktes Netzwerk sein, z.B. ein Intranet, oder kann ein weltweites Netzwerk sein, insbesondere ein WAN und/oder das Internet.

Im Sinne der Erfindung dient eine "Schnittstelleneinrichtung" der Verbindung von zwei Einheiten - insbesondere auch von Systemen, Vorrichtungen, Einrichtungen oder Mechanismen, insbesondere mit solchen Einheiten -, die jeweils Signale, insbesondere Informationen, insbesondere Daten, verarbeiten, also insbesondere senden und/oder empfangen, können. Eine Schnittstelleneinrichtung kann wenigstens eine Hardwareschnittstelle und/oder wenigstens eine aufweisen und insbesondere als Bestandteil in einer physischen Geräteeinheit integrieren.

Im Sinne der Erfindung sind "Hardwareschnittstelleneinrichtungen" - oder kurz "Hardwareschnittstellen" - insbesondere Schnittstellen zwischen elektrisch, vorzugsweise elektronisch, arbeitenden Einheiten, gemäß dem üblichen Verständnis in der Elektrotechnik und Elektronik. Vorliegend bezeichnet der Begriff "Hardwareschnittstelle" insbesondere auch die Verbindungskomponenten zwischen wenigstens zwei elektrisch arbeitenden Einheiten selbst, also insbesondere alle Bestandteile, die diese Verbindung ermöglichen, vorzugsweise etwa integrierte Schaltkreise, Elektronik und Leitungen, über die zwischen den wenigstens zwei elektrisch arbeitenden Einheiten elektrische Signale versandt werden. Diese zwei elektrisch arbeitenden Einheiten können insbesondere ein Steuerungssystem und ein Herausgabemechanismus sein oder zwei elektrisch arbeitende Einheiten innerhalb eines Behandlungswagens. Eine Hardwareschnittstelle muss nicht, aber kann eine lösbare Verbindungseinrichtung zum Lösen und/oder Wiederherstellen dieser Verbindung aufweisen, insbesondere mindestens einen Stecker. Entsprechend und zusätzlich oder alternativ können die mittels einer Hardwareschnittstelle verbundenen Einheiten auch ganz oder teilweise optisch arbeiten und/oder die Signale zwischen den beiden Einheiten optisch versandt werden.

Im Sinne der Erfindung sind "Softwareschnittstellen" insbesondere logische Berührungspunkte in einem System, das ganz oder zumindest teilweise durch Softwarefunktionen implementiert ist, insbesondere einem Softwaresystem, insbesondere einem Informationsverwaltungssystem. Sie ermöglichen und steuern, insbesondere regeln, den Austausch von Kommandos und Daten zwischen verschiedenen Prozessen oder Komponenten des Systems und/oder mit einem weiteren System. Softwareschnittstellen können insbesondere nur zur Kommunikation benutzte, datenorientierte Schnittstellen sein. In diesem Fall enthält die Softwareschnittstelle lediglich die Informationen - also insbesondere die Daten -, die zwischen den beteiligten Bestandteilen des Systems und/oder mit einem Bestandteil des weiteren Systems ausgetauscht werden.

Im Sinne der Erfindung ist eine "Benutzerschnittstelleneinrichtung" - oder kurz "Benutzerschnittstelle" - eine Einrichtung, welche zur Kommunikation zwischen einem Benutzer und einer Einheit - insbesondere auch einem System, einer Vorrichtung, einer Einrichtung oder eine Mechanismus, insbesondere mit einer solchen Einheit - dient. Insbesondere kann dabei die Einheit Signale, insbesondere Informationen, insbesondere Daten, elektrisch oder optisch verarbeiten, also insbesondere senden und/oder empfangen. Die Benutzerschnittstelle weist ein erstes Kommunikationsmittel, insbesondere eine Hardwareschnittstelle, zur Kommunikation mit der Einheit und ein zweites Kommunikationsmittel zur Kommunikation mit dem Benutzer auf. Vorzugsweise ist das erste Kommunikationsmittel zur Ausbildung einer Datenverbindung mit der Einheit eingerichtet und/oder weist eine Datenverbindung mit der Einheit auf.

Insbesondere kann die Kommunikation von der Einheit zu dem Benutzer erfolgen, also Daten von der Einheit über das erste Kommunikationsmittel zur Benutzerschnittstelle übertragen und dort dem Benutzer mittels des zweiten Kommunikationsmittels signalisiert, d.h. insbesondere Informationen, welche durch die Daten codiert werden, ausgegeben werden. Vorzugsweise gibt eine derartige Benutzerschnittstelle die Informationen optisch, akustisch und/oder haptisch aus. Derartige Benutzerschnittstellen sind insbesondere eine Anzeigetafel, ein Bildschirm, eine Signallampe, ein Lautsprecher, ein Vibrationsalarm oder ein Brailledisplay.

Zusätzlich oder alternativ kann die Kommunikation insbesondere auch in umgekehrter Richtung erfolgen, also Daten von dem Benutzer, insbesondere eine oder mehrere Eingaben von dem Benutzer, mittels des zweiten Kommunikationsmittels erfasst und über das erste Kommunikationsmittel zur Einheit übertragen werden. Vorzugsweise erfassen derartige Benutzerschnittstellen die Daten mittels einer auf der Messung von Strahlung, insbesondere elektromagnetischer Strahlung wie Licht oder Radiowellen, von Schall, insbesondere Ultraschall oder Schall im hörbaren Bereich, von elektrischen Kontaktierungen und/oder von Kraft bzw. Druck basierenden Sensorik und/oder sind zur Betätigung durch den Benutzer eingerichtet. Derartige Benutzerschnittstellen sind insbesondere eine Kamera - vorzugsweise zur Gestensteuerung -, ein Infrarotsensor, ein Mikrofon - insbesondere für den hörbaren Bereich, vorzugsweise zur Sprachsteuerung, oder für Ultraschall und zusammen mit einem korrespondierenden Ultraschallsender, vorzugsweise zur Gestensteuerung -, ein Lesegerät - vorzugsweise ein optischer Scanner etwa für Barcodes oder Dokumente, ein RFID-Lesegerät, ein Magnetstreifen-Lesegerät oder ein Chip-Reader -, ein Schalter, ein Steuerpedal, eine Tastatur, eine Computer-Maus oder eine berührungsempfindliche Eingabefläche.

Weiter bevorzugt kann die Kommunikation in beide Richtungen erfolgen, wobei die Benutzerschnittstelle dazu insbesondere wenigstens ein weiteres erstes Kommunikationsmittel und/oder wenigstens ein weiteres zweites Kommunikationsmittel aufweist. Insbesondere kann dabei das zweite Kommunikationsmittel zur Ausgabe von Daten eingerichtet sein und das weitere zweite Kommunikationsmittel zum Erfassen von Daten, d.h. insbesondere für Eingaben von dem Benutzer, eingerichtet sein. Eine derartige Benutzerschnittstelle ist insbesondere ein berührungsempfindlicher Bildschirm.

Weitere Vorteile, Merkmale und Anwendungsmöglichkeiten der vorliegenden Erfindung ergeben sich aus der nachfolgenden detaillierten Beschreibung wenigstens eines Ausführungsbeispiels und/oder aus den Figuren. Gleiche Bauteile der Ausführungsformen werden im Wesentlichen durch gleiche Bezugszeichen gekennzeichnet, falls dies nicht anders beschrieben wird oder sich nicht anders aus dem Kontext ergibt.

Dabei zeigen, teilweise schematisiert:
Figur 1 ein Ausführungsbeispiel des erfindungsgemäßen mobilen Auswahlsystems;
Figur 2 einen Behandlungswagen gemäß einer nicht beanspruchten Ausführungsform; und
Figur 3 ein Verfahren zur Auswahl von medizinischen Zubehören gemäß einer nicht beanspruchten Ausführungsform.

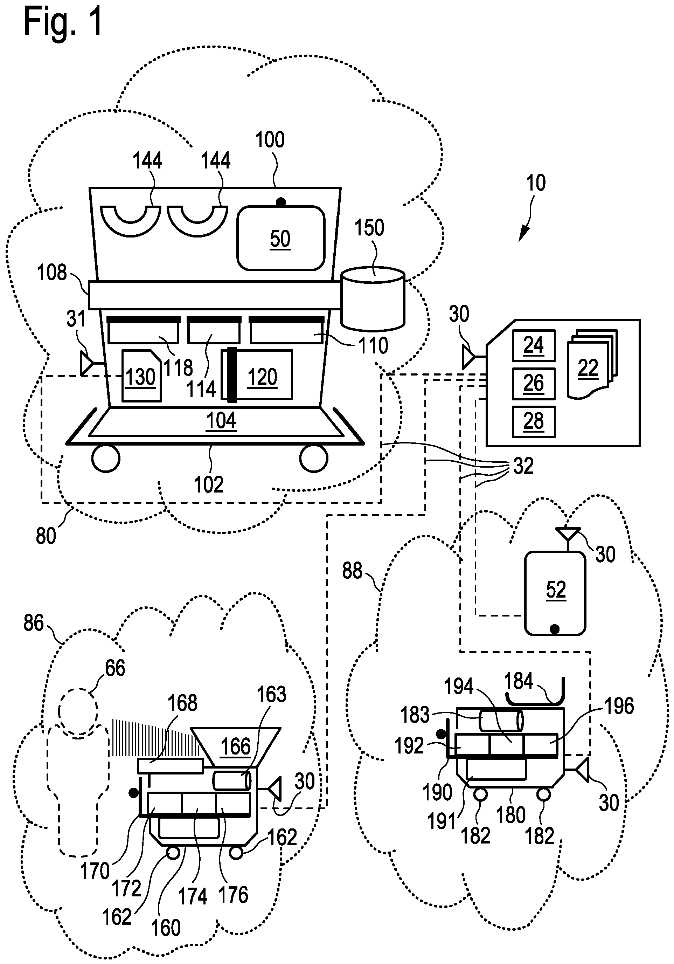

In Figur 1 ist ein Ausführungsbeispiel des erfindungsgemäßen mobilen Auswahlsystems 10 dargestellt. Das mobile Auswahlsystem weist ein Steuerungssystem 20, drei Behandlungswagen 100, 160, 180 und zwei Benutzerschnittstellen 50, 52 auf. Das mobile Auswahlsystem 10, insbesondere das Steuerungssystem 20, weist je eine Datenverbindung 32 für die Behandlungswagen 100, 160, 180 und die Benutzerschnittstelle 52 auf oder ist zumindest zur Ausbildung davon eingerichtet. Dabei weist das mobile Auswahlsystem entsprechende Kommunikationseinrichtungen 30, 31 für die Datenverbindungen 32 auf, wobei insbesondere die Kommunikationseinrichtungen 30 je ein Bestandteil des Steuerungssystems 20 oder eines der Behandlungswagen 160, 180 oder der Benutzerschnittstelle 52 sind und/oder die Kommunikationseinrichtung 31 ein Bestandteil des Behandlungswagens 100 ist. Die Kommunikationseinrichtungen 30, 31 sind vorzugsweise als Netzwerkschnittstelleneinrichtungen ausgebildet, insbesondere für ein drahtloses Netzwerk, - etwa vorzugsweise mit einem so genannten Ethernet-Adapter bzw. WiFi-Adapter -, und zur Ausbildung der Datenverbindungen 32 über ein Netzwerk eingerichtet, insbesondere über ein lokales Netzwerk (LAN bzw. WLAN), ein Metropolitan Area Network (MAN) und/oder ein Weitverkehrsnetz (WAN) und/oder vorzugsweise über das Internet und/oder weiter bevorzugt über ein durch ein anderes Netzwerk, insbesondere das Internet, getunneltes - und damit insbesondere abgesichertes - virtuelles privates Netzwerk (VPN).

Das Steuerungssystem 20 weist eine Zubehördatenbank 22, welche für Datensätze über eine Mehrzahl an medizinischen Zubehören eingerichtet ist, eine Datenspeichervorrichtung 24, insbesondere für diese Zubehördatenbank 22, wobei die Zubehördatenbank 22 auf der Datenspeichervorrichtung 24 gespeichert ist, eine Datenverarbeitungsvorrichtung 26 und einen Arbeitsspeicher 28 auf und ist eingerichtet, die Zubehördatenbank 22 zu betreiben. Vorzugsweise ist die Zubehördatenbank 22 als relationale Datenbank ausgebildet, insbesondere als SQL-Datenbank, welche mittels einer SQL-Query abgefragt werden kann. Vorzugsweise ist die Datenspeichervorrichtung 24 für die Zubehördatenbank 22 als nichtflüchtiger Datenspeicher, insbesondere als Magnet-Festplatte (HDD) oder als Solid-State-Disk (SSD) oder als ein Array daraus (RAID), ausgebildet. Vorzugsweise ist die Datenverarbeitungsvorrichtung 26 als Mikroprozessor ausgebildet oder weist zumindest einen solchen auf. Vorzugsweise ist der Arbeitsspeicher 28 als flüchtiger Datenspeicher, insbesondere flüchtige elektrischer Datenspeicher, insbesondere Dynamisches RAM (DRAM), insbesondere Double Data Rate Synchronous Dynamic Random Access Memory (DDR-SDRAM), ausgebildet. Insbesondere kann die Datenverarbeitungsvorrichtung 26 mit dem Mikroprozessor, dem Arbeitsspeicher 28 und der Datenspeichervorrichtung 24 als handelsüblicher Computer ausgebildet sein, auf welchem die Zubehördatenbank 22 gespeichert ist und welcher eingerichtet zum Betreiben der Zubehördatenbank ist; also insbesondere auf dem Computer, insbesondere der Datenspeichervorrichtung 24, die Datenbasis und der Programmcode der Zubehördatenbank 22 gespeichert sind und der Computer eingerichtet ist, den Programmcode in den Arbeitsspeicher zu laden und auszuführen sowie, insbesondere bei Datenbankabfragen, auf die Datenbasis in mittels der Ausführung des Programmcodes vorbestimmter Weise zuzugreifen.

Die Behandlungswagen sind an drei geographisch verschiedenen Behandlungsorten 80, 86, 88 angeordnet, wobei es sich versteht, dass die Behandlungswagen auch zu anderen Behandlungsorten transportiert, insbesondere gefahren werden können.

Insbesondere können die Behandlungsorte 86 und 88 innerhalb eines Klinikums sein und/oder der Behandlungsort 80 der Wohnort eines Patienten sein. Ein Vorteil der Anordnung des Behandlungswagens 100 bei dem Wohnort 80 des Patienten kann insbesondere darin liegen, dass dem Patienten medizinische Zubehöre für eine durchzuführende Behandlung bei sich zuhause herausgegeben werden können. Dies kann besonders vorteilhaft bei der Behandlung von chronischen Erkrankungen sein, womit häufige und/oder regelmäßige Aufenthalte in einem Klinikum vermieden oder zumindest reduziert werden können. So lässt sich insbesondere eine Dialysebehandlung eines Dialysepatienten als Heimdialyse durchführen und/oder eine Unterstützung des Patienten bei der Heimdialyse durch Lagerung sowie Herausgabe der dazu erforderlichen medizinischen Zubehöre erzielen. Ein Vorteil der Anordnung des Behandlungswagens 160 bei dem Behandlungsort 86 bzw. des Behandlungswagens 180 bei dem Behandlungsort 88 kann insbesondere darin liegen, dass medizinische Zubehöre an den jeweiligen Behandlungsorten, etwa bei verschiedenen Bereichen oder Zimmern in einer oder in mehreren Stationen eines Klinikums, herausgegeben werden können und bedarfsabhängig die Behandlungswagen 160, 180 zu verschiedenen Behandlungsorten gefahren werden können. Ferner können das Steuerungssystem 20 oder Bestandteile davon, insbesondere die Zubehördatenbank 22, die Datenspeichervorrichtung 24, die Datenverarbeitungsvorrichtung 26 und/oder der Arbeitsspeicher 28, an einem weiteren, anderen geographischen Ort als den Behandlungsorten 80, 86, 88 angeordnet sein.

Die Benutzerschnittstelle 50 ist mit dem Behandlungswagen 100 unlösbar, vorzugsweise integral, verbunden. Die Benutzerschnittstelle 52 ist, wie exemplarisch illustriert in Figur 1, beim Behandlungsort 88 angeordnet und mit keinem Behandlungswagen physisch verbunden. Vorzugsweise ist die Benutzerschnittstelle 52 als Behandlungswagenmodul ausgebildet und kann mittels einer Verbindungsvorrichtung für diese mit einem der Behandlungswagen, insbesondere mittels einer Verbindungsvorrichtung 184 des Behandlungswagens 180 mit diesem, lösbar verbunden werden.

Zur Bedienung der Behandlungswagen 100, 160, 180 sind die Benutzerschnittstellen 50, 52 jeweils einem Behandlungswagen zugeordnet bzw. diesem zuordenbar. Dazu weist das Steuerungssystem 20 vorzugsweise eine Zuordnungsvorschrift für Benutzerschnittstellen und eine Datenspeichervorrichtung für diese auf, wobei diese Datenspeichervorrichtung insbesondere die Datenspeichervorrichtung 24 und/oder der Arbeitsspeicher 28 ist. Diese Zuordnungsvorschrift ist eingerichtet, anhand von Zuordnungsdaten, welche auf dieser Datenspeichervorrichtung gespeichert sind, eine Benutzerschnittstelle 50, 52, welche mittels einer Benutzerschnittstellenkennung gekennzeichnet ist, einen Behandlungswagen 100,160, 180, welcher mittels einer Behandlungswagenkennung gekennzeichnet ist, zuzuordnen. Vorzugsweise kann die jeweilige Zuordnung durch eine Eingabe eines Benutzers erfolgen. So kann diesem vorzugsweise eine Auflistung von im System vorhandenen Behandlungswagen 100, 160, 180 angezeigt werden, worauf dieser einen Behandlungswagen in dieser Auflistung auswählen kann, etwa durch Antippen bei einer als berührungsempfindlicher Bildschirm ausgebildeten Benutzerschnittstelle, und in Folge dieser Auswahl das Steuerungssystem 20 die Zuordnungsdaten entsprechend anpasst und der Benutzerschnittstelle 52 den ausgewählten Behandlungswagen zugeordnet. Zusätzlich oder alternativ kann eine Benutzerschnittstelle einem Behandlungswagen dadurch zugeordnet sein bzw. werden, dass diese mit dem Behandlungswagen, insbesondere physisch, verbunden wird. So ist vorzugsweise die Benutzerschnittstelle 50 aufgrund ihrer unlösbaren Verbindung mit dem Behandlungswagen 100 zugeordnet, wobei diese Zuordnung in der Zuordnungsvorschrift nicht änderbar ist bzw. unabhängig von der Zuordnungsvorschrift besteht oder alternativ auch diese Zuordnung durch eine entsprechende Benutzereingabe überschrieben werden kann.

Die Benutzerschnittstellen 50, 52 sind für eine oder mehrere Eingaben von einem Benutzer eingerichtet, durch die eine durchzuführende Behandlung festgelegt wird und auf Basis derer das Steuerungssystem 20 infolge dieser Eingaben eine der durchzuführenden Behandlung zugeordnete Behandlungskennung bestimmt. Zur Eingabe und für Ausgaben weisen die Benutzerschnittstellen 50, 52 vorzugsweise einen berührungsempfindlichen Bildschirm auf. Vorzugsweise ist die Benutzerschnittstelle 50 als ein solcher berührungsempfindlicher Bildschirm ausgebildet und ist im Behandlungswagen 100 intern mit weiteren Bestandteilen des Behandlungswagens 100 und/oder des Steuerungssystems 20 datenverbunden. Der Übersichtlichkeit halber sind solche internen Verbindungen in Figur 1 nicht dargestellt. Vorzugsweise ist die Benutzerschnittstelle 52 als mobiles Endgerät, insbesondere als Smartphone oder Tablet-PC, ausgebildet und weist eine der Kommunikationseinrichtungen 30 auf. Alternativ oder zusätzlich können die Benutzerschnittstellen 50, 52 auch weitere Eingabe- oder Ausgabegeräte - wie Drehregler, Tastschalter, eine Tastatur, eine Computermaus und/oder ein Mikrofon bzw. einen Lautsprecher, elektrische Signallampen und/oder ein LCD-Display - aufweisen oder daraus bestehen.

Die Benutzerschnittstellen 50, 52 sind eingerichtet, die Eingaben, insbesondere die Eingaben zur Bestimmung der Behandlungskennung der durchzuführenden Behandlung, an das Steuerungssystem 20 über eine der Datenverbindungen 32 zu übertragen. Vorzugsweise ist dazu die Benutzerschnittstelle 50 intern mit weiteren Bestandteilen des Behandlungswagens 100 und insbesondere mit der Kommunikationseinrichtung 31 mittelbar oder unmittelbar, insbesondere mittels Signalleitungen, datenverbunden. Die Benutzerschnittstelle 52 weist dazu vorzugsweise eine der Kommunikationseinrichtungen 30 auf und ist somit unabhängig von den Behandlungswagen. Auf diese vorteilhafte Weise kann die Benutzerschnittstelle 52, auch während diese für Eingaben eingerichtet ist bzw. während Eingaben erfolgen, an einem geographisch anderen Ort als dem des zugeordneten Behandlungswagens bzw. unabhängig von den Orten der Behandlungswagen positioniert werden. Insbesondere kann die Benutzerschnittstelle 52 also dem am Behandlungsort 86 befindlichen Behandlungswagen 160 zugeordnet werden und es können an der Benutzerschnittstelle 52 Eingaben für diesen auch erfolgen, während sich die Benutzerschnittstelle 52 bei dem Behandlungsort 88 befindet.

Vorzugsweise ist wenigstens eine der Benutzerschnittstellen 50, 52, insbesondere die Benutzerschnittstelle 52, eingerichtet, eine oder mehrere Eingaben von einem Benutzer zu erfassen, durch die ein zu behandelnder Patient festgelegt wird und auf Basis derer das Steuerungssystem infolge dieser Eingaben eine dem zu behandelnden Patienten zugeordnete Patientenkennung bestimmt. Dazu kann die Benutzerschnittstelle 50, 52 insbesondere eingerichtet sein, eines oder mehrerer Eingabefelder für Merkmale, die den zu behandelnden Patienten kennzeichnen - etwa Name oder Patientennummer -, auf dem berührungsempfindlichen Bildschirm auszugeben und Eingaben bezüglich dieser Eingabefelder zu erfassen und an das Steuerungssystem 20 zu senden. Auch kann die Benutzerschnittstelle 50, 52 dazu insbesondere ein Lesegerät, vorzugsweise etwa ein Versicherungskarten-Lesegerät, einen Barcodescanner oder einen Dokumentenscanner, mit welchem Merkmale, die den zu behandelnden Patienten kennzeichnen, eingelesen werden können, aufweisen.

Der Behandlungswagen 180 weist eine Lagereinrichtung 190 mit drei Lagerbereichen 192, 194, 196 je für eine Zubehörgattung aus der Mehrzahl an medizinischen Zubehören auf. Insbesondere ist der Behandlungswagen 180 in Figur 1 als seitlicher Schnitt durch den Behandlungswagen schematisch dargestellt. Insbesondere können dabei, wenn der Behandlungswagen 180 für eine Dialysebehandlung, insbesondere für die dabei durchzuführende Kanülierung - etwa einer AV-Fistel -, bestückt ist, der Lagerbereich 192 eine oder mehrere Kanülen, der Lagerbereich 194 einen oder mehrere Seldinger-Drähte und der Lagerbereich 196 einen oder mehrere Katheter aufweisen. Zudem weist der Behandlungswagen 180 einen Herausgabemechanismus 191 zur Herausgabe von medizinischen Zubehören, insbesondere für die Lagereinrichtung 190, auf. Ferner weist der Behandlungswagen 180 wenigstens zwei, vorzugsweise vier, Räder 182 auf. Vorzugsweise weist der Behandlungswagen 180 außerdem eine der Kommunikationseinrichtungen 30 auf oder ist mit dieser zumindest physisch verbunden. Daneben kann der Behandlungswagen 180, wie oben beschrieben, eine Verbindungsvorrichtung 184 für eine Benutzerschnittstelle aufweisen. Vorzugsweise ist die Verbindungsvorrichtung 184 eingerichtet, eine Benutzerschnittstelle, insbesondere die Benutzerschnittstelle 52, zumindest teilweise aufzunehmen und den Behandlungswagen 180 mit der Benutzerschnittstelle formschlüssig und lösbar zu verbinden. Vorzugsweise ist die Lagereinrichtung 190, wie in Figur 1 illustriert, als Schubfach ausgebildet oder weist zumindest ein solches auf. Auch vorzugsweise ist der Herausgabemechanismus 191 für die Lagereinrichtung 190 als motorisierter Seilzug oder als motorbetriebene Spindel ausgebildet und eingerichtet, das Schubfach 190 gemäß der Steuerung des Steuerungssystems 20 heraus und/oder wieder hereinzufahren.

Für den Behandlungswagen 180 ist das Steuerungssystem 20 eingerichtet, ein Auswahlverfahren mit den folgenden Verfahrensschritten auszuführen. In einem Verfahrensschritt wird die Behandlungskennung der durchzuführenden Behandlung auf Basis der Eingaben an der Benutzerschnittstelle 52, sofern diese dem Behandlungswagen 160 zugeordnet ist, bestimmt. In einem weiteren Verfahrensschritt wird ein Zubehörsetparameter anhand der Zubehördatenbank 22 und auf Basis der Behandlungskennung der durchzuführenden Behandlung bestimmt, wobei der Zubehörsetparameter ein medizinisches Zubehörset aus einem oder mehreren für die durchzuführende Behandlung geeigneten medizinischen Zubehören kennzeichnet. Insbesondere kennzeichnet der Zubehörsetparameter die Positionen der Lagerbereiche 192, 194, 196, welche mit den jeweils geeigneten medizinischen Zubehören bestückt sind - d.h. insbesondere welche für die jeweiligen Zubehörgattungen sind -, und vorzugsweise bei dem Schubfach die Strecke, bis zu welcher das Schubfach heraus gefahren werden muss, um den jeweiligen Lagerbereich zugänglich zu machen, d.h. insbesondere die jeweils dort gelagerten medizinischen Zubehöre herauszugeben. Zudem ist das Steuerungssystem 20 eingerichtet, den Herausgabemechanismus 191 auf Basis der Zubehörsetparameter so zu steuern, dass der Herausgabemechanismus 191 jene medizinische Zubehöre herausgibt, welche durch den Zubehörsetparameter gekennzeichnet sind. Vorzugsweise steuert das Steuerungssystem 20 den Herausgabemechanismus 191 für das Schubfach 190 so, dass dieser das Schubfach jeweils die durch den Zubehörsetparameter gekennzeichnete Strecke heraus fährt und somit den jeweiligen Lagerbereich für das jeweilige medizinische Zubehör des Zubehörsets zugänglich macht.

Zur Versorgung mit elektrischer Energie weist der Behandlungswagen 180 vorzugsweise einen elektrischen Energiespeicher 183, insbesondere eine oder mehrere wiederaufladbare Batterien, auf. Bevorzugt und zusätzlich oder alternativ kann der Behandlungswagen 180 auch eine Anschlusseinrichtung für das Stromnetz, insbesondere einen Stecker mit Kabel oder Kabelrolle, aufweisen.

Entsprechend weist der Behandlungswagen 160 eine Lagereinrichtung 170 mit drei Lagerbereichen 172, 174, 176, einen Herausgabemechanismus 171, Räder 162 und vorzugsweise eine der Kommunikationseinrichtungen 30 auf. Insbesondere ist der Behandlungswagen 160 in Figur 1 als seitlicher Schnitt durch den Behandlungswagen schematisch dargestellt. Insbesondere können dabei, wenn der Behandlungswagen 160 für eine Dialysebehandlung, insbesondere für die Vorbereitung und Durchführung einer Kanülierung, bestückt ist, die Lagerbereiche jeweils medizinische Zubehöre der folgenden Zubehörgattungen aufweisen: der Lagerbereich 172 Desinfektionstücher; der Lagerbereich 174 Pflaster bzw. Klebestreifen mit einem schmerzstillenden Wirkstoff; und der Lagerbereich 176 Zubehörsets für die Kanülierung je mit einer Kanüle, einem Seldinger-Draht und einem Katheter.

Zur Versorgung mit elektrischer Energie weist der Behandlungswagen 160 vorzugsweise einen elektrischen Energiespeicher 163, insbesondere eine oder mehrere wiederaufladbare Batterien, auf. Bevorzugt und zusätzlich oder alternativ kann der Behandlungswagen 160 auch eine Anschlusseinrichtung für das Stromnetz, insbesondere einen Stecker mit Kabel oder Kabelrolle, aufweisen.

Des Weiteren weist der Behandlungswagen 160 eine Ablageeinrichtung 168 mit einer desinfizierbaren Ablagefläche und ein Erkennungssystem 166 auf. Die Ablagefläche der Ablageeinrichtung 168 kann vor der Behandlung und/oder vor Teilbehandlungen der Behandlung und/oder vor einzelnen Behandlungsschritten der Behandlung desinfiziert werden. Dazu kann der Behandlungswagen 160 insbesondere eine, in Figur 1 nicht dargestellte, Desinfektionsvorrichtung aufweisen. Die Ablagefläche ist dafür vorgesehen, dass vor der Behandlung oder vor einzelnen Behandlungsschritten die jeweils erforderlichen medizinischen Zubehöre auf dieser abgelegt werden können und somit für die Behandlung bzw. dem jeweiligen Behandlungsschritt unmittelbar bereitstehen und/oder greifbar sind. Das Erkennungssystem 166 erfasst mittels einer oder vorzugsweise mehrerer Bildsensoren eine an der Behandlung beteiligte Person 66, welche sich am Behandlungsort 86 befindet, und/oder medizinisches Zubehör, welches von der Person 66 verwendet wird und/oder welches auf der Ablagefläche liegt, vorzugsweise dreidimensional.

In einer bevorzugten Variante ist das Erkennungssystem 166 eingerichtet, die Bildsensoren als Sensoreinrichtung für Identifizierungsdaten zu nutzen und mittels dieser Identifizierungsdaten zu erfassen, welche eine biometrische Identifikation der Person 66 ermöglichen. Zudem ist das Steuerungssystem 20 vorzugsweise eingerichtet, ein Identifizierungsverfahren auszuführen und die Person 66 auf Basis der erfassten Identifizierungsdaten zu identifizieren oder andernfalls die Person 66 als nicht-identifizierte Person festzulegen. Außerdem ist das Steuerungssystem 20 eingerichtet in Abhängigkeit von der erfolgreichen Identifizierung die Person 66 als Benutzer am System anzumelden und eine Benutzerkennung zu bestimmen, wobei vorzugsweise angemeldeten Benutzern bestimmte Autorisierungen zugeordnet sind oder, insbesondere eine nicht-identifizierte Person bzw. ein nicht-angemeldeter Benutzer keine Autorisierung hat, also ein nicht-autorisierter Benutzer ist.

In einer bevorzugten Variante ist das Erkennungssystem 166 eingerichtet, auf Basis der erfassten Verwendung des medizinischen Zubehörs und/oder der auf der Ablagefläche liegenden bzw. von dieser genommenen medizinischen Zubehöre sowie vorzugsweise auf Basis der durchzuführenden Behandlung, sofern diese festgelegt worden ist, den jeweils durchgeführten Behandlungsschritt zu bestimmen.

Entsprechend der vorhergehenden Beschreibung bezüglich des Steuerungssystems 20 für den Behandlungswagen 180 ist das Steuerungssystem 20 für den Behandlungswagen 160 eingerichtet, ein entsprechendes Auswahlverfahren auszuführen und den Herausgabemechanismus 161 zu steuern. Dabei werden im Auswahlverfahren vorzugsweise mehrere Zubehörsetparameter bestimmt, wobei einer davon der vorhergehenden Beschreibung entspricht und wenigstens ein weiterer davon die Lagereinrichtung 170 kennzeichnet, so dass mehrere Lagereinrichtungen adressiert werden können.

Zudem ist dabei das Steuerungssystem 20 vorzugsweise eingerichtet, das Auswahlverfahren nur bei angemeldeten Benutzern und gemäß ihrer Autorisierung auszuführen und nur solche medizinischen Zubehöre herauszugeben, für welche der jeweilige Benutzer autorisiert ist. Insbesondere weist dazu das Auswahlverfahren des Weiteren den Verfahrensschritt Überprüfen, ob der angemeldete Benutzer für die durch die Zubehörsetparameter gekennzeichneten medizinischen Zubehöre autorisiert ist, auf. Vorzugsweise sind für eine derartige Überprüfung in der Zubehördatenbank Datensätze gespeichert, welche einem medizinischen Zubehör einen oder mehrere Benutzer oder Benutzergruppen, welche für dieses medizinische Zubehör autorisiert sind, oder bestimmte Autorisierungen, welche für dieses medizinische Zubehör autorisieren, zuordnet. Insbesondere wird im Auswahlverfahren ein Zubehörsetparameter bestimmt, welcher kennzeichnet, ob der Benutzer für das Zubehörset und/oder für welche der medizinischen Zubehöre des Zubehörsets autorisiert ist.

Auch ist das Steuerungssystem 20 vorzugsweise eingerichtet, ein Assistenzverfahren, insbesondere als Teil des Auswahlverfahrens, auszuführen. In einem Verfahrensschritt des Assistenzverfahren wird mittels des Erkennungssystems 166 ein Assistenzparameter bestimmt, welcher den durchzuführenden Behandlungsschritt der durchzuführenden Behandlung kennzeichnet, und ein Assistenzparameter bestimmt, welcher die für den jeweiligen Behandlungsschritt benötigten medizinischen Zubehöre kennzeichnet, oder alternativ oder zusätzlich, insbesondere im Auswahlverfahren, einer oder mehrere Zubehörsetparameter bestimmt, welche den medizinischen Zubehören des Zubehörsets jeweils einen Behandlungsschritt zuordnen.

Schließlich ist das Steuerungssystem 20 eingerichtet, den Herausgabemechanismus 171 so zu steuern, dass dieser jene medizinische Zubehöre herausgibt, welche durch die Zubehörsetparameter gekennzeichnet sind, und vorzugsweise jeweils jenen Teil der medizinischen Zubehöre des Zubehörsets herausgibt, welche gemäß der Assistenzparameter und der Zubehörsetparameter für den jeweils durchzuführenden Behandlungsschritt benötigt werden und/oder für welche der Benutzer autorisiert ist.

In einer Variante ist das Steuerungssystem 20 eingerichtet, den Herausgabemechanismus 171 so zu steuern, dass dieser die medizinischen Zubehöre für eine Serie an Behandlungsschritten - und nicht nur für den jeweils durchzuführenden Behandlungsschritt - herausgibt. Auf diese vorteilhafte Weise wird ermöglicht, diese medizinischen Zubehöre vorab auf der desinfizierbaren Ablagefläche der Ablageeinrichtung 168 abzulegen, so dass während des Durchführens der Serie der Behandlungsschritte kein Zugriff auf die Lagerbereiche erforderlich ist.

Insbesondere kann ferner das Assistenzverfahren einen Verfahrensschritt Bestimmen eines Assistenzparameters, welcher kennzeichnet, ob die desinfizierbare Ablagefläche der Ablageeinrichtung 168 desinfiziert werden soll - vorzugsweise etwa vor Beginn der Behandlung, vor, nach oder während bestimmter Behandlungsschritte oder am Ende der Behandlung -, aufweisen und das Steuerungssystem 20 eingerichtet sein, die Desinfektionsvorrichtung für die Ablageeinrichtung 168 so auf Basis der Assistenzparameter zu steuern, dass diese die Ablagefläche der Ablageeinrichtung 168 desinfiziert, wenn diese gemäß der Assistenzparameter desinfiziert werden soll.

So kann insbesondere, wenn als durchzuführende Behandlung eine Dialysebehandlung eines Patienten mit AV-Fistel festgelegt worden ist, zunächst die Person 66 erfasst werden. Falls diese Person 66 der zu behandelnde Patient ist, steuert das Steuerungssystem 20 den Herausgabemechanismus 171 so an, dass die medizinischen Zubehöre aus den Lagerbereichen 172 und 174 ausgegeben werden, insbesondere also das Schubfach soweit heraus fährt, dass diese beiden Lagerbereiche 172 und 174 für den Benutzer 66, also den Patienten, zugänglich sind. Daraufhin kann der Patient aus dem Lagerbereich 172 ein Desinfektionstuch entnehmen und den Hautbereich bei seiner AV-Fistel desinfizieren. Anschließend kann der Patient aus dem Lagerbereich 174 ein Pflaster mit einem schmerzstillenden Wirkstoff entnehmen und auf den Hautbereich kleben. Für weitere Behandlungsschritte ist der Benutzer 66, also der Patient, zunächst nicht autorisiert. Ein Arzt, welcher hinzukommt, kann nun als an der Behandlung beteiligte Person 66 erfasst werden und für weitere Behandlungsschritte autorisiert sein. Daraufhin steuert das Steuerungssystem 20 vorzugsweise die Desinfektionsvorrichtung zur Desinfektion der Ablagefläche der Ablageeinrichtung 168 an. Anschließend steuert das Steuerungssystem 20 den Herausgabemechanismus 171 zur Herausgabe weiterer medizinischer Zubehöre an, insbesondere eines der Zubehörsets für die Kanülierung aus dem Lagerbereich 176; insbesondere wird der Herausgabemechanismus 171 also vom Steuerungssystem 20 so gesteuert, dass dieser das Schubfach soweit heraus fährt, dass der Lagerbereich 176 zugänglich ist. Der Arzt 66 kann nun das Zubehörset mit der Kanüle, dem Seldinger-Draht und dem Katheter aus dem Lagerbereich 176 entnehmen und auf der Ablagefläche der Ablageeinrichtung 168 ablegen, womit ihm diese medizinischen Zubehöre für die weiteren Behandlungsschritte, insbesondere für die Kanülierung, bereitstehen.

Der Behandlungswagen 100 weist mehrere Lagereinrichtungen 110, 114, 118, 120, wenigstens einen Herausgabemechanismus - in Figur 1 nicht dargestellt -, eine Ablageeinrichtung 108, eine Verbindungsvorrichtung 104 für eine von dem Behandlungswagen 100 abtrennbare Fahrvorrichtung 102, eine Ansteuerungsvorrichtung 130, zwei Verbindungsvorrichtungen 144 für Behandlungswagenmodule, eine Entsorgungsvorrichtung 150 und vorzugsweise die Kommunikationseinrichtung 31 und/oder vorzugsweise die Benutzerschnittstelle 50 auf. Insbesondere ist der Behandlungswagen 100 in Figur 1 als teil-perspektivische Ansicht von vorne-oben eines frontalen Teilschnitts durch den Behandlungswagen schematisch dargestellt. So ist insbesondere die Ansteuerungsvorrichtung 130 vorzugsweise in einem Gehäuse des Behandlungswagens 100 angeordnet und daher bei einer frontalen Ansicht üblicherweise nicht sichtbar.

Insbesondere entspricht der Behandlungswagen 100 sowie das Steuerungssystem 20 und/oder das Auswahlverfahren zumindest teilweise, insbesondere im Wesentlichen, der Beschreibung bezüglich der Behandlungswagen 160 und 180. Dabei weist der Behandlungswagen 100 jedoch selbst keine Räder für den Transport auf, sondern die Verbindungsvorrichtung 104 für die Fahrvorrichtung 102. Für den Transport kann der Behandlungswagen 100 mit der Fahrvorrichtung 102, welche insbesondere kein Bestandteil oder zumindest kein dauerhafter Bestandteil des Behandlungswagens 100 ist, lösbar verbunden werden. Diese Verbindung ist vorzugsweise formschlüssig und/oder kraftschlüssig, insbesondere als Einrastmechanismus, ausgebildet. Dabei weist die Verbindungsvorrichtung 104 vorzugsweise eine Vertiefung, insbesondere wenigstens drei Vertiefungen, und die Fahrvorrichtung 102 damit korrespondierende Haken auf, welche vorzugsweise je mit einem Federmechanismus versehen sind, so dass die Haken, wenn diese bei den Vertiefungen angeordnet sind, in diese einrasten. Alternativ oder zusätzlich können die Fahrvorrichtung 102 und die Verbindungsvorrichtung 104 auch mittels einer oder mehrerer Schraubverbindungen lösbar verbunden werden.

Ferner erfolgt das Steuern des wenigstens einen Herausgabemechanismus zumindest teilweise mittels der Ansteuerungsvorrichtung 130. Dazu ist das Steuerungssystem 20 vorzugsweise über die Kommunikationseinrichtung 31 mit der Ansteuerungsvorrichtung 130 datenverbunden und die Ansteuerungsvorrichtung 130 steuert zumindest teilweise auf Basis der Daten, welche diese über die Datenverbindung 32 mittels der Kommunikationseinrichtung 31 von dem Steuerungssystem 20 empfängt, den wenigstens einen Herausgabemechanismus an.

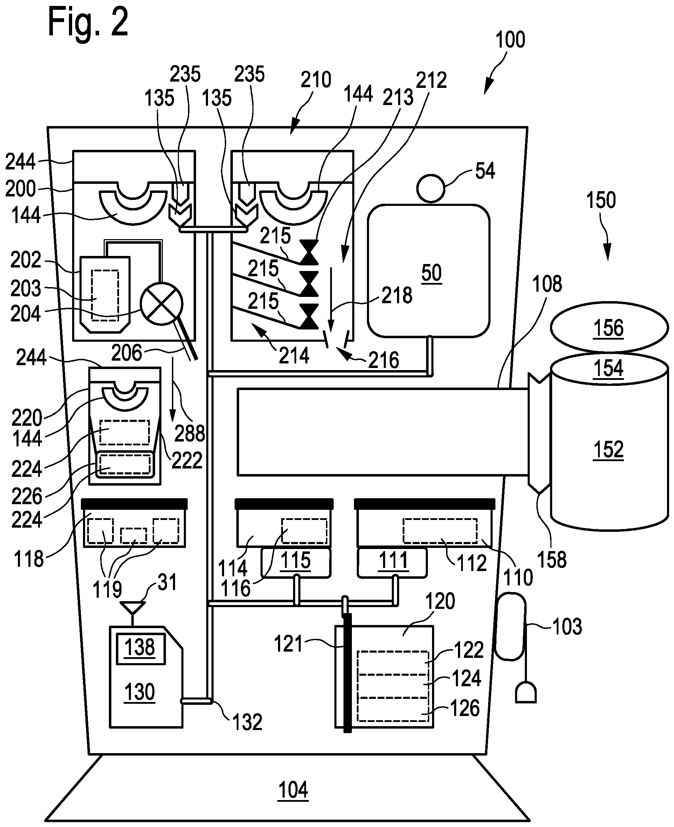

Figur 2 zeigt einen Behandlungswagen 100 gemäß einer nicht beanspruchten Ausführungsform, insbesondere zur Aufstellung beim Wohnort eines Patienten, insbesondere zur Heimdialyse. Insbesondere entspricht dieser dem Behandlungswagen 100 aus Figur 1. Der Behandlungswagen 100 lässt sich mittels einer Verbindungsvorrichtung 104 des Behandlungswagens 100 mit einer - in Figur 2 nicht dargestellten - Fahrvorrichtung verbinden und so zum Aufstellungsort, insbesondere zum Wohnort des Patienten, transportieren. Insbesondere ist der Behandlungswagen 100 in Figur 2 als teil-perspektivische Ansicht eines frontalen Teilschnitts durch den Behandlungswagen schematisch dargestellt. In Figur 2 sind hierbei insbesondere die Lagerbereiche und/oder medizinischen Zubehöre, welche sich innerhalb einer Lagereinrichtung oder eines Behandlungswagenmoduls befinden, durch Strichlinien schematisch angedeutet. Auch weist der Behandlungswagen 100 vorzugsweise eine Anschlusseinrichtung 103 für das Stromnetz, insbesondere einen Stecker, ein Stromkabel und eine Kabeltrommel für das Stromkabel, auf, um den Behandlungswagen 100 mit elektrischer Energie zu versorgen. Daneben kann eine Fahrvorrichtung für den Behandlungswagen einen elektrischen Energiespeicher aufweisen, so dass der Behandlungswagen auch während er mittels der Fahrvorrichtung gefahren wird - also insbesondere ein Stecker der Anschlusseinrichtung 103 nicht in einer Steckdose des Stromnetzes bleiben kann - mit elektrischer Energie versorgt werden kann.

Der Behandlungswagen 100 weist des Weiteren zwei Lagereinrichtungen 110 und 114, welche vorzugsweise je als Schubfach ausgebildet sind, mit jeweils genau einem Lagerbereich 112 bzw. 116 sowie einen Herausgabemechanismus 111 für die Lagereinrichtung 110 und einen Herausgabemechanismus 115 für die Lagereinrichtung 114 auf. Vorzugsweise weist der Herausgabemechanismus 111 einen motorisierten Seilzug, eine motorbetriebene Spindel oder einen Zahnstangenantrieb auf oder besteht daraus, wodurch dieser auf vorteilhafte Weise erlaubt, die Lagereinrichtung 110, insbesondere das Schubfach, voll-automatisiert zu öffnen und schließen, um medizinische Zubehöre, die im Lagerbereich 112 gelagert sind, herauszugeben und insbesondere anschließend das Schubfach wieder zu schließen. Vorzugsweise weist der Herausgabemechanismus 115 eine elektrisch steuerbare Magnetverriegelung und, insbesondere für das Schubfach, eine oder mehrere - speziell passive - Führungsschienen auf oder besteht daraus, wodurch dieser auf vorteilhafte Weise erlaubt, die Lagereinrichtung 114 elektrisch freizugeben, so dass diese, insbesondere manuell, geöffnet werden kann und so medizinische Zubehöre, die im Lagerbereich 116 gelagert sind, kontrolliert herauszugeben werden können. Vorzugsweise ist dabei der Lagerbereich 116 für Notfallmedikamente vorgesehen, auf welche der Patient erst nach Autorisierung durch einen Arzt zugreifen darf.

Zudem weist der Behandlungswagen 100 gemäß einer bevorzugten semiautomatisierten Variante eine Lagereinrichtung 118, welche vorzugsweise als Schubfach ausgebildet ist, mit einem oder mehreren Lagerbereichen 119 auf. Diese Lagereinrichtung 118 ist zur manuellen Betätigung ausgebildet und der Behandlungswagen 100 weist keinen Herausgabemechanismus für diese auf. Vielmehr können medizinische Zubehöre in den Lagerbereichen 119 der Lagereinrichtung 118 unabhängig von den Herausgabemechanismen, einem Auswahlverfahren, einer Steuerung und/oder einem mobilen Auswahlsystem entnommen und/oder dort gelagert werden. Die Lagerbereiche 119 bieten sich insbesondere für häufig verwendete und/oder nicht-sicherheitsrelevante und/oder für eine Vielzahl von Behandlungen verwendete medizinische Zubehöre an.

Überdies weist der Behandlungswagen 100 vorzugsweise eine Lagereinrichtung 120, welche als Schrankfach mit Schranktüre ausgebildet ist, mit, wie exemplarisch in Figur 2 illustriert, drei Lagerbereichen 122, 124 und 126 sowie einen Herausgabemechanismus 121 dafür auf. Vorzugsweise weist der Herausgabemechanismus 121 eine Signallampe - etwa eine LED - auf, wobei mittels der Signallampe signalisiert wird, wenn ein medizinisches Zubehör, für dessen Gattung die Lagereinrichtung 120 einen Lagerbereich 122,124 oder 126 aufweist, durch die Zubehörsetparameter gekennzeichnet ist. Bei der Variante mit Schrankfach und Schranktüre weist der Herausgabemechanismus 121 zudem eine Griffleiste auf, so dass die Schranktüre, insbesondere bei Signalisierung durch die Signallampe, mittels der Griffleiste geöffnet werden kann. Dies erlaubt es einen Benutzer, insbesondere den Patienten, bei der Auswahl und/oder dem Entnehmen der für die durchzuführende Behandlung erforderlichen medizinischen Zubehöre zu unterstützen, insbesondere ohne seinen Zugriff auf die in der Lagereinrichtung 120 gelagerten medizinischen Zubehöre einzuschränken. Zudem lässt sich bei einer als Schrankfach ausgebildeten Lagereinrichtung eine Vielzahl von medizinischen Zubehören, auch mit verschiedener Größe, - etwa auch Medizingeräte - lagern.

Ferner weist der Behandlungswagen 100 eine Benutzerschnittstelle 50 - insbesondere einen berührungsempfindlichen Bildschirm -, ein Kameramodul 54, eine Ablageeinrichtung 108 mit desinfizierbare Ablagefläche und eine oder mehrere Verbindungsvorrichtungen 144 für Behandlungswagenmodule auf, wobei wenigstens die Benutzerschnittstelle 50 und vorzugsweise weitere Bestandteile, insbesondere eine der Verbindungsvorrichtungen 144, mit dem Behandlungswagen 100 physisch, insbesondere integral, verbunden sind.

Der Behandlungswagen 100 weist eine Ansteuerungsvorrichtung 130 mit einer Kommunikationseinrichtung 31 auf und ist mittels dieser zur Ausbildung einer Datenverbindung mit einem Steuerungssystem eines mobilen Auswahlsystems eingerichtet oder weist eine solche Datenverbindung auf. Dabei steuert das Steuerungssystem die Herausgabemechanismen zumindest teilweise mittels der Ansteuerungsvorrichtung 130. Vorzugsweise empfängt die Ansteuerungsvorrichtung 130 die Zubehörsetparameter, welche das Steuerungssystem in einem Auswahlverfahren bestimmt, und setzt diese in eine, insbesondere für die jeweiligen Lagereinrichtungen und/oder Behandlungswagenmodul mit medizinischen Zubehören konkrete, Ansteuerung. Insbesondere kann die Ansteuerungsvorrichtung 130 dazu eine oder mehrere Regelungsschleifen aufweisen, was insbesondere gegenüber einer, insbesondere unmittelbaren, Regelung über das Steuerungssystem, speziell in Bezug auf Zeitanforderungen bzw. Rückkopplungszeiten, vorteilhaft ist. Insbesondere kann die Ansteuerungsvorrichtung 130 ein handelsüblicher Computer sein, der zur Ansteuerung eingerichtet ist. Vorzugsweise steuert die Ansteuerungsvorrichtung 130 die Herausgabemechanismen und ggf. weiteren Bestandteile des Behandlungswagens 100 elektrisch an und weist dazu einen Datenbus 132, vorzugsweise ausgebildet als Universal Serial Bus (USB), auf. Zur Verbindung mit Behandlungswagenmodulen weist der Datenbus insbesondere eine oder mehrere Verbindungseinrichtungen, insbesondere Verbindungseinheiten 135, insbesondere Stecker oder Buchsen auf. Dabei ist wenigstens eine der Verbindungseinheiten 135 benachbart zu einer der Verbindungsvorrichtungen 144 für Behandlungswagenmodule angeordnet. Auch kann der Datenbus 132 weitere Bestandteile des Behandlungswagens 100 - etwa die Benutzerschnittstelle 50 oder das Kameramodul 54 -, vorzugsweise elektrisch, datenverbinden, wobei diese Verbindungen für eine bessere Übersichtlichkeit in Figur 2 nur teilweise dargestellt sind. Weiter bevorzugt ist der Datenbus zur Energieversorgung, insbesondere mit elektrischem Strom, von Bestandteilen des Behandlungswagens 100, insbesondere eines oder mehrerer der Herausgabemechanismen und/oder eines oder mehrerer Behandlungswagenmodule und/oder der Benutzerschnittstelle 50 und/oder des Kameramoduls 54, eingerichtet.

Außerdem weist der Behandlungswagen 100 eine Entsorgungsvorrichtung 150 auf. Die Entsorgungsvorrichtung 150 weist ein Entsorgungsbehältnis 152, eine Öffnung 154 für zu entsorgende Sachen, insbesondere medizinische Zubehöre, einen Verschlussmechanismus 156 für diese Öffnung und eine auf Gewicht bzw. Gewichtsänderung basierende Sensorik 158 auf.

Vorzugsweise ist dabei die Ansteuerungsvorrichtung 130 zusammen mit der Sensorik 158 als Erkennungssystem für Behandlungen eingerichtet. Vorzugsweise weist dazu die Ansteuerungsvorrichtung 130 des Weiteren ein Zeitmessgerät, insbesondere eine Uhr oder eine Vorrichtung zur Synchronisation mit einer globalen Zeit - etwa GPS oder DCF77 -, auf und ist eingerichtet, bei festgelegter Behandlung und vorzugsweise bei zudem festgelegten jeweils durchzuführenden Behandlungsschritt eine Gewichtsänderung sowie die Zeitspanne zwischen dieser Gewichtsänderung und einer vorhergehenden Gewichtsänderung oder eine Initialisierung zu bestimmen und basierend darauf zu bestimmen: welcher Behandlungsschritt durchgeführt worden ist und/oder ob der Behandlungsschritt erfolgreich durchgeführt worden ist. Dabei führt das Steuerungssystem - oder die Ansteuerungsvorrichtung 130 als Bestandteil des Steuerungssystems - vorzugsweise ein Assistenzverfahren aus, wobei in einem Verfahrensschritt einer oder mehrere Behandlungsparameter bestimmt werden, welche die durchzuführende Behandlung, ihre Behandlungsschritte und/oder übliche Zeitspannen zur Durchführung der Behandlungsschritte und/oder übliche Gewichte der für die jeweilige Behandlungsschritte benötigten medizinischen Zubehöre, insbesondere im gebrauchten und ungebrauchten Zustand, kennzeichnen. Diese Behandlungsparameter stellt das Steuerungssystem der Ansteuerungsvorrichtung 130 bereit und sendet diese dazu vorzugsweise mittels der Datenverbindung über die Kommunikationseinrichtung 31 an die Ansteuerungsvorrichtung 130.

In einer bevorzugten Variante weist das Steuerungssystem oder die Ansteuerungsvorrichtung 130, insbesondere als Bestandteil des Steuerungssystems, eine Datenspeichervorrichtung 138 für Assistenzinformationen auf. Vorzugsweise sind diese Assistenzinformationen jeweils Bildfolgen oder Videos bezüglich der jeweils durchzuführenden Behandlungsschritte. Im Assistenzverfahren werden die folgenden Verfahrensschritte ausgeführt: Im ersten Verfahrensschritt Festlegen eines ersten durchzuführenden Behandlungsschritts auf Basis der durchzuführenden Behandlung gemäß Auswahlverfahren und/oder mittels des Erkennungssystems für Behandlungen; im zweiten Verfahrensschritt Abrufen von Assistenzinformationen aus der Datenspeichervorrichtung 138 bezüglich des jeweils durchzuführenden Behandlungsschritts und Ausgeben dieser Assistenzinformationen mittels der Benutzerschnittstelle 50; in einem weiteren Verfahrensschritt Bestimmen mittels des Erkennungssystems für Behandlungen, ob dieser Behandlungsschritt durchgeführt worden ist und ob dies erfolgreich gewesen ist; im Erfolgsfall Fortfahren mit dem zweiten Verfahrensschritt für den nächsten Behandlungsschritt der durchzuführenden Behandlung und andernfalls Fortfahren mit dem zweiten Verfahrensschritt für den nicht erfolgreich durchgeführten Behandlungsschritt. Dabei wird das Verfahren bei Vorliegen eines Abbruchkriteriums, insbesondere nach erfolgreichem Durchführen aller Behandlungsschritte, beendet. Auch kann die Ansteuerungsvorrichtung 130 und/oder das Steuerungssystem eingerichtet sein, jene medizinische Zubehöre, welche für den jeweils durchzuführenden Behandlungsschritt erforderlich sind herauszugeben und insbesondere medizinische Zubehöre wiederholt herauszugeben, wenn ein Behandlungsschritt nicht erfolgreich durchgeführt worden ist. Zusätzlich oder alternativ ist das Steuerungssystem oder die Ansteuerungsvorrichtung 130 vorzugsweise eingerichtet, eine Eingabe von dem Benutzer, insbesondere den Patienten, zu empfangen, welche einen nicht-erfolgreich durchgeführten Behandlungsschritt kennzeichnet, und daraufhin im Assistenzverfahren mit dem zweiten Verfahrensschritt für den nicht-erfolgreich durchgeführten Behandlungsschritt fortzufahren. Entsprechendes gilt für eine Eingabe bezüglich eines erfolgreich durchgeführten Behandlungsschritts. Auf diese vorteilhafte Weise wird es ermöglicht, dass sich die automatische Erkennung mit einer manuellen Eingabe ergänzt, wodurch einerseits die Assistenz automatisch erfolgen und der Benutzungskomfort gesteigert werden kann und andererseits, falls bei der automatischen Erkennung ein Fehler auftritt, dies manuell korrigiert werden kann.

Bei einer Dialysebehandlung mit AV-Fistel oder AV-Graft können als Assistenzinformationen insbesondere gespeichert sein und/oder ausgegeben werden: eine Illustration der medizinischen Zubehöre und ihrer Verwendung zur Desinfektion des zu kanülierenden Hautbereichs; eine Illustration des Einstechens der Kanüle; eine Illustration des Einführens des Katheters; und/oder eine Illustration des Befestigen des Katheters, insbesondere mit einem Klebeband oder Pflaster. Bezüglich des Einstechens ist das Steuerungssystem vorzugsweise eingerichtet, die ich jeweilige Einstichstelle und insbesondere den Einstichwinkel patientenspezifisch - also etwa gemäß Strickleiter-Technik oder Knopfloch-Technik - zu bestimmen. Bei der Strickleiter-Technik wird für jedes Einstechen eine neue Einstichstelle entlang des Blutgefäßes, d.h. insbesondere der AV-Fistel bzw. des AV-Graft, bestimmt, welche ein Stück weit, üblicherweise etwa 2 cm, von der vorhergehenden Einstichstelle entfernt ist. Bei der Knopfloch-Technik wird wiederholt dieselbe Einstichstelle verwendet, wobei speziell hierbei immer im gleichen Winkel eingestochen wird, so dass sich insbesondere ein Nadelloch ähnlich dem Loch für einen Ohrring ausbildet. Erst nach einer vorbestimmten Anzahl von Einstichen / Kanülierungen wird schließlich eine neue Einstichstelle gewählt.

Ferner ist die Ansteuerungsvorrichtung 130 vorzugsweise eingerichtet, mittels des Kameramoduls 54 das Durchführen einer Behandlung und/oder den Benutzer, insbesondere den Patienten, zu erfassen. Vorzugsweise ist die Ansteuerungsvorrichtung 130 eingerichtet, die so erfassten Daten auf einer Datenspeichervorrichtung des Behandlungswagens 100 zu speichern und/oder über eine Datenverbindung, insbesondere mittels der Kommunikationseinrichtung 31, an ein Steuerungssystem eines mobilen Auswahlsystems zu senden. Auf diese vorteilhafte Weise kann die Behandlung später nachvollzogen werden, etwa zur Qualitätssteigerung, und/oder der Patient während der Behandlung beobachtet werden und diesem gegebenenfalls Fernhilfe beim Durchführen der Behandlung oder von bestimmten Behandlungsschritten geleistet werden.

Schließlich ist der Behandlungswagen 100 vorzugsweise mit dem Behandlungswagenmodul 200 und/oder dem Behandlungswagenmodul 210 und/oder dem Behandlungswagenmodul 220 bestückt. Die Behandlungswagenmodule 200, 210, 220 weisen je eine Verbindungsstelleneinrichtung 244 mit wenigstens einer Verbindungsstelle zur formschlüssigen und/oder kraftschlüssigen Verbindung mit der jeweiligen Verbindungsvorrichtung 144 des Behandlungswagens 100 auf. Vorzugsweise weist ein Behandlungswagenmodul, insbesondere das Behandlungswagenmodul 200 und das Behandlungswagenmodul 210, eine Verbindungseinheit 235 für einen Datenbus auf. Dabei ist insbesondere die Verbindungseinheit 235 zur Verbindung mit einer Verbindungseinheit 135 des Datenbusses 132 eingerichtet, insbesondere als Buchse bzw. Stecker ausgebildet. Auch ist dabei insbesondere die Verbindungseinheit 235 benachbart zur Verbindungsstelleneinrichtung 244 zumindest so angeordnet, dass, wenn die Verbindungsstelleneinrichtung zu 144 benachbart zu der korrespondierenden Verbindungsvorrichtung 144 des Behandlungswagens angeordnet ist, auch die Verbindungseinheit 235 benachbart zur korrespondierenden Verbindungseinheit 135 angeordnet und damit verbindbar ist. Vorzugsweise sind die Verbindungsstelleneinrichtung 244 und die Verbindungseinheit 235 so angeordnet und eingerichtet, dass, wenn die Verbindungsstelleneinrichtung 244 mit der korrespondierenden Verbindungsvorrichtung 144 verbunden ist, auch die Verbindungseinheit 235 mit der Verbindungseinheit 135 verbunden ist.

Auch können andere Bestandteile des Behandlungswagens 100, insbesondere eine der Lagereinrichtungen 110, 114, 118, 120 oder die Entsorgungsvorrichtung 150, als Behandlungswagenmodul ausgebildet sein. Für eine solche als Behandlungswagenmodul ausgebildete Lagereinrichtung, insbesondere für ein Schubfach zusammen mit einem korrespondierenden Herausgabemechanismus, kann der Behandlungswagen vorzugsweise einen Verbindungsschacht aufweisen, welche eingerichtet ist, das Behandlungswagenmodul, insbesondere aus wenigstens dem Schubfach und dem Herausgabemechanismus, aufzunehmen.

Das Behandlungswagenmodul 220 weist eine manuelle Spendervorrichtung, insbesondere eine Handschuhbox, auf oder besteht aus einer als Behandlungswagenmodul ausgebildeten manuellen Spendervorrichtung. Diese weist einen Schacht 222 zum Lagern von medizinischen Zubehören 224, insbesondere paarweise verpackten Einmalhandschuhen, und eine Öffnung 226 zur Entnahme der medizinischen Zubehöre auf. Dabei ist, zumindest wenn das Behandlungswagenmodul 220 zu üblichen Verwendung mit dem Behandlungswagen 100 verbunden ist, der Schacht 222 bezüglich seiner Längsachse 228 so ausgerichtet und die Öffnung 226 so angeordnet und/oder sind beide so eingerichtet, dass, sobald das medizinische Zubehör 224, welches im Schacht 222 bei der Öffnung 226 angeordnet ist, durch die Öffnung 226 entnommen wird, ein weiteres medizinisches Zubehör 224 entlang der Längsachse 228 durch den Schacht 222 zur Öffnung 226 fällt oder gleitet. Da die manuelle Spendervorrichtung manuell betätigt wird, also insbesondere keinen Herausgabemechanismus aufweist, weist das Behandlungswagenmodul 220 vorzugsweise keine Verbindungseinheit 235 für den Datenbus auf. Alternativ und bevorzugt kann das Behandlungswagenmodul 220 eine Verbindungseinheit 235 aufweisen, insbesondere um mittels einer Sensorik den Füllstand der manuellen Spendervorrichtung zu erfassen und über den Datenbus 132 an die Ansteuerungsvorrichtung 130 zu übertragen.

Das Behandlungswagenmodul 200 weist eine Dosiervorrichtung auf oder besteht aus einer als Behandlungswagenmodul ausgebildeten Dosiervorrichtung. Als Ausführungsbeispiel ist die Dosiervorrichtung zur Dosierung eines flüssigen Desinfektionsmittels - etwa vorzugsweise einer Flüssigkeit mit Wasser, Ethanol, 2-Phenoxyethanol, Propan-1-ol, Propan-2-ol, Didecyldimethylammoniumchlorid und/oder Mecetroniumetilsulfat - ausgebildet. Diese weist eine Lagereinrichtung 202, welche als Behältnis für das Desinfektionsmittel ausgebildet ist, eine Aussonderungsvorrichtung 204, welche als Pumpe mit Zulauf ausgebildet ist, und einen Auslass 206 auf. Insbesondere ist also das Innere des Behältnisses 202 der Lagerbereich 203 für das Desinfektionsmittel. Die Pumpe 204 ist mittels des Zulaufs so mit dem Behältnis 202 flüssigkeitsverbunden, dass diese ein im Behältnis gelagertes Desinfektionsmittel dosiert abpumpen kann. Zudem ist die Pumpe mit dem Auslass 206 so flüssigkeitsverbunden, dass diese ein abgepumptes Desinfektionsmittel, d.h. insbesondere eine Dosis des Desinfektionsmittels, mittels des Auslass 206 herausgeben, insbesondere heraus sprühen kann.

Vorzugsweise ist der Auslass 206 so ausgerichtet, dass das Desinfektionsmittel in Richtung der Ablageeinrichtung 108 gesprüht wird. Auf diese vorteilhafte Weise lässt sich die Ablagefläche der Ablageeinrichtung 108 desinfizieren und/oder lassen sich weitere Sachen oder Körperteile, insbesondere Hände, desinfizieren, wenn sie in den Bereich zwischen dem Auslass 206 und der Ablageeinrichtung 108 gehalten werden.

Ferner weist das Auswahlverfahren, für dessen Ausführung das Steuerungssystem eingerichtet ist, des Weiteren die folgenden Verfahrensschritte auf. In einem Verfahrensschritt des Auswahlverfahrens wird überprüft, ob die Zubehörsetparameter ein medizinisches Zubehör einer dosierbaren Zubehörgattung, d.h. hier speziell das Desinfektionsmittel, kennzeichnen. In einem weiteren Verfahrensschritt des Auswahlverfahrens wird, falls dies der Fall ist, ein Dosierparameter bestimmt, welcher das dosierbare medizinische Zubehör, d.h. hier speziell das Desinfektionsmittel, kennzeichnet und/oder ein Dosierparameter bestimmt, welcher die für das Zubehörset benötigte Dosis kennzeichnet. Diese Dosis kann insbesondere die flüssigkeitsbezogene oder gewichtsbezogene Menge an Desinfektionsmittel sein. Außerdem ist das Steuerungssystem eingerichtet, die Pumpe 204 auf Basis der Dosierparameter und vorzugsweise der Zubehörsetparameter so zu steuern, dass die Pumpe 204 einen Teil des Desinfektionsmittels, welcher der benötigten Dosis entspricht, aus dem Behältnis 202 abpumpt. Vorzugsweise erfolgt das Steuern über eine Datenverbindung und mittels der Ansteuerungsvorrichtung 130, welche wiederum über den Datenbus 132 und über die Verbindungseinheit 135 und die korrespondierende, mit dieser verbundenen Verbindungseinheit 235 die Pumpe 204 ansteuert. Die Ansteuerung kann dabei ein Signal zum Betrieb und anschließend zum Stoppen der Pumpe sein oder alternativ ein Signal, welches die Dosis, das heißt die abzugebende Menge, codiert, wobei dieses Signal von einer Datenverarbeitungseinrichtung der Dosiervorrichtung bzw. des Behandlungswagenmoduls 200 verarbeitet und in ein Steuerungssignal - d.h. insbesondere: Pumpe betreiben, Abwarten einer vorbestimmten Zeitspanne und Pumpe nicht betreiben - für die Pumpe transformiert wird.

Das Behandlungswagenmodul 210 weist eine Lagereinrichtung 214, insbesondere als Lagereinrichtung des Behandlungswagens 100, mit einem oder mehreren, insbesondere wie in Figur 2 illustriert drei, Lagerbereichen 215 auf sowie einen Herausgabemechanismus, insbesondere als Herausgabemechanismus des Behandlungswagens, welcher als Aussonderungsvorrichtung ausgebildet ist. Die Lagerbereiche 215 sind insbesondere jeweils für genau ein medizinisches Zubehör vorgesehen, wobei die Lagereinrichtung 214 vorzugsweise Lagerbereiche für eine oder mehrere Zubehörgattungen aufweist. Die Aussonderungsvorrichtung weist einen Schacht 212, eine Öffnung 216 zur Herausgabe eines auszusondernden medizinischen Zubehörs und jeweils ein Aussonderungselement 213 für je einen der Lagerbereiche 215 auf. Vorzugsweise sind die Aussonderungselemente 213 als Klappmechanismen mit einer verstellbaren Klappe ausgebildet, so dass, bei einer Ansteuerung zur Aussonderung des im jeweiligen korrespondierenden Lagerbereich 215 gelagerten medizinischen Zubehörs, sich die Klappe öffnet, den jeweiligen Lagerbereich 215 zum Schacht 212 hin freigibt, dass im jeweiligen Lagerbereich 215 lagernde medizinische Zubehör zum Schacht 212 hin gleitet oder fällt und entlang der Längsachse 218 des Schachts 212 zur Öffnung 216 fällt oder gleitet. Des Weiteren kann dieses medizinische Zubehör durch die Öffnung 216 gelangen und somit an einen Herausgabebereich, insbesondere unterhalb der Öffnung 216, abgegeben werden. Zudem ist das Behandlungswagenmodul 210 vorzugsweise so bei dem Behandlungswagen 100 angeordnet, dass ein medizinisches Zubehör, welches durch die Öffnung 216 abgegeben wird, auf die Ablagefläche der Ablageeinrichtung 108 gelangt.

Ferner ist das Steuerungssystem eingerichtet, die Aussonderungsvorrichtung des Behandlungswagenmoduls 210 so zu steuern, dass diese, sofern die Zubehörsetparameter ein medizinisches Zubehör kennzeichnen, für welches die Lagereinrichtung 214 einen Lagerbereich 215 aufweist, dieses medizinische Zubehör aus dem Lagerbereich 215 entnimmt, insbesondere indem das mit dem Lagerbereich 215 korrespondierende Aussonderungselement 213 betätigt wird, und über den Schacht 212 sowie die Öffnung 216 an den Herausgabebereich, insbesondere die Ablagefläche der Ablageeinrichtung 108, abgibt. Vorzugsweise erfolgt das Steuern mittels der Ansteuerungsvorrichtung 130 und entsprechend der vorhergehenden Beschreibung bezüglich des Behandlungswagenmoduls 100.

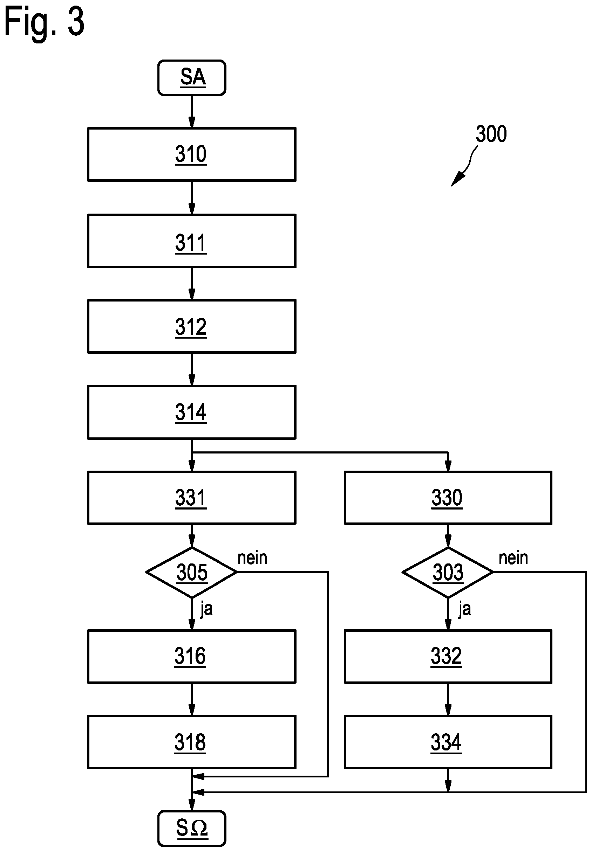

In Figur 3 ist ein Verfahren 300 zur Auswahl von medizinischen Zubehören gemäß einer nicht beanspruchten Ausführungsform dargestellt. Verfahrensschritte dieses Verfahrens 300 können auch Verfahrensschritte des Auswahlverfahrens und/oder des Assistenzverfahrens gemäß dem Ausführungsbeispiel bzw. der Ausführungsform bezüglich Figur 1 oder Figur 2 sein. Auch kann eine Ausführungsform des erfindungsgemäßen mobilen Auswahlsystems eingerichtet sein, ein solches Verfahren 300 auszuführen. Für das Verfahren 300 sind oder werden als Teil des Verfahrens 300 einer oder mehrere Behandlungswagen - etwa die Behandlungswagen 100, 160, 180 gemäß den Figuren 1 und/oder 2 - bereitgestellt, welche für eine Mehrzahl an medizinischen Zubehören je eine oder mehrere Lagereinrichtungen und je wenigstens zwei Lagerbereiche der Lagereinrichtungen aufweisen. Insbesondere ist dabei wenigstens einer, vorzugsweise jeder, Lagerbereich vorgesehen für und insbesondere eingerichtet für eines oder mehrere medizinische Zubehöre je genau einer Zubehörgattung der Mehrzahl an medizinischen Zubehören.

Das Verfahren 300 weist die Verfahrensschritte 310, 312, 314, 316, 318, 330, 331, 332, 334 und vorzugsweise den Verfahrensschritt 311 auf. Das Verfahren 300 beginnt bei dem Verfahrensstart SA und endet bei dem Verfahrensende SΩ, wobei einer oder mehrere Verfahrensschritte, insbesondere eine Sequenz von Verfahrensschritten, und vorzugsweise das gesamte Verfahren wiederholt ausgeführt werden kann.

In einem ersten Verfahrensschritt 310 wird eine oder werden mehrere Eingaben von einem Benutzer erfasst, durch die wenigstens eine durchzuführende Behandlung und vorzugsweise ein zu behandelnder Patient festgelegt wird. Die Erfassung erfolgt mittels einer Benutzerschnittstelle, welche in Datenverbindung mit einem Steuerungssystem, insbesondere eines mobilen Auswahlsystems, steht. Dabei kann das Verfahren 300 vorzugsweise in einer Warteschleife oder ereignis-gesteuerten Schleife verweilen, bis die Eingaben erfolgt sind und/oder bis ein Abbruchkriterium, welches ein Wiederholen des Verfahrensschritts 310 oder das Verfahrensende SΩ kennzeichnet, erfüllt ist - vorzugsweise etwa über eine Eingabe eines Benutzers für das erneute Ausführen des Verfahrensschritts 310 oder für das Beenden des Verfahrens 300 und/oder nach Ablauf einer vorbestimmten Zeitspanne.

Vorzugsweise wird in einem weiteren Verfahrensschritt 311 eine Patientenkennung des zu behandelnden Patienten auf Basis dieser Eingaben und mittels des Steuerungssystems bestimmt, sofern die Eingaben den zu behandelnden Patienten festlegen.

In einem weiteren Verfahrensschritt 312 wird eine Behandlungskennung der durchzuführenden Behandlung auf Basis der Eingaben mittels des Steuerungssystems bestimmt. Insbesondere kann dabei die durchzuführende Behandlung und entsprechend die zu bestimmende Behandlungskennung bereits ganz oder zumindest teilweise durch den zu behandelnden Patienten und seine Patientenkennung festgelegt sein.

In einem weiteren Verfahrensschritt 314 wird einer oder werden mehrere Zubehörsetparameter mittels des Steuerungssystems anhand einer Zubehördatenbank und wenigstens auf Basis der Behandlungskennung der durchzuführenden Behandlung sowie vorzugsweise auf Basis der Patientenkennung bestimmt, wobei die Zubehörsetparameter ein Zubehörset aus einem oder mehreren für die durchzuführende Behandlung und vorzugsweise für den zu behandelnden Patienten, insbesondere besonders, geeigneten medizinischen Zubehören kennzeichnen. Zudem ist für das Verfahren 300 oder wird als Teil des Verfahrens 300 die Zubehördatenbank bereitgestellt, wobei die Zubehördatenbank Datensätze über die Mehrzahl an medizinischen Zubehören aufweist und auf einer Datenspeichervorrichtung des Steuerungssystems gespeichert ist.

In einem weiteren Verfahrensschritt 331 wird überprüft, ob die Zubehörsetparameter wenigstens ein medizinisches Zubehör einer Zubehörgattung kennzeichnen, welche nicht zu dosieren ist, insbesondere da das medizinische Zubehör von einer nichtdosierbaren Zubehörgattung und/oder bereits vordosiert, vorportioniert und/oder einzeln verpackt ist - etwa Kanülen, einzeln verpackte Klebeband-Abschnitte oder Infusionsschläuche.

Falls die Verfahrensbedingung 305 erfüllt ist, das heißt die Zubehörsetparameter wenigstens ein nicht zu dosierendes medizinisches Zubehör kennzeichnen, wird in einem weiteren Verfahrensschritt 316 ein Herausgabemechanismus auf Basis der Zubehörsetparameter gesteuert, der zur Herausgabe von medizinischen Zubehören aus wenigstens einer der Lagereinrichtungen eingerichtet ist und der in Datenverbindung mit dem Steuerungssystem steht.

Schließlich werden in einem weiteren Verfahrensschritt 318 mittels des Herausgabemechanismus jene, insbesondere nicht zu dosierende, medizinischen Zubehöre herausgegeben, für deren Zubehörgattungen die wenigstens eine der Lagereinrichtungen Lagerbereiche aufweist und welche durch die Zubehörsetparameter, auf denen die Steuerung des Herausgabemechanismus basiert, gekennzeichnet sind.

Außerdem wird nach dem Verfahrensschritt 314 in einem weiteren Verfahrensschritt 330 überprüft, ob die Zubehörsetparameter ein medizinisches Zubehör einer dosierbaren Zubehörgattung kennzeichnen, also insbesondere eine Zubehörgattung bzw. ein medizinisches Zubehör kennzeichnen, von welchem nur ein Teil, das heißt nur eine bestimmte Dosis, auszuwählen sowie herauszugeben ist - etwa ein flüssiges Desinfektionsmittel oder ein Abschnitt einer Klebebandrolle.

Falls die Verfahrensbedingung 303 erfüllt ist, d.h. die Zubehörsetparameter ein medizinisches Zubehör einer dosierbaren Zubehörgattung kennzeichnen, wird in einem weiteren Verfahrensschritt 332 einer oder werden mehrere Dosierparameter bestimmt, welche das dosierbare medizinische Zubehör der dosierbaren Zubehörgattung und die für das Zubehörset benötigte Dosis kennzeichnen.

Daraufhin wird in einem weiteren Verfahrensschritt 334 eine Aussonderungsvorrichtung einer Dosiervorrichtung mit einem Lagerbereich, in welchem das dosierbare medizinische Zubehör lagert, so gesteuert, dass die Aussonderungsvorrichtung einen Teil des wenigstens einen dosierbaren medizinischen Zubehörs, welcher der benötigten Dosis entspricht, aus dem Lagerbereich entnimmt und an einem Herausgabebereich abgibt.

Während vorausgehend wenigstens ein bevorzugtes Ausführungsbeispiel beschrieben wurde, ist zu bemerken, dass eine große Anzahl von Variationen dazu existiert. Es ist auch zu beachten, dass die beschriebenen Ausführungsbeispiele nur nichtlimitierende Beispiele darstellen, und es nicht beabsichtigt ist, dadurch den Umfang, die Anwendbarkeit oder die Konfiguration der hier beschriebenen Systeme, Vorrichtungen und Verfahren zu beschränken. Vielmehr wird die vorausgehende Beschreibung dem Fachmann eine Anleitung zur Implementierung mindestens einer Ausführungsform liefern, wobei es sich versteht, dass verschiedene Änderungen in der Funktionsweise und der Anordnung der in einem bevorzugten Ausführungsbeispiel beschriebenen Elemente vorgenommen werden können, ohne dass dabei von dem in den angehängten Ansprüchen jeweils festgelegten Gegenstand abgewichen wird.

### Bezugszeichenlist

- 10: mobiles Auswahlsystem
- 20: Steuerungssystem
- 22: Zubehördatenbank
- 24: Datenspeichervorrichtung, insbesondere für die Zubehördatenbank
- 26: Datenverarbeitungsvorrichtung
- 28: Arbeitsspeicher
- 30: Kommunikationseinrichtung
- 31: Kommunikationseinrichtung für Behandlungswagen 100
- 32: Datenverbindung
- 50: Benutzerschnittstelle
- 52: Benutzerschnittstelle, insbesondere ausgebildet als Behandlungswagenmodul
- 54: Kameramodul, insbesondere ausgebildet als Behandlungswagenmodul
- 66: an der Behandlung beteiligte Person, insbesondere am Behandlungsort 86, insbesondere Patient oder Arzt
- 80, 86, 88: Behandlungsort
- 100, 160, 180: Behandlungswagen
- 102: Fahrvorrichtung, insbesondere ausgebildet als Behandlungswagenmodul
- 103: Anschlusseinrichtung für Stromnetz
- 104: Verbindungsvorrichtung für Fahrvorrichtung
- 108: Ablageeinrichtung des Behandlungswagens 100
- 110, 114, 118: Lagereinrichtung des Behandlungswagens 100, Insbesondere Schubfach
- 111: Herausgabemechanismus für die Lagereinrichtung 110
- 112: Lagerbereich der Lagereinrichtung 110
- 115: Herausgabemechanismus für die Lagereinrichtung 114
- 116: Lagerbereich der Lagereinrichtung 114
- 119: Lagerbereiche der Lagereinrichtung 118
- 120: Lagereinrichtung des Behandlungswagens 100, insbesondere Schrankfach
- 121: Herausgabemechanismus für die Lagereinrichtung 120
- 122, 124, 126: Lagerbereiche der Lagereinrichtung 120
- 130: Ansteuerungsvorrichtung des Behandlungswagens 100
- 132: Datenbus
- 135: Verbindungseinheit des Datenbus 132
- 138: Datenspeichervorrichtung für Assistenzinformationen
- 144: Verbindungsvorrichtung für Behandlungswagenmodul
- 150: Entsorgungsvorrichtung
- 152: Entsorgungsbehältnis
- 154: Öffnung für zu entsorgende Sachen, insbesondere medizinische Zubehöre
- 156: Verschlussmechanismus für die Öffnung 154
- 158: Sensorik für Gewicht bzw. Gewichtsänderung
- 162: Rad des Behandlungswagens 160
- 163: elektrischer Energiespeicher des Behandlungswagens 160
- 166: Erkennungssystem des Behandlungswagens 160
- 168: Ablageeinrichtung des Behandlungswagens 160
- 170: Lagereinrichtung des Behandlungswagens 160
- 171: Herausgabemechanismus für die Lagereinrichtung 170
- 172, 174, 176: Lagerbereiche der Lagereinrichtung 170
- 182: Rad des Behandlungswagens 180
- 183: elektrischer Energiespeicher des Behandlungswagens 180
- 184: Verbindungsvorrichtung für eine Benutzerschnittstelle
- 190: Lagereinrichtung des Behandlungswagens 180
- 191: Herausgabemechanismus für die Lagereinrichtung 190
- 192, 194, 196: Lagerbereiche der Lagereinrichtung 190
- 200: Behandlungswagenmodul, insbesondere ausgebildet als Dosiervorrichtung
- 202: Lagereinrichtung der Dosiervorrichtung
- 203: Lagerbereich der Lagereinrichtung 202
- 204: Aussonderungsvorrichtung der Dosiervorrichtung
- 206: Auslass der Dosiervorrichtung
- 210: Behandlungswagenmodul, insbesondere ausgebildet mit Aussonderungsvorrichtung
- 212: Schacht der Aussonderungsvorrichtung des Behandlungswagenmoduls 210
- 213: Aussonderungselement
- 214: Lagereinrichtung des Behandlungswagenmoduls 210
- 215: Lagerbereich der Lagereinrichtung 214
- 216: Öffnung zur Herausgabe eines auszusondernden medizinischen Zubehörs
- 218: Längsachse des Schachtes 212
- 220: Behandlungswagenmodul, insbesondere ausgebildet als Handschuhbox
- 222: Schacht einer manuellen Spendervorrichtung
- 224: medizinisches Zubehör, insbesondere gelagert in der manuellen Spendervorrichtung
- 226: Öffnung zur Entnahme eines medizinischen Zubehörs
- 228: Längsachse des Schachtes 222
- 235: Verbindungseinheit für den Datenbus
- 244: Verbindungsstelleneinrichtungen
- 300: Verfahren zur Auswahl von medizinischen Zubehören
- SA: Verfahrensstart
- SΩ: Verfahrensende
- 303: Verfahrensbedingung: die Zubehörsetparameter kennzeichnen ein medizinisches Zubehör einer dosierbaren Zubehörgattung
- 305: Verfahrensbedingung: die Zubehörsetparameter kennzeichnen wenigstens ein medizinisches Zubehör, welches nicht zu dosieren ist
- 310 bis 334: Verfahrensschritte

## Patentansprüche

1. Mobiles Auswahlsystem (10) zur Auswahl von medizinischen Zubehören, insbesondere für eine Kanülierung von Blutgefäßen von Patienten und/oder für eine Dialysebehandlung, wobei das mobile Auswahlsystem (10) aufweist:
ein Steuerungssystem (20) mit einer Zubehördatenbank (22), welche für Datensätze über eine Mehrzahl an medizinischen Zubehören eingerichtet und auf einer Datenspeichervorrichtung (24) des Steuerungssystems (20) gespeichert ist;
eine Benutzerschnittstelle (50, 52), die für eine oder mehrere Eingaben von einem Benutzer eingerichtet ist, durch die eine durchzuführende Behandlung festgelegt wird und auf Basis derer das Steuerungssystem (20) infolge dieser Eingaben eine der durchzuführenden Behandlung zugeordnete Behandlungskennung bestimmt;
einen Behandlungswagen (100, 160, 180) mit einer oder mehreren Lagereinrichtungen (110, 114, 120; 170; 190; 202, 214), wobei die Lagereinrichtungen wenigstens zwei Lagerbereiche (112, 116, 122, 124, 126; 172, 174, 176; 192, 194, 196; 203, 215) je für eine Zubehörgattung aus der Mehrzahl an medizinischen Zubehören aufweisen;
und wobei das Steuerungssystem (20) eingerichtet ist, ein Auswahlverfahren auszuführen, welches die folgenden Verfahrensschritte aufweist:
- (312) Bestimmen der Behandlungskennung der durchzuführenden Behandlung; und
- (314) Bestimmen eines oder mehrerer Zubehörsetparameter, welche ein medizinisches Zubehörset aus einem oder mehreren für die durchzuführende Behandlung geeigneten medizinischen Zubehören kennzeichnen, anhand der Zubehördatenbank (22) und wenigstens auf Basis der Behandlungskennung der durchzuführenden Behandlung;
wobei der Behandlungswagen (100, 160, 180) wenigstens einen Herausgabemechanismus (111, 115, 121; 171; 191; 204; 213 mit 212) zur Herausgabe von medizinischen Zubehören aufweist und das Steuerungssystem (20) eingerichtet ist, den wenigstens einen Herausgabemechanismus auf Basis der Zubehörsetparameter so zu steuern, dass der Herausgabemechanismus wenigstens für eine der Lagereinrichtungen jene medizinische Zubehöre herausgibt, für deren Zubehörgattungen diese Lagereinrichtung Lagerbereiche aufweist und welche durch die Zubehörsetparameter, auf denen die Steuerung basiert, gekennzeichnet sind,
wobei der Behandlungswagen (100, 160) eine desinfizierbare Ablagefläche oder eine Ablageeinrichtung (108, 168) mit einer desinfizierbaren Ablagefläche aufweist, wobei die Ablagefläche für die Ablage von medizinischen Zubehören eingerichtet ist und/oder einen oder mehrere Herausgabebereiche für das Aussondern von medizinischen Zubehören aufweist, und
der Behandlungswagen eine Desinfektionsvorrichtung für die Ablagefläche aufweist, und das Steuerungssystem (20) dazu eingerichtet ist, die Desinfektionsvorrichtung für die Ablagefläche so zu steuern, dass die Desinfektionsvorrichtung die Ablagefläche vor, während und/oder nach der durchzuführenden Behandlung und/oder vor, während und/oder nach bestimmten Behandlungsschritten der durchzuführenden Behandlung desinfiziert.

2. Mobiles Auswahlsystem (10) gemäß Anspruch 1, wobei eine der Lagereinrichtungen ein Schubfach (110, 114; 170; 190) mit einem oder mehreren Lagerbereichen (112, 116; 172, 174, 176; 192, 194, 196) je für eine Zubehörgattung aus der Mehrzahl an medizinischen Zubehören aufweist oder daraus besteht und der wenigstens eine Herausgabemechanismus (111; 171; 191) oder ein weiterer Herausgabemechanismus (115) des Behandlungswagens für diese Lagereinrichtung eingerichtet ist, das Schubfach zu öffnen, vorzugsweise herauszufahren.

3. Mobiles Auswahlsystem (10) gemäß Anspruch 1 oder 2, wobei:
der wenigstens eine Herausgabemechanismus oder ein weiterer Herausgabemechanismus des Behandlungswagens (100) eine Aussonderungsvorrichtung (213 mit 212) für eine der Lagereinrichtungen (214) aufweist oder daraus besteht, welche zur Aussonderung von medizinischen Zubehören aus wenigstens einem der Lagerbereiche (215) dieser Lagereinrichtung (214) eingerichtet ist; und
das Steuerungssystem (20) eingerichtet ist, die Aussonderungsvorrichtung auf Basis der Zubehörsetparameter so zu steuern, dass die Aussonderungsvorrichtung wenigstens ein medizinisches Zubehör aus dem wenigstens einen Lagerbereich (215) dieser Lagereinrichtung entnimmt und an einem Herausgabebereich abgibt, wenn die Zubehörsetparameter ein solches medizinisches Zubehör kennzeichnen.

4. Mobiles Auswahlsystem (10) gemäß einem der vorhergehenden Ansprüche, wobei der Behandlungswagen (100) eine Dosiervorrichtung (200) mit einer der Lagereinrichtungen (202), welche wenigstens einen Lagerbereich (203) für wenigstens ein medizinisches Zubehör einer dosierbaren Zubehörgattung aufweist, und mit einer Aussonderungsvorrichtung (204) zur teilweisen Entnahme des wenigstens einen dosierbaren medizinischen Zubehörs aufweist;
wobei das Auswahlverfahren des Weiteren die folgenden Verfahrensschritte aufweist:
- (330) Überprüfen, ob die Zubehörsetparameter ein medizinisches Zubehör dieser dosierbaren Zubehörgattung kennzeichnen; und
- falls dies der Fall (303) ist, (332) Bestimmen eines oder mehrerer Dosierparameter, welche das dosierbare medizinische Zubehör und die für das Zubehörset benötigte Dosis kennzeichnen;
und wobei das Steuerungssystem (20) eingerichtet ist, die Aussonderungsvorrichtung (204) der Dosiervorrichtung (200) auf Basis der Dosierparameter und vorzugsweise der Zubehörsetparameter so zu steuern, dass die Aussonderungsvorrichtung einen Teil des wenigstens einen dosierbaren medizinischen Zubehörs, welcher der benötigten Dosis entspricht, aus dem wenigstens einen Lagerbereich (203) der Dosiervorrichtung entnimmt und an einem Herausgabebereich abgibt.

5. Mobiles Auswahlsystem (10) gemäß einem der vorhergehenden Ansprüche, wobei:
der Behandlungswagen (100; 180) eine Verbindungsvorrichtung (104, 144; 184) für Behandlungswagenmodule (102, 200, 210, 220; 52; 54) aufweist; und
die Verbindungsvorrichtung eingerichtet ist, eine formschlüssige, kraftschlüssige und/oder stoffschlüssige Verbindung zwischen dem Behandlungswagen und einem oder mehreren Behandlungswagenmodulen auszubilden.

6. Mobiles Auswahlsystem (10) gemäß einem der vorhergehenden Ansprüche, wobei der Behandlungswagen (100) eine Verbindungsvorrichtung (104) für eine von dem Behandlungswagen abtrennbare Fahrvorrichtung (102) aufweist.

7. Mobiles Auswahlsystem (10) gemäß einem der vorhergehenden Ansprüche, wobei:
entweder die Benutzerschnittstelle (50) mit dem Behandlungswagen (100) unlösbar verbunden ist;
oder die Benutzerschnittstelle (52) mit dem Behandlungswagen (180) lösbar verbunden oder verbindbar ist und/oder die Benutzerschnittstelle (52), während diese für Eingaben eingerichtet ist, infolge derer das Steuerungssystem (20) die Behandlungskennung der durchzuführenden Behandlung bestimmt, an einem geographisch anderen Ort als dem Ort des Behandlungswagens (160, 180) positioniert oder positionierbar ist.

8. Mobiles Auswahlsystem (10) gemäß einem der vorhergehenden Ansprüche, welches wenigstens einen weiteren Behandlungswagen (100, 160, 180) aufweist und wobei das mobile Auswahlsystem (10) wenigstens eine weitere Benutzerschnittstelle (50, 52) aufweist und/oder wenigstens eine der Benutzerschnittstellen (52) dem Behandlungswagen und dem weiteren Behandlungswagen für Eingaben zuordenbar ist und dazu:
wenigstens eine der Benutzerschnittstellen (50) mit einem der Behandlungswagen (100, 160, 180) unlösbar verbunden und damit diesem Behandlungswagen (100) zugeordnet ist;
das mobile Auswahlsystem für jeden der Behandlungswagen jeweils eine solche unlösbar verbundene Benutzerschnittstelle aufweist, welche damit dem jeweiligen Behandlungswagen zugeordnet ist;
wenigstens eine der Benutzerschnittstellen mit einem der Behandlungswagen lösbar verbunden und damit diesem Behandlungswagen zugeordnet ist; und/oder das Steuerungssystem (20) eine Zuordnungsvorschrift für Benutzerschnittstellen (50, 52) und eine Datenspeichervorrichtung (24) für diese aufweist und diese Zuordnungsvorschrift eingerichtet ist, anhand von Zuordnungsdaten, welche auf dieser Datenspeichervorrichtung (24) gespeichert sind, einer Benutzerschnittstelle,
welche mittels einer Benutzerschnittstellenkennung gekennzeichnet ist, einen Behandlungswagen (100, 160, 180), welcher mittels einer Behandlungswagenkennung gekennzeichnet ist, zuzuordnen.

9. Mobiles Auswahlsystem (10) gemäß einem der vorhergehenden Ansprüche, wobei das Steuerungssystem (20) eingerichtet ist, den Benutzer in Abhängigkeit von einer erfolgreichen Identifizierung am System anzumelden, eine Benutzerkennung zu bestimmen, das Auswahlverfahren auszuführen und/oder die Auswahl oder die Herausgabe von medizinischen Zubehören auf Zubehörgattungen zu beschränken, für welche der angemeldete Benutzer autorisiert ist, und/oder, falls der Benutzer nicht erfolgreich identifiziert worden ist, das Auswahlverfahren nicht auszuführen und/oder die Auswahl oder die Herausgabe von medizinischen Zubehören auf Zubehörgattungen für nicht-autorisierte Benutzer zu beschränken.

10. Mobiles Auswahlsystem (10) gemäß einem der vorhergehenden Ansprüche, wobei:
eine der Benutzerschnittstellen (50, 52) für eine oder mehrere Eingaben von dem Benutzer eingerichtet ist, durch die ein zu behandelnder Patient festgelegt wird und auf Basis derer das Steuerungssystem (20) infolge dieser Eingaben eine dem zu behandelnden Patienten zugeordnete Patientenkennung bestimmt;
das Auswahlverfahren des Weiteren den Verfahrensschritt (311) Bestimmen der Patientenkennung des zu behandelnden Patienten aufweist; und
bei dem Auswahlverfahren die Zubehörsetparameter auf Basis der Behandlungskennung und/oder der Patientenkennung bestimmt (314) werden.

11. Mobiles Auswahlsystem (10) gemäß einem der vorhergehenden Ansprüche, welches des Weiteren ein Erkennungssystem (130 mit 158; 166) für Behandlungen aufweist, das mittels einer auf Messung von Strahlung, von Ultraschall, von Gewicht und/oder von Trägheit basierenden Sensorik (158) eine an der Behandlung beteiligte Person (66), ein medizinisches Zubehör und/oder deren Anordnung und/oder deren Bewegung zueinander erfasst und basierend darauf eine durchgeführte Behandlung und/oder einen durchgeführten Behandlungsschritt bestimmt.

12. Mobiles Auswahlsystem (10) gemäß einem der vorhergehenden Ansprüche, wobei das Steuerungssystem (20) eingerichtet ist, ein Assistenzverfahren auszuführen, welches wenigstens den Verfahrensschritt Bestimmen eines oder mehrerer Assistenzparameter, welche die durchzuführende Behandlung, einen durchzuführenden Behandlungsschritt der durchzuführenden Behandlung und/oder ein medizinisches Zubehör dafür kennzeichnen.

13. Behandlungswagen (100) für ein mobiles Auswahlsystem (10) gemäß einem der vorhergehenden Ansprüche, wobei der Behandlungswagen (100) aufweist:
die Benutzerschnittstelle (50) des mobilen Auswahlsystems;
eine oder mehrere Lagereinrichtungen (110; 114; 120; 202; 214), wobei die Lagereinrichtungen wenigstens zwei Lagerbereiche (112; 116; 122, 124, 126; 203; 215) für medizinische Zubehöre je von einer Zubehörgattung aufweisen;
wenigstens einen Herausgabemechanismus (111; 115; 121; 204; 213 mit 212) zur Herausgabe von medizinischen Zubehören;
eine Ansteuerungsvorrichtung (130), welche zumindest einen Teil des Steuerungssystems (20) des mobilen Auswahlsystems (10) und/oder eine Kommunikationseinrichtung (31) zur Ausbildung einer Datenverbindung (32) mit dem Steuerungssystem (20) bzw. einem anderen Teil des Steuerungssystems aufweist, wobei die Ansteuerungsvorrichtung (130) für die Steuerung des wenigstens einen Herausgabemechanismus durch das Steuerungssystem (20) eingerichtet ist, den wenigstens einen Herausgabemechanismus auf Basis der Zubehörsetparameter elektrisch anzusteuern; und
eine desinfizierbare Ablagefläche oder eine Ablageeinrichtung (108, 168) mit einer desinfizierbaren Ablagefläche, wobei die Ablagefläche für die Ablage von medizinischen Zubehören eingerichtet ist und/oder einen oder mehrere Herausgabebereiche für das Aussondern von medizinischen Zubehören aufweist, und eine Desinfektionsvorrichtung für die Ablagefläche, wobei
die Ansteuerungsvorrichtung (130) für die Steuerung der Desinfektionsvorrichtung für die Abgabefläche durch das Steuerungssystem (20) eingerichtet ist, die Desinfektionsvorrichtung für die Ablagefläche so zu steuern, dass die Desinfektionsvorrichtung die Ablagefläche vor, während und/oder nach der durchzuführenden Behandlung und/oder vor, während und/oder nach bestimmten Behandlungsschritten der durchzuführenden Behandlung desinfiziert.

14. Verfahren (300) zur Auswahl von medizinischen Zubehören aus einer Mehrzahl an medizinischen Zubehören, insbesondere für eine Kanülierung von Blutgefäßen von Patienten und/oder für eine Dialysebehandlung, und dabei zur Herausgabe dieser medizinischen Zubehöre aus einem Behandlungswagen (100, 160, 180) mit einer oder mit mehreren Lagereinrichtungen (110, 114, 120; 170; 190; 202, 214), mit einer desinfizierbaren Ablagefläche oder einer Ablageeinrichtung (108, 168) mit einer desinfizierbaren Ablagefläche, wobei die Ablagefläche für die Ablage von medizinischen Zubehören eingerichtet ist und/oder einen oder mehrere Herausgabebereiche für das Aussondern von medizinischen Zubehören aufweist, und mit einer Desinfektionsvorrichtung für die Ablagefläche, wobei die medizinischen Zubehöre der Mehrzahl an medizinischen Zubehören gemäß ihrer jeweiligen Zubehörgattungen sortiert in wenigstens zwei Lagerbereichen (112, 116, 122, 124, 126; 172, 174, 176; 192, 194, 196; 203, 215) der Lagereinrichtungen lagern, mit den folgenden Verfahrensschritten:
(310) Erfassen einer oder mehrere Eingaben von einem Benutzer, durch die eine durchzuführende Behandlung festgelegt wird, mittels einer Benutzerschnittstelle (50, 52), die in Datenverbindung mit einem Steuerungssystem (20) steht;
(312) Bestimmen einer Behandlungskennung der durchzuführenden Behandlung auf Basis dieser Eingaben mittels des Steuerungssystems (20);
(314) Bestimmen eines oder mehrerer Zubehörsetparameter, welche ein medizinisches Zubehörset aus einem oder mehreren für die durchzuführende Behandlung geeigneten medizinischen Zubehören kennzeichnen, anhand einer Zubehördatenbank (22) und wenigstens auf Basis der Behandlungskennung der durchzuführenden Behandlung mittels des Steuerungssystems (20), wobei die Zubehördatenbank Datensätze über die Mehrzahl an medizinischen Zubehören aufweist und auf einer Datenspeichervorrichtung (24) des Steuerungssystems (20) gespeichert ist;
(316) Steuern eines Herausgabemechanismus (111, 115, 121; 171; 191; 204; 213 mit 212), der zur Herausgabe von medizinischen Zubehören aus wenigstens einer der Lagereinrichtungen eingerichtet ist und der in Datenverbindung mit dem Steuerungssystem (20) steht, auf Basis der Zubehörsetparameter, wobei der Behandlungswagen (100, 160, 180) den Herausgabemechanismus aufweist; und
(318) Herausgeben mittels des Herausgabemechanismus jener medizinischen Zubehöre, für deren Zubehörgattungen die wenigstens eine der Lagereinrichtungen Lagerbereiche aufweist und welche durch die Zubehörsetparameter, auf denen die Steuerung des Herausgabemechanismus basiert, gekennzeichnet sind,
Ansteuern der Desinfektionsvorrichtung für die Ablagefläche mittels des Steuerungssystems (20) derart, dass die Desinfektionsvorrichtung die Ablagefläche vor, während und/oder nach der durchzuführenden Behandlung und/oder vor, während und/oder nach bestimmten Behandlungsschritten der durchzuführenden Behandlung desinfiziert.

## Claims

1. Mobile selection system (10) for selecting medical supplies, in particular for cannulation of blood vessels of patients and/or for dialysis treatment, the mobile selection system (10) comprising:
a control system (20) with a supply database (22), which is set up for data records about a plurality of medical supplies and is stored on a data storage device (24) of the control system (20);
a user interface (50, 52) adapted for one or more inputs from a user by which a treatment to be performed is determined and based on which the control system (20) determines a treatment identifier associated with the treatment to be performed as a result of those inputs;
a treatment trolley (100, 160, 180) having one or more storage means (110, 114, 120; 170; 190; 202, 214), the storage means having at least two storage areas (112, 116, 122, 124, 126; 172, 174, 176; 192, 194, 196; 203, 215) each for one type of supply among the plurality of medical supplies;
and wherein the control system (20) is adapted to execute a selection method comprising the following method steps:
(312) determining the treatment identifier of the treatment to be performed; and
(314) determining one or more supply set parameters characterizing a medical supply set of one or more medical supplies appropriate for the treatment to be performed from the supply database (22) and based on at least the treatment identifier of the treatment to be performed;
and wherein the control system (20) is arranged to execute a selection method comprising the following method steps:
wherein the treatment trolley (100, 160, 180) comprises at least one discharge mechanism (111, 115, 121; 171; 191; 204; 213 with 212) for discharging medical supplies and the control system (20) is arranged to control the at least one discharge mechanism on the basis of the supply set parameters such that the discharge mechanism discharges, at least for one of the storage devices, those medical supplies for whose supply types this storage device comprises storage areas and which are **characterized by** the supply set parameters on which the control is based,
wherein the treatment trolley (100, 160) comprises a disinfectable storage area or a storage means (108, 168) comprising a disinfectable storage area, said storage area being arranged for the storage of medical supplies and/or comprising one or more discharge areas for the discharge of medical supplies, and
the treatment trolley comprises a disinfection device for the storage surface, and the control system (20) is adapted to control the disinfection device for the storage surface such that the disinfection device disinfects the storage surface before, during and/or after the treatment to be performed and/or before, during and/or after certain treatment steps of the treatment to be performed.

2. Mobile selection system (10) according to claim 1, wherein one of the storage devices comprises or consists of a drawer (110, 114; 170; 190) with one or more storage areas (112, 116; 172, 174, 176; 192, 194, 196) each for one supply type from the plurality of medical supplies and the at least one release mechanism (111; 171; 191) or a further retrieval mechanism (115) of the treatment trolley for this storage device is arranged to open, preferably to drive out, the drawer.

3. Mobile selection system (10) according to claim 1 or 2, wherein:
the at least one retrieval mechanism or a further retrieval mechanism of the treatment trolley (100) comprises or consists of a reject device (213 with 212) for one of the storage devices (214), which is arranged to reject medical supplies from at least one of the storage areas (215) of this storage device (214); and
said control system (20) is adapted to control said reject device based on said supply set parameters such that said reject device removes at least one medical supply from said at least one storage area (215) of said storage device and delivers it to a discharge area when said supply set parameters identify such medical supply.

4. Mobile selection system (10) according to any one of the preceding claims,
wherein said treatment trolley (100) comprises a dosing device (200) with one of said storage means (202) comprising at least one storage area (203) for at least one medical supply of a dosing supply type, and with a reject device (204) for partial removal of said at least one dosing medical supply;
the selection method further comprising the steps of:
(330) checking whether the supply set parameters identify a medical supply of that dispensable supply type; and
if so (303), (332) determining one or more dosage parameters identifying the dosable medical supply and the dose required for the supply set;
and wherein the control system (20) is adapted to control the reject device (204) of the dosing device (200) based on the dosing parameters and preferably the supply set parameters such that the reject device removes a portion of the at least one dosable medical supply corresponding to the required dose from the at least one storage area (203) of the dosing device and delivers it to a discharge area.

5. Mobile selection system (10) according to any one of the preceding claims, wherein:
the treatment trolley (100; 180) comprises a connection device (104, 144; 184) for treatment trolley modules (102, 200, 210, 220; 52; 54); and
the connecting device is arranged to form a positive, frictional and/or material connection between the treatment trolley and one or more treatment trolley modules.

6. Mobile selection system (10) according to any one of the preceding claims, wherein the treatment trolley (100) comprises a connecting device (104) for a travelling device (102) detachable from the treatment trolley.

7. Mobile selection system (10) according to any one of the preceding claims, wherein:
either the user interface (50) is non-detachably connected to the treatment trolley (100); or the user interface (52) is detachably connected or connectable to the treatment trolley (180) and/or the user interface (52), while arranged for inputs as a result of which the control system (20) determines the treatment identifier of the treatment to be performed, is positioned or positionable at a geographically different location than the location of the treatment trolley (160, 180).

8. Mobile selection system (10) according to any one of the preceding claims, which comprises at least one further treatment trolley (100, 160, 180) and wherein the mobile selection system (10) comprises at least one further user interface (50, 52) and/or at least one of the user interfaces (52) is assignable to the treatment trolley and the further treatment trolley for inputs and thereto:
at least one of the user interfaces (50) is non-detachably connected to one of the treatment trolleys (100, 160, 180) and is thus assigned to this treatment trolley (100); the mobile selection system comprises for each of the treatment trolleys a respective such non-detachably connected user interface which is associated therewith with the respective treatment trolley;
at least one of the user interfaces is detachably connected to one of the treatment trolleys and thus associated with this treatment trolley; and/or
the control system (20) has an assignment rule for user interfaces (50, 52) and a data storage device (24) for the latter, and this assignment rule is set up to assign, on the basis of assignment data which are stored on this data storage device (24), a treatment trolley (100, 160, 180) which is identified by means of a treatment trolley identifier, to a user interface which is identified by means of a user interface identifier.

9. Mobile selection system (10) according to any one of the preceding claims, wherein the control system (20) is arranged to register the user with the system in dependence on successful identification, to determine a user ID, to execute the selection procedure and/or to restrict the selection or discharge of medical supplies to supply types for which the registered user is authorized and/or, if the user has not been successfully identified, not to execute the selection procedure and/or to restrict the selection or discharge of medical supplies to supply types for non-authorized users.

10. Mobile selection system (10) according to any one of the preceding claims, wherein:
one of the user interfaces (50, 52) is arranged for one or more inputs from the user by which a patient to be treated is determined and based on which the control system (20) determines a patient identifier associated with the patient to be treated as a result of these inputs;
the selection method further comprises the step (311) of determining the patient identifier of the patient to be treated; and
in the selection method, the supply set parameters are determined (314) based on the treatment identifier and/or the patient identifier.

11. Mobile selection system (10) according to any one of the preceding claims, further comprising a treatment recognition system (130 with 158; 166) that detects a person (66) involved in the treatment, a medical supply and/or their arrangement and/or their movement relative to each other by means of a sensory system (158) based on measurement of radiation, of ultrasound, of weight and/or of inertia, and determines a performed treatment and/or a performed treatment step based thereon.

12. Mobile selection system (10) according to any one of the preceding claims, wherein the control system (20) is adapted to perform an assistance procedure comprising at least the step of determining one or more assistance parameters characterizing the treatment to be performed, a treatment step to be performed of the treatment to be performed and/or a medical supply therefor.

13. Treatment trolley (100) for a mobile selection system (10) according to any one of the preceding claims, wherein the treatment trolley (100) comprises:
the user interface (50) of the mobile selection system;
one or more storage devices (110; 114; 120; 202; 214), the storage devices comprising at least two storage areas (112; 116; 122, 124, 126; 203; 215) for medical supplies each of a type of supply;
at least one discharge mechanism (111; 115; 121; 204; 213 with 212) for discharging medical supplies;
a control device (130) comprising at least a part of the control system (20) of the mobile selection system (10) and/or communication means (31) for forming a data connection (32) with the control system (20) or another part of the control system, respectively, wherein the control device (130) for controlling the at least one discharge mechanism by the control system (20) is adapted to electrically control the at least one discharge mechanism based on the supply set parameters; and
a disinfectable storage surface or a storage device (108, 168) having a disinfectable storage surface, the storage surface being adapted for storage of medical supplies and/or having one or more discharge areas for discharging medical supplies, and
a disinfection device for the storage surface, wherein
the control device (130) for controlling the disinfection device for the delivery surface by the control system (20) is adapted to control the disinfection device for the delivery surface such that the disinfection device disinfects the delivery surface before, during and/or after the treatment to be performed and/or before, during and/or after certain treatment steps of the treatment to be performed.

14. Method (300) for selecting medical supplies from a plurality of medical supplies, in particular for cannulation of blood vessels of patients and/or for dialysis treatment, and thereby for discharging these medical supplies from a treatment trolley (100, 160, 180) having one or more storage devices (110, 114, 120; 170; 190; 202, 214), having a disinfectable storage area or storage means (108, 168) having a disinfectable storage area, the storage area being arranged for the storage of medical supplies and/or having one or more discharge areas for the discharge of medical supplies, and having a disinfection device for the storage area, wherein the medical supplies of the plurality of medical supplies are sorted according to their respective supply classes in at least two storage areas (112, 116, 122, 124, 126; 172, 174, 176; 192, 194, 196; 203, 215) of the storage facilities, comprising the following method steps:
(310) acquiring one or more inputs from a user by which a treatment to be performed is determined, by means of a user interface (50, 52) in data communication with a control system (20);
(312) determining a treatment identifier of the treatment to be performed based on these inputs by means of the control system (20);
(314) determining one or more supply set parameters characterizing a medical supply set of one or more medical supplies appropriate for the treatment to be performed from an supply database (22) and at least based on the treatment identifier of the treatment to be performed by the control system (20), wherein the supply database comprises records of the plurality of medical supplies and is stored on a data storage device (24) of the control system (20);
(316) controlling a retrieval mechanism (111, 115, 121; 171; 191; 204; 213 with 212) adapted to retrieve medical supplies from at least one of said storage devices and in data communication with said control system (20) based on said supply set parameters, wherein said treatment trolley (100, 160, 180) comprises said retrieval mechanism; and
(318) discharge, by the discharge mechanism, those medical supplies for which the at least one of the storage devices has storage areas and which are identified by the supply set parameters on which control of the discharge mechanism is based,
controlling the disinfection device for the storage area by means of the control system (20) in such a way that the disinfection device disinfects the storage area before, during and/or after the treatment to be performed and/or before, during and/or after certain treatment steps of the treatment to be performed.

## Revendications

1. Système de sélection (10) mobile destiné à sélectionner des accessoires médicaux, en particulier pour une cannulation de vaisseaux sanguins de patients et/ou pour un traitement par dialyse, dans lequel le système de sélection (10) mobile présente :
un système de commande (20) avec une banque de données d'accessoires (22), laquelle est conçue pour des ensembles de données sur une pluralité d'accessoires médicaux et mémorisée sur un dispositif de mémoire de données (24) du système de commande (20) ;
une interface utilisateur (50, 52) qui est conçue pour une ou plusieurs entrées par un utilisateur, via lesquelles un traitement à réaliser est établi et sur la base duquel le système de commande (20) détermine à la suite de ces entrées un identificateur de traitement attribué au traitement à réaliser ;
un chariot de traitement (100, 160, 180) avec un ou plusieurs équipements de stockage (110, 114, 120 ; 170 ; 190 ; 202, 214), dans lequel les équipements de stockage présentent au moins deux zones de stockage (112, 116, 122, 124, 126 ; 172, 174, 176 ; 192, 194, 196 ; 203, 215) chacune pour une catégorie d'accessoires parmi la pluralité d'accessoires médicaux ;
et dans lequel le système de commande (20) est conçu pour exécuter un procédé de sélection, lequel présente les étapes de procédé suivantes :
- (312) déterminer l'identificateur de traitement du traitement à réaliser ; et
- (314) déterminer un ou plusieurs paramètres de réglage d'accessoire, lesquels identifient un ensemble d'accessoires médical parmi un ou plusieurs des accessoires médicaux appropriés pour le traitement à réaliser, à l'aide de la banque de données d'accessoires (22) et au moins sur la base de l'identificateur de traitement du traitement à réaliser ;
dans lequel le chariot de traitement (100, 160, 180) présente au moins un mécanisme de restitution (111, 115, 121 ; 171 ; 191 ; 204, 213 avec 212) pour restituer des accessoires médicaux et le système de commande (20) est conçu pour commander le au moins un mécanisme de restitution sur la base des paramètres de réglage d'accessoire de sorte que le mécanisme de restitution restitue, pour au moins un des équipements de stockage, les accessoires médicaux pour les catégories d'accessoires desquels cet équipement de stockage présente des zones de stockage et lesquels sont **caractérisés par** des paramètres de réglage d'accessoire sur lesquels se base la commande,
dans lequel le chariot de traitement (100, 160) présente une surface de dépôt pouvant être désinfectée ou un équipement de dépôt (108, 168) avec une surface de dépôt pouvant être désinfectée, dans lequel la surface de dépôt est conçue pour le dépôt d'accessoires médicaux et/ou présente une ou plusieurs zones de restitution pour l'extraction d'accessoires médicaux, et
le chariot de traitement présente un dispositif de désinfection pour la surface de dépôt et le système de commande (20) est conçu pour commander le dispositif de désinfection pour la surface de dépôt de sorte que le dispositif de désinfection désinfecte la surface de dépôt avant, pendant et/ou après le traitement à réaliser et/ou avant, pendant et/ou après des étapes de traitement déterminées du traitement à réaliser.

2. Système de sélection (10) mobile selon la revendication 1, dans lequel l'un des équipements de stockage présente un tiroir (110, 114 ; 170 ; 190) avec une ou plusieurs zones de stockage (112, 116 ; 172, 174, 176 ; 192, 194, 196) chacun pour une catégorie d'accessoires parmi la pluralité d'accessoires médicaux, ou en est composé, et le au moins un mécanisme de restitution (111 ; 171 ; 191) ou un autre mécanisme de restitution (115) du chariot de traitement pour cet équipement de stockage est conçu pour ouvrir, de préférence faire sortir le tiroir.

3. Système de sélection (10) mobile selon la revendication 1 ou 2, dans lequel :
le au moins un mécanisme de restitution ou un autre mécanisme de restitution du chariot de traitement (100) présente un dispositif d'extraction (213 avec 212) pour l'un des équipements de stockage (214), ou en est composé, lequel est conçu pour extraire des accessoires médicaux depuis au moins l'une des zones de stockage (215) de cet équipement de stockage (214) ; et
le système de commande (20) est conçu pour commander le dispositif d'extraction sur la base des paramètres de réglage d'accessoire, de sorte que le dispositif d'extraction prélève au moins un accessoire médical depuis la au moins une zone de stockage (215) de cet équipement de stockage et le transmette à une zone de restitution lorsque les paramètres de réglage d'accessoire identifient un tel accessoire médical.

4. Système de sélection (10) mobile selon l'une des revendications précédentes,
dans lequel le chariot de traitement (100) présente un dispositif de dosage (200) avec l'un des équipements de stockage (202), lequel présente au moins une zone de stockage (203) pour au moins un accessoire médical d'une catégorie d'accessoires pouvant être dosée, et avec un dispositif d'extraction (204) pour le prélèvement partiel du au moins un accessoire médical pouvant être dosé ;
dans lequel le procédé de sélection présente en outre les étapes de procédé suivantes :
- (330) vérifier si les paramètres de réglage d'accessoire identifient un accessoire médical de cette catégorie d'accessoires pouvant être dosée ; et
- si tel est le cas (303), (332) déterminer un ou plusieurs paramètres de dosage, lesquels identifient l'accessoire médical pouvant être dosé et la dose requise pour l'ensemble d'accessoires ;
et dans lequel le système de commande (20) est conçu pour commander le dispositif d'extraction (204) du dispositif de dosage (200) sur la base des paramètres de dosage et de préférence des paramètres de réglage d'accessoire, de sorte que le dispositif d'extraction prélève une partie du au moins un accessoire médical pouvant être dosé, lequel correspond à la dose requise, depuis la au moins une zone de stockage (203) du dispositif de dosage et la transmette à une zone de restitution.

5. Système de sélection (10) mobile selon l'une des revendications précédentes, dans lequel :
le chariot de traitement (100 ; 180) présente un dispositif de liaison (104, 144 ; 184) pour des modules de chariot de traitement (102, 200, 210, 220 ; 52 ; 54) ; et
le dispositif de liaison est conçu pour former une liaison de forme, de force et/ou matérielle entre les chariots de traitement et un ou plusieurs modules de chariot de traitement.

6. Système de sélection (10) mobile selon l'une des revendications précédentes, dans lequel le chariot de traitement (100) présente un dispositif de liaison (104) pour un dispositif de déplacement (102) pouvant être séparé du chariot de traitement.

7. Système de sélection (10) mobile selon l'une des revendications précédentes, dans lequel :
soit l'interface utilisateur (50) est reliée de façon inamovible au chariot de traitement (100) ;
soit l'interface utilisateur (52) est ou peut être reliée de façon amovible au chariot de traitement (180) et/ou l'interface utilisateur (52), tandis que celle-ci est conçue pour des entrées à la suite desquelles le système de commande (20) détermine l'identificateur de traitement du traitement à réaliser, est ou peut être positionnée en un emplacement géographique autre que l'emplacement du chariot de traitement (160, 180).

8. Système de sélection (10) mobile selon l'une des revendications précédentes, lequel présente au moins un autre chariot de traitement (100, 160, 180) et dans lequel le système de sélection (10) mobile présente au moins une autre interface utilisateur (50, 52) et/ou au moins l'une des interfaces utilisateur (52) peut être attribuée au chariot de traitement et à l'autre chariot de traitement pour des entrées et à cet effet :
au moins une des interfaces utilisateur (50) est reliée de façon inamovible à l'un des chariots de traitement (100, 160, 180) et est ainsi attribuée à ce chariot de traitement (100) ;
le système de sélection mobile présente respectivement pour chacun des chariots de traitement une telle interface utilisateur reliée de façon inamovible, laquelle est ainsi attribuée au chariot de traitement respectif ;
au moins l'une des interfaces utilisateur est reliée de façon amovible à l'un des chariots de traitement et est ainsi attribuée à ce chariot de traitement ; et/ou
le système de commande (20) présente une instruction d'attribution pour des interfaces utilisateur (50, 52) et un dispositif de mémoire de données (24) pour celles-ci et cette instruction d'attribution est conçue pour, à l'aide de données d'attribution, lesquelles sont mémorisées sur ce dispositif de mémoire de données (24), attribuer un chariot de traitement (100, 160, 180), lequel est caractérisé au moyen d'un identificateur de chariot de traitement, à une interface utilisateur, laquelle est **caractérisée** au moyen d'un identificateur d'interface utilisateur.

9. Système de sélection (10) mobile selon l'une des revendications précédentes, dans lequel le système de commande (20) est conçu pour annoncer l'utilisateur au système en fonction d'une identification réussie, déterminer un identificateur d'utilisateur, réaliser le procédé de sélection et/ou limiter la sélection ou la restitution d'accessoires médicaux à des catégories d'accessoires pour lesquelles l'utilisateur annoncé bénéficie d'une autorisation, et/ou, dans le cas où l'utilisateur n'a pas réussi à être identifié, ne pas réaliser le procédé de sélection et/ou ne pas limiter la sélection ou la restitution d'accessoires médicaux à des catégories d'accessoires pour des utilisateurs non autorisés.

10. Système de sélection (10) mobile selon l'une des revendications précédentes, dans lequel :
l'une des interfaces utilisateur (50, 52) est conçue par l'utilisateur pour une ou plusieurs entrées via lesquelles un patient à traiter est établi et sur la base desquelles le système de commande (20) détermine à la suite de ces entrées un identificateur de patient attribué au patient à traiter ;
le procédé de sélection présente en outre l'étape de procédé (311) consistant à déterminer l'identificateur de patient du patient à traiter ; et
s'agissant du procédé de sélection, les paramètres de réglage d'accessoire sont déterminés (314) sur la base de l'identificateur de traitement et/ou de l'identificateur de patient.

11. Système de sélection (10) mobile selon l'une des revendications précédentes, lequel présente en outre un système d'identification (130 avec 158 ; 166) pour des traitements qui, au moyen d'une technologie de capteurs (158) fondée sur une mesure de rayonnement, d'ultrason, de poids et/ou d'inertie, saisit une personne (66) participant au traitement, un accessoire médical et/ou leur agencement et/ou leur déplacement l'un par rapport à l'autre, est enregistré et sur cette base détermine un traitement réalisé et/ou une étape de traitement réalisée.

12. Système de sélection (10) mobile selon l'une des revendications précédentes, dans lequel le système de commande (20) est conçu pour réaliser un procédé d'assistance, lequel comprend au moins l'étape de procédé consistant à déterminer un ou plusieurs paramètres d'assistance, lesquels identifient à cet effet le traitement à réaliser, une étape de traitement à réaliser du traitement à réaliser et/ou un accessoire médical.

13. Chariot de traitement (100) destiné à un système de sélection (10) mobile selon l'une des revendications précédentes, dans lequel le chariot de traitement (100) présente :
l'interface utilisateur (50) du système de sélection mobile ;
un ou plusieurs équipements de stockage (110 ; 114 ; 120 ; 202 ; 214), dans lequel les équipements de stockage présentent au moins deux zones de stockage (112 ; 116 ; 122, 124, 126 ; 203 ; 215) pour des accessoires médicaux chacun d'une catégorie d'accessoires ;
au moins un mécanisme de restitution (111 ; 115 ; 121 ; 204 ; 213 avec 212) pour restituer des accessoires médicaux ;
un dispositif de commande (130), lequel présente au moins une partie du système de commande (20) du système de sélection (10) mobile et/ou un équipement de communication (31) pour former une liaison de données (32) avec le système de commande (20) ou une autre partie du système de commande, dans lequel le dispositif de commande (130) pour commander le au moins un mécanisme de restitution par le système de commande (20) est conçu pour commander électriquement le au moins un mécanisme de restitution sur la base des paramètres de réglage d'accessoire ; et
une surface de dépôt pouvant être désinfectée ou un équipement de dépôt (108, 168) avec une surface de dépôt pouvant être désinfectée, dans lequel la surface de dépôt est conçue pour le dépôt d'accessoires médicaux et/ou présente une ou plusieurs zones de restitution pour l'extraction d'accessoires médicaux, et un dispositif de désinfection pour la surface de dépôt, dans lequel
le dispositif de commande (130) pour commander le dispositif de désinfection pour la surface de dépôt par le système de commande (20) est conçu pour commander le dispositif de désinfection pour la surface de dépôt de sorte que le dispositif de désinfection désinfecte la surface de dépôt avant, pendant et/ou après le traitement à réaliser et/ou avant, pendant et/ou après des étapes de traitement déterminées du traitement à réaliser.

14. Procédé (300) destiné à sélectionner des accessoires médicaux parmi une pluralité d'accessoires médicaux, en particulier pour une cannulation de vaisseaux sanguins de patients et/ou pour un traitement par dialyse, et ainsi restituer ces accessoires médicaux depuis un chariot de traitement (100, 160, 180) avec un ou plusieurs équipements de stockage (110, 114, 120 ; 170 ; 190 ; 202, 214), avec une surface de dépôt pouvant être désinfectée ou un équipement de dépôt (108, 168) avec une surface de dépôt pouvant être désinfectée, dans lequel la surface de dépôt est conçue pour le dépôt d'accessoires médicaux et/ou présente une ou plusieurs zones de restitution pour l'extraction d'accessoires médicaux, et avec un dispositif de désinfection pour la surface de dépôt, dans lequel les accessoires médicaux de la pluralité d'accessoires médicaux, triés en fonction de leurs catégories d'accessoires respectives, sont stockés dans au moins deux zones de stockage (112, 116, 122, 124, 126 ; 172, 174, 176 ; 192, 194, 196 ; 203, 215) des équipements de stockage, avec les étapes de procédé suivantes :
(310) saisir une ou plusieurs entrées par un utilisateur, via lesquelles un traitement à réaliser est établi, au moyen d'une interface utilisateur (50, 52) qui se situe en liaison de données avec un système de commande (20) ;
(312) déterminer un identificateur de traitement du traitement à réaliser sur la base de ces entrées au moyen du système de commande (20) ;
(314) déterminer un ou plusieurs paramètres de réglage d'accessoire, lesquels identifient un ensemble d'accessoires médical parmi un ou plusieurs accessoires médicaux appropriés pour le traitement à réaliser, à l'aide d'une banque de données d'accessoires (22) et au moins sur la base de l'identificateur de traitement du traitement à réaliser au moyen du système de commande (20), dans lequel la banque de données d'accessoires présente des ensembles de données sur la pluralité d'accessoires médicaux et est mémorisée sur un dispositif de mémoire de données (24) du système de commande (20) ;
(316) commander un mécanisme de restitution (111, 115, 121 ; 171 ; 191 ; 204, 213 avec 212) qui est conçu pour restituer des accessoires médicaux depuis au moins un des équipements de stockage et qui se situe en liaison de données avec le système de commande (20), sur la base des paramètres de réglage d'accessoire, dans lequel le chariot de traitement (100, 160, 180) présente le mécanisme de restitution ; et
(318) restituer, au moyen du mécanisme de restitution, les accessoires médicaux pour les catégories d'accessoires desquels le au moins un des équipements de stockage présente des zones de stockage et lesquels sont **caractérisés par** les paramètres de réglage d'accessoire sur lesquels se base la commande du mécanisme de restitution,
commander le dispositif de désinfection pour la surface de dépôt au moyen du système de commande (20), de sorte que le dispositif de désinfection désinfecte la surface de dépôt avant, pendant et/ou après le traitement à réaliser et/ou avant, pendant et/ou après des étapes de traitement déterminées du traitement à réaliser.
